# EUROPEAN PATENT SPECIFICATION

(11) **EP 1 763 586 B1**
(45) Date of publication and mention of the grant of the patent: **14.11.2007**
(21) Application number: 05756870.1
(22) Date of filing: 07.07.2005
(51) Int. Cl.: C12Q 1/68

(54) **NOVEL SEQUENCE FOR IMPROVING EXPRESSION OF NUCLEIC ACID**
NEUE SEQUENZ UM DIE EXPRESSION EINER NUKLEINSÄURE ZU ERHÖHEN
NOUVELLE SEQUENCE POUR AMELIORER L'EXPRESSION D'ACIDE NUCLEIQUE

(30) Priority: 08.07.2004 US 587007 P; 08.07.2004 EP 04051405
(43) Date of publication of application: 21.03.2007
(73) Proprietor: ChromaGenics B.V., 2333 CN Leiden (NL)
(72) Inventor: OTTE, Arie, Pieter, NL-3823 SG Amersfoort (NL); KWAKS, Theodorus, Hendrikus, Jacobus, NL-1022 XW Amsterdam (NL); SEWALT, Richard George Antonius Bernardus, NL-6814 HD Arnhem (NL); VAN BLOKLAND, Henricus Johannes Maria, NL-1456 NH Wijdewormer (NL)
(74) Representative: Verhage, Richard Abraham
(86) International application number: PCT/EP2005/053251
(87) International publication number: WO 2006/005718

(56) References cited:
- WO-A-03/004704
- WO-A-03/106684
- WO-A-20/04056986

## Description

The invention relates to the field of molecular biology and biotechnology. More specifically the present invention relates to means and methods for improving the production of one or more nucleic acids that may encode proteins in a host cell.

Proteins can be produced in various host cells for a wide range of applications in biology and biotechnology, for instance as biopharmaceuticals. Methods for such production are well established, and generally entail the expression in a host cell of a nucleic acid (also referred to as 'transgene') encoding the protein of interest. One problem associated with the expression of transgenes is that it is unpredictable, stemming from the high likelihood that the transgene will become inactive due to gene silencing (McBurney et al., 2002), and therefore many host cell clones have to be tested for high expression of the transgene. Furthermore, once such a clone has been established, the expression of the transgene is often not stable and silencing of transgene expression during prolonged host cell cultivation is a commonly observed phenomenon. In vertebrate cells it can be caused by formation of heterochromatin at the transgene locus, which prevents transcription of the transgene (Whitelaw et al, 2001). Transgene silencing is stochastic; it can occur shortly after integration of the transgene into the genome, or only after a number of cell divisions. This results in heterogeneous cell populations after prolonged cultivation, in which some cells continue to express high levels of recombinant protein while others express low or undetectable levels of the protein (Martin & Whitelaw, 1996, McBurney et al., 2002). A cell line that is used for heterologous protein production is derived from a single cell, yet is often scaled up to, and maintained for long periods at, cell densities in excess often million cells per ml in cultivators of 1,000 liters or more. These large cell populations (10¹⁴ - 10¹⁶ cells) are prone to serious declines in productivity due to transgene silencing (Migliaccio et al., 2000, Strutzenberger et al., 1999).

One possibility to overcome the problems described above is to employ so-called STAR sequences in the expression system. A variety of such STAR sequences have been described in WO 03/004704. These sequences can be used to improve predictability, yield and/or stability of protein production using expression constructs in several types of host cells (WO 03/004704; Kwaks et al, 2003).

International publication WO 03/106674 describes the use of STAR sequences for expressing nucleic acid encoding recombinant proteins in several cell lines.

International publication WO 03/106684 describes the use of STAR sequences for expressing nucleic acid encoding multimeric proteins, such as antibodies.

The present invention aims at providing alternative means and methods for improving protein production.

### Summary of the invention

The invention provides recombinant nucleic acid molecules, cells, methods and a use according to the claims.

### Detailed description of the invention

### Anti-repressor sequences

Sequences having anti-repressor activity as used herein are sequences that are capable of at least in part counteracting the repressive effect of HP1 or HPC2 proteins when these proteins are tethered to DNA. Sequences having anti-repressor activity (sometimes also referred to as anti-repressor sequences or anti-repressor elements herein) suitable for the present invention, have been disclosed in WO 03/004704, incorporated herein by reference, and were coined "STAR" sequences therein (wherever a sequence is referred to as a STAR sequence herein, this sequence has anti-repressor activity according to the invention). As a non-limiting example, the sequences of 65 anti-repressor elements, named STAR1-65 (see WO 03/004704), are presented herein as SEQ. ID. NOs. 1-65, respectively.

According to the invention, a functional fragment or derivative of a given anti-repressor element is considered equivalent to said anti-repressor element, when it still has anti-repressor activity. The presence of such anti-repressor activity can easily be checked by the person skilled in the art, for instance by the assay described below. Functional fragments or derivatives can easily be obtained by a person skilled in the art of molecular biology, by starting with a given anti-repressor sequence, and making deletions, additions, substitutions, inversions and the like (see e.g. WO 03/004704). A functional fragment or derivative also comprises orthologs from other species, which can be found using the known anti-repressor sequences by methods known by the person skilled in the art (see e.g. WO 03/004704). Hence, the present invention encompasses fragments of the anti-repressor sequences, wherein said fragments still have anti-repressor activity. For fragments of a given sequence, percent identity refers to that portion of the reference native sequence that is found in the fragment. The invention also encompasses sequences that are at least 70% identical in nucleotide sequence to said sequences having anti-repressor activity or to functional fragments thereof having anti-repressor activity, as long as these sequences that are at least 70% identical still have the anti-repressor activity according to the invention. Preferably, said sequences are at least 80% identical, more preferably at least 90% identical and still more preferably at least 95% identical to the reference native sequence or functional fragment thereof.

Sequences having anti-repressor activity according to the invention can be obtained by various methods, including but not limited to the cloning from the human genome or from the genome of another organism, or by for instance amplifying known anti-repressor sequences directly from such a genome by using the knowledge of the sequences, e.g. by PCR, or can in part or wholly be chemically synthesized.

Sequences having anti-repressor activity, and functional fragments or derivatives thereof, are structurally defined herein by their sequence and in addition are functionally defined as sequences having anti-repressor activity, which can be determined with the assay described below.

Any sequence having anti-repressor activity according to the present invention should at least be capable of surviving the following functional assay (see WO 03/004704, example 1, incorporated herein by reference). Human U-2 OS cells (ATCC HTB-96) are stably transfected with the pTet-Off plasmid (Clontech K1620-A) and with nucleic acid encoding a LexA-repressor fusion protein containing the LexA DNA binding domain and the coding region of either HP 1 or HPC2 (Drosophila Polycomb group proteins that repress gene expression when tethered to DNA; the assay works with either fusion protein) under control of the Tet-Off transcriptional regulatory system (Gossen and Bujard, 1992). These cells are referred to below as the reporter cells for the anti-repressor activity assay. A reporter plasmid, which provides hygromycin resistance, contains a polylinker sequence positioned between four LexA operator sites and the SV40 promoter that controls the zeocin resistance gene. The sequence to be tested for anti-repressor activity can be cloned in said polylinker. Construction of a suitable reporter plasmid, such as pSelect, is described in example 1 and Fig. 1 of WO 00/004704, The reporter plasmid is transfected into the reporter cells, and the cells are cultured under hygromycin selection (25 µg/ml; selection for presence of the reporter plasmid) and tetracycline repression (doxycycline, 10 ng/ml; prevents expression of the LexA-repressor fusion protein). After 1 week of growth under these conditions, the doxycycline concentration is reduced to 0.1 ng/ml (or lower) to induce the LexA-repressor gene, and after 2 days zeocin is added to 250 µg/ml. The cells are cultured for 5 weeks, until the control cultures (transfected with empty reporter plasmid, i.e. lacking a cloned anti-repressor sequence in the polylinker) are killed by the zeocin (in this control plasmid, the SV40 promoter is repressed by the LexA-repressor fusion protein that is tethered to the LexA operating sites, resulting in insufficient zeocin expression in such cells to survive zeocin selection). A sequence has anti-repressor activity according to the present invention if, when said sequence is cloned in the polylinker of the reporter plasmid, the reporter cells survive the 5 weeks selection under zeocin. Cells from such colonies can still be propagated onto new medium containing zeocin after the 5 weeks zeocin selection, whereas cells transfected with reporter plasmids lacking anti-repressor sequences cannot be propagated onto new medium containing zeocin. Any sequence not capable of conferring such growth after 5 weeks on zeocin in this assay, does not qualify as a sequence having anti-repressor activity, or functional fragment or functional derivative thereof according to the present invention. As an example, known boundary sequences such as those tested by Van der Vlag et al (2000), including Drosophila scs (Kellum and Schedl, 1991), 5'-HS4 of the chicken β-globin locus (Chung et al, 1993, 1997) or Matrix Attachment Regions (MARs) (Phi-Van et al., 1990), do not survive this assay.

In addition, it is preferred that the anti-repressor sequence or functional fragment or derivative thereof confers a higher proportion of reporter over-expressing clones when flanking a reporter gene (e.g. luciferase, GFP) which is integrated into the genome of U-2 OS or CHO cells, compared to when said reporter gene is not flanked by anti-repressor sequences, or flanked by weaker repression blocking sequences such as Drosophila scs. This can be verified using for instance the pSDH vector, or similar vectors, as described in example 1 and Fig. 2 of WO 03/004704.

Anti-repressor elements of the invention can have at least one of three consequences for production of protein: (1) they increase the predictability of identifying host cell lines that express a protein at industrially acceptable levels; (2) they result in host cell lines with increased protein yields; and/or (3) they result in host cell lines that exhibit more stable protein production during prolonged cultivation. Each of these attributes is discussed in more detail below:
(1) Increased predictability: Integration of transgene expression cassettes can occur at random positions throughout the host cell genome. However, much of the genome is transcriptionally silent heterochromatin. When the expression cassettes include anti-repressor elements flanking the transgene, the position of integration has a reduced effect on expression. The anti-repressor elements impair the ability of adjacent heterochromatin to silence the transgene. Consequently, the proportion of transgene-containing host cells with acceptable expression levels is increased. Indeed, incorporation of anti-repressor sequences results in the establishment of up to 10 times more colonies, compared to the same transgene lacking anti-repressor sequences, when the same amount of DNA is transfected.
(2) Yield: The levels of protein expression in primary populations of recombinant host cells, directly after transgene integration, have been surveyed. The expression level of individuals in the populations varies. However, when the transgenes are protected by anti-repressor elements, the variability is reduced. This reduced variability is most conspicuous in that fewer clones are recovered that have low levels of expression. Furthermore, the populations with anti-repressor elements commonly have individuals with strikingly high expression. These high-yielding individuals are favourable for production of proteins, either for harvesting purposes or for purposes of changing the phenotype of the cell or an organism comprising such cells.
(3) Increased stability: anti-repressor elements increase the stability of transgenes in recombinant host cell lines by ensuring that the transgenes are not transcriptionally silenced during prolonged cultivation. Comparative trials show that, under conditions in which transgenes that are not protected by anti-repressor elements are progressively silenced (5 - 25 passages in cultivation), anti-repressor element-protected transgenes continue to be expressed at high levels. This is an advantage during industrial production of proteinaceous molecules, during which cell cultivation continues for prolonged periods, from a few weeks to many months. Similarly, stability of expression over prolonged periods may be advantageous in plants or animals with altered phenotypes as a consequence of recombinant expression of anti-repressor protected transgenes.

### STAR67

The present invention provides a novel sequence having anti-repressor acitivity, which was coined STAR67 (SEQ. ID. NO. 66). This anti-repressor sequence already strongly increases expression when it is placed only upstream of a promoter driving expression of a gene of interest, as is evident from the examples provided herein, whereas hitherto known potent anti-repressor sequences such as STAR6 or STAR7 appear to provide much less advantage in this configuration (they function especially well when flanking a transgene, i.e. when present both upstream and downstream of the transgene). Hence, STAR67 provides an alternative to already known anti-repressor sequences, and when placed upstream of a promoter appears to have increased benefit when compared to such known anti-repressor sequences. STAR67 is not an enhaucer-blocker, in contrast to other anti-repressor sequences tested for this property (Kwaks et al, 2003), providing another difference between STAR67 and other anti-repressor sequences disclosed before. Also, STAR67 may operate in a bi-directional manner, as shown in example 7. According to the invention the presence of STAR67 sequence in an expression cassette provides improved predictability and/or yield and/or stability of expression. It is demonstrated herein that STAR67 is functional in combination with various promoters, and in different cell lines.

### Expression cassette

A nucleic acid molecule according to the invention, comprising STAR67, may be in any format, e.g. as DNA fragment, optionally present on a cloning vector such as a plasmid, preferably an expression vector, and can be used for instance for cloning purposes using standard recombinant DNA technology. In preferred embodiments of the invention, nucleic acid comprises an expression cassette, which is useful to express sequences of interest, for instance in host cells.

An 'expression cassette' as used herein is a nucleic acid sequence comprising at least a promoter functionally linked to a sequence of which expression is desired, which sequence of which expression is desired preferably is an open reading frame encoding all or part of a protein of interest. The promoter is preferably a heterologous promoter with respect to said nucleic acid of interest, whereby a heterologous promoter is defined as a promoter which is not the natural promoter of said sequence of interest. In other words, some form of human intervention, e.g. molecular cloning, has been used at any point in time to make the functional combination of a heterologous promoter with a nucleic acid of interest, and it is readily understood in this context that a heterologous promoter can be derived from the same or from a different organism as the sequence of interest. Preferably, an expression cassette further contains transcription termination and polyadenylation sequences. Other regulatory sequences such as enhancers may also be included. The expression units according to the invention further comprise at least one anti-repressor sequence, such as STAR67. In certain preferred embodiments said anti-repressor sequence, preferably STAR67, is placed upstream of said promoter, preferably such that less than 2kb are present between the 3' end of the anti-repressor sequence and the start of the promoter sequence. In preferred embodiments, less than 1 kb, more preferably less than 500 nucleotides (nt), still more preferably less than about 200, 100, 50, or 30 nt are present between the 3' end of the anti-repressor sequence and the start of the promoter sequence. In certain preferred embodiments, the anti-repressor sequence is cloned directly upstream of the promoter, resulting in only about 0-20 nt between the 3' end of the anti-repressor sequence and the start of the promoter sequence.

To obtain expression of nucleic acid sequences encoding recombinant protein, it is well known to those skilled in the art that sequences capable of driving such expression, can be functionally linked to the nucleic acid sequences encoding the protein, resulting in recombinant nucleic acid molecules encoding a recombinant protein in expressible format. In general, the promoter sequence is placed upstream of the sequences encoding the protein of interest. Useful expression vectors are available in the art, e.g. the pcDNA and pEF vector series of Invitrogen, pMSCV and pTK-Hyg from BD Sciences, pCMV-Script from Stratagene, etc.

Where the sequence encoding the polypeptide of interest is properly inserted with reference to sequences governing the transcription and translation of the encoded polypeptide, the resulting expression cassette is useful to produce the protein of interest, referred to as expression. Sequences driving expression may include promoters, enhancers and the like, and combinations thereof. These should be capable of functioning in the host cell, thereby driving expression of the nucleic acid sequences that are functionally linked to them. The person skilled in the art is aware that various promoters can be used to obtain expression of a gene of interest in host cells. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter (Kaufman, 2000). Some well-known and much used promoters for expression in eukaryotic cells comprise promoters derived from viruses, such as adenovirus, e.g. the E1A promoter, promoters derived from cytomegalovirus (CMV), such as the CMV immediate early (IE) promoter (referred to herein as the CMV promoter) (obtainable for instance from pcDNA, Invitrogen), promoters derived from Simian Virus 40 (SV40) (Das et al, 1985), and the like. Suitable promoters can also be derived from eukaryotic cells, such as methallothionein (MT) promoters, elongation factor 1α (EF-1α) promoter (Gill et al., 2001), ubiquitin C or UB6 promoter (Gill et al., 2001; Schorpp et al, 1996), actin promoter, an immunoglobulin promoter, heat shock promoters, and the like. Some preferred promoters for obtaining expression in eukaryotic cells, which are suitable promoters in the present invention, are the CMV-promoter, a mammalian EF1-alpha promoter, a mammalian ubiquitin promoter such as a ubiquitin C promoter, or a SV40 promoter (e.g. obtainable from pIRES, cat.no. 631605, BD Sciences). Testing for promoter function and strength of a promoter is a matter of routine for a person skilled in the art, and in general may for instance encompass cloning a test gene such as lacZ, luciferase, GFP, etc. behind the promoter sequence, and test for expression of the test gene. Of course, promoters may be altered by deletion, addition, mutation of sequences therein, and tested for functionality, to find new, attenuated, or improved promoter sequences.

An expression cassette according to the invention may be monocistronic, bicistronic or multicistronic. The term "bicistronic gene," is defined as a gene capable of providing a RNA molecule that encodes two proteins/polypeptides. The term "monocistronic gene" is defined as a gene capable of providing a RNA molecule that encodes one protein/polypeptide. The term "multicistronic" is defined as a gene capable of providing a RNA molecule that encodes two or more proteins/polypeptides, and a bicistronic gene is therefore encompassed within the definition of a multicistronic gene. A "gene" as used in the present invention can comprise chromosomal DNA, cDNA, artificial DNA, combinations thereof, and the like, and could also be in the form of other nucleic acid, e.g. RNA. In certain embodiments, a protein expression unit comprises a multicistronic gene. Units comprising several cistrons can be transcribed as a single mRNA. Translation of the second and further coding regions present on that RNA can be achieved in various ways, including the use of translation reinitiation sites or internal ribosome entry sites, the latter of which is preferred. One advantage of bi- or multi-cistronic units can be an easy selection of clones expressing a protein of interest, by placing the nucleic acid encoding a selectable marker protein downstream of nucleic acid encoding a protein or polypeptide of interest.

For the production of multimeric proteins, two or more expression cassettes can be used. This embodiment has proven to give good results, e.g. for the expression of the heavy and light chain of antibodies. According to the invention, at least one of the expression cassettes, but preferably each of them, should comprise a STAR sequence. In another embodiment, the different subunits or parts of a multimeric protein are present on a single expression cassette.

Instead of or in addition to the presence of an anti-repressor sequence placed upstream of a promoter in an expression cassette, it has proven highly beneficial to provide an anti-repressor sequence on both sides of an expression cassette, such that expression cassette comprising the transgene is flanked by two anti-repressor sequences, which in certain embodiments are essentially identical to each other. Of course, this can also be done with STAR67, to obtain an expression cassette flanked by two STAR67 sequences. Alternative positions of a single STAR67 sequence in an expression cassette, e.g. behind the transgene, preferably behind transcriptional termination and polyadenylation signal, with the 3' end of the STAR67 sequence facing the transgene, are also possible.

An expression cassette according to the invention may optionally comprise a selection marker gene. The term "selection marker or selectable marker" is typically used to refer to a gene and/or protein whose presence can be detected directly or indirectly in a cell, for example a gene and/or a protein that inactivates a selection agent and protects the host cell from the agent's lethal or growth-inhibitory effects (e.g. an antibiotic resistance gene and/or protein). Another possibility is that said selection marker induces fluorescence or a color deposit (e.g. green fluorescent protein and derivatives, luciferase, lacZ, alkaline phosphatase, etc.). In certain embodiments, a selection marker used for the invention is zeocin, and for selecting a second expression cassette puromycin is used. The person skilled in the art will know that other selection markers are available and can be used, e.g. neomycin, blasticidin, puromycin, bleomycin, hygromycin, dhfr, etc.
The term "selection" is typically defined as the process of using a selection marker/selectable marker and a selection agent to identify host cells with specific genetic properties (e.g. that the host cell contains a transgene integrated into its genome). It is clear to a person skilled in the art that numerous combinations of selection markers are possible. An example of a possible antibiotic is provided above. The one antibiotic that is particularly advantageous is zeocin, because the zeocin-resistance protein (zeocin-R) acts by binding the drug and rendering it harmless. Therefore it is easy to titrate the amount of drug that kills cells with low levels of zeocin-R expression, while allowing the high-expressors to survive. All other antibiotic-resistance proteins in common use are enzymes, and thus act catalytically (not 1:1 with the drug). When a two-step selection is performed it is therefore advantageous to use an antibiotic resistance protein with this 1:1 binding mode of action. Hence, the antibiotic zeocin is a preferred selection marker. For convenience the zeocin antibiotic can in a two-step selection method combined with for instance puromycin, blasticidin or hygromycin, which may for instance be present in a monocistronic gene.

It is also possible to combine an antibiotic selection marker with a selection marker which provides induction of fluorescence or which provide a colour deposit. Different promoters can be used as long as they are functional in the used cell.

In certain embodiments an expression cassette is provided with a (weak) Internal Ribosome Binding Site (IRES) as an example of a protein translation initiation site with a reduced translation efficiency, e.g. between the open reading frame of the protein of interest and the selection marker open reading frame. Translation of proteins from IRES elements is less efficient than cap-dependent translation: the amount of protein from IRES-dependent open reading frames (ORFs) ranges from less than 20% to 50% of the amount from the first ORF (Mizuguchi et al., 2000). Furthermore, mutation of IRES elements can attenuate their activity, and lower the expression from the IRES-dependent ORFs to below 10% of the first ORF (Lopez de Quinto & Martinez-Salas, 1998, Rees et al., 1996). When the IRES-dependent ORF encodes a selectable marker protein, its low relative level of translation means that high absolute levels of transcription must occur in order for the recombinant host cell to be selected. Therefore, selected recombinant host cell isolates will by necessity express high amounts of the transgene mRNA. Since the recombinant protein is translated from the cap-dependent ORF, it can be produced in abundance resulting in high product yields.

Conventional expression systems are DNA molecules in the form of a recombinant plasmid or a recombinant viral genome. The plasmid or the viral genome is introduced into (eukaryotic host) cells and preferably integrated into their genomes by methods known in the art. In preferred embodiments, the present invention also uses these types of DNA molecules to deliver its improved transgene expression system. A preferred embodiment of the invention is the use of plasmid DNA for delivery of the expression system. A plasmid contains a number of components: conventional components, known in the art, are an origin of replication and a selectable marker for propagation of the plasmid in bacterial cells; a selectable marker that functions in eukaryotic cells to identify and isolate host cells that carry an integrated transgene expression system; nucleic acid encoding the protein of interest, whose high-level transcription is brought about by a promoter that is functional in eukaryotic cells (e.g. the human cytomegalovirus major immediate early promoter/enhancer, pCMV (Boshart et al., 1985); and transcriptional terminators (e.g. the SV40 polyadenylation site (Kaufman & Sharp, 1982) for the transgene of interest and the selectable marker.
The vector used can be any vector that is suitable for cloning DNA and that can be used for transcription of a nucleic acid of interest. When host cells are used it is preferred that the vector is an integrating vector. Alternatively, the vector may be an episomally replicating vector.

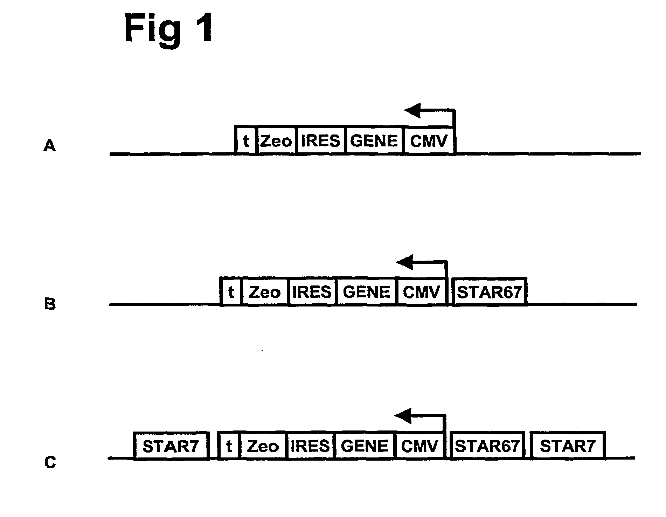

Some non-limiting, schematic representations of possible configurations of expression cassettes are provided in Fig. 1. This is the configuration of the DNA elements of the expression cassettes in the plasmid as well as after integration into the genome. Construct A contains an expression unit that encompasses an open reading frame encoding a protein (Gene). This is upstream of the attenuated EMCV IRES (Martinez-Salas et al 1999; Mizuguchi et al 2000; Rees et al 1996), and of the open reading frame encoding the zeocin resistance selectable marker protein (zeo). The gene cassette has the SV40 transcriptional terminator at their 3' ends (t). This bicistronic transgene is transcribed at high levels from the CMV promoter. Construct B is derived from construct A, but STAR67 is now cloned upstream of the CMV promoter. Construct C is derived from construct B, but now two anti-repressor elements (in this case STAR7) are cloned to flank the entire cassette.

### Combination of anti-repressor sequences in expression cassette

It is shown herein that the combination of a first anti-repressor element upstream of a promoter and flanking the expression cassette by two other anti-repressor sequences provides superior results. In particular, when an SV40 promoter is used in CHO cells, expression is already very high in the absence of anti-repressor sequences, but is considerably enhanced when STAR67 is placed upstream of said promoter, and the whole expression cassette is flanked by two anti-repressor elements, such as STAR6, STAR7, or STAR 40.

It is therefore an object of the present invention to provide a recombinant nucleic acid molecule comprising an expression cassette comprising (from 5' to 3'): anti-repressor sequence A - promoter - nucleic acid encoding a protein of interest - anti-repressor sequence B, characterized in that said expression cassette further comprises a anti-repressor sequence C between said anti-repressor sequences A and B. In a preferred embodiment, said anti-repressor sequence C is present upstream of said promoter, in a configuration as described above under the heading 'expression cassette'. The expression cassette between the anti-repressor sequences A and B may further comprise the elements as described above for expression cassettes, e.g. transcription terminator sequence, polyadenylation signal, selection marker gene, enhancer, etc., and may be monocistronic, bicistronic or multicistronic as described above.

Two or all three of anti-repressor sequences A, B and C may be the same, or all three may be different. Anti-repressor sequences A and B may the same or different as anti-repressor sequence C. In certain embodiments, anti-repressor sequence A and B are (essentially) identical to each other. In one embodiment, anti-repressor sequence C is STAR67, or a functional fragment or derivative thereof. Anti-repressor sequences A and B can be any anti-repressor sequence, and in certain embodiments comprise one of SEQ. ID. NOs. 1-65, or functional fragments or derivatives thereof. In certain embodiments, the expression cassette contains a multicistronic gene, and in preferred embodiments thereof said multicistronic gene comprises a sequence encoding a protein of interest and a selection marker gene. Alternatively, a selection marker gene is present under control of a separate promoter.

In certain embodiments, a fourth anti-repressor sequence D may be present between STAR sequences A and B. In such an embodiment, said anti-repressor sequence D is preferably positioned downstream of the nucleic acid encoding the protein of interest. Again, this anti-repressor sequence D may be the same or different from the other anti-repressor sequences in the recombinant nucleic acid molecule, it can be any anti-repressor sequence, and in certain embodiments is chosen from any one of SEQ. ID. NOs. 1-66, or functional fragments or derivatives thereof.

As at least some and-repressor sequences can be directional (WO 00/004704), the anti-repressor sequences flanking the expression cassette (anti-repressor sequences A and B) may beneficially placed in opposite direction with respect to each other, such that the 3' end of each of these anti-repressor sequences is facing inwards to the expression cassette (and to each other). Hence, in preferred embodiments, the 5' side of an anti-repressor element faces the DNA/chromatin of which the influence on the transgene is to be diminished by said anti-repressor element. For an anti-repressor sequence upstream of a promoter in an expression cassette, the 3' end faces the promoter. The sequences of the anti-repressor elements (SEQ. ID. NOs. 1-66) in the sequence listing are given in 5' to 3' direction, unless otherwise indicated.

### Cells according to the invention

Nucleic acid molecules comprising STAR sequences and/or expression cassettes according to the present invention can be used for improving expression of nucleic acid, preferably in host cells. The terms "cell"/"host cell" and "cell line"/"host cell line" are respectively typically defined as a cell and homogeneous populations thereof that can be maintained in cell culture by methods known in the art, and that have the ability to express heterologous or homolagous proteins. A host cell according to the present invention preferably is a eukaryotic cell, more preferably a mammalian cell, such as a rodent cell or a human cell or fusion between different cells. In certain non-limiting embodiments, said host cell is a U-2 OS osteosarcoma, CHO (Chinese hamster ovary), HEK 293, HuNS-1 myeloma, WERI-Rb-1 retinoblastoma, BHK, Vero, non-secreting mouse myeloma Sp2/0-Ag 14, non-secreting mouse myeloma NSO, NCI-H295R adrenal gland carcinomal or a PER.C6^{®} cell.
In certain embodiments of the invention, a host cell is a cell expressing at least E1A, and preferably also E1B, of an adenovirus. As non-limiting examples, such a cell can be derived from for instance human cells, for instance from a kidney (example: HEK 293 cells, see Graham et al, 1977), lung (e.g. A549, see e.g. WO 98/39411) or retina (example: HER cells marketed under the trade mark PER.C6^{®}, see US patent 5,994,128), or from amniocytes (e.g. N52.E6, described in US patent 6,558,948), and similarly from other cells. Methods for obtaining such cells are described for instance in US patents 5,994,128 and US 6,558,948. PER.C6® cells for the purpose of the present application means cells from an upstream or downstream passage or a descendent of an upstream or downstream passage of cells as deposited under ECACC no. 96022940. It has been previously shown that such cells are capable of expression of proteins at high levels (e.g. WO 00/63403, and Jones et al, 2003).

Such host cells expressing the desired protein according to the invention can be obtained by introduction of a nucleic acid molecule, preferably in the form of an expression cassette according to the invention, into the cells. In an alternative embodiment, the STAR67 sequence is targeted for integration into a chromosomal region to improve the expression of a gene of interest that is already integrated into the genome, e.g. a naturally occurring gene, optionally under control of a heterologous promoter that was targeted upstream of said gene to regulate expression of said gene.
Preferably the host cells are from a stable clone that can be selected and propagated according to standard procedures known to the person skilled in the art. A culture of such a clone is capable of producing recombinant protein of interest. Cells according to the invention preferably are able to grow in suspension culture in serum-free medium.

A protein of interest according to the invention can be any protein, and may be a monomeric protein or a multimeric protein. A multimeric protein comprises at least two polypeptide chains. Non-limiting examples of a protein of interest according to the invention are enzymes, hormones, immunoglobulin chains, therapeutic proteins like anti-cancer proteins, blood coagulation proteins such as Factor VIII, multifunctional proteins, such as erythropoietin, diagnostic proteins, or proteins or fragments thereof useful for vaccination purposes, all known to the person skilled in the art.

In certain embodiments, an expression cassette of the invention encodes an immunoglobulin heavy or light chain or an antigen binding part, derivative and/or analogue thereof. In a preferred embodiment a protein expression unit according to the invention is provided, wherein said protein of interest is an immunoglobulin heavy chain. In yet another preferred embodiment a protein expression unit according to the invention is provided, wherein said protein of interest is an immunoglobulin light chain. When these two protein expression units are present within the same (host) cell a multimeric protein and more specifically an antibody is assembled. Hence, in certain embodiments, the protein of interest is an immunoglobulin, such as an antibody, which is a multimeric protein. Preferably, such an antibody is a human or humanized antibody. In certain embodiments thereof, it is an IgG, IgA, or IgM antibody. An innmunoglobulin may be encoded by the heavy and light chains on different expression cassettes, or on a single expression cassette, wherein the gene encoding each individual chain may each be under control of a separate promoter in monocistronic transcription units, or alternatively both chains may be encoded on a multicistronic gene.

The protein of interest may be from any source, and in certain embodiments is a mammalian protein, an artificial protein (e.g. a fusion protein or mutated protein), and preferably is a human protein.

Obviously, the anti-repressor sequences and configurations of the expression cassettes of the present invention may also be used when the ultimate goal is not the production of a protein, but the RNA itself, for instance for producing increased quantities of RNA from an expression cassette, which may be used for purposes of regulating other genes (e.g. RNAi, antisense RNA), gene therapy, in vitro protein production, etc.

### Method for producing protein of interest

In one aspect, the invention provides a method for expressing a sequence of interest, preferably encoding a protein of interest, by providing a host cell with a nucleic acid molecule or expression cassette according to the invention, culturing the cell and expressing the sequence of interest.

The term "expression" is typically used to refer to the production of a specific RNA product or products, or a specific protein or proteins, in a cell. In the case of RNA products, it refers to the process of transcription. In the case of protein products, it refers to the processes of transcription, translation and optionally post-translational modifications (e.g. glycosylation, disulfide bond formation, etc.). In the case of secreted proteins, it refers to the processes of transcription, translation, and optionally post-translational modification, followed by secretion.

Introduction of the nucleic acid that is to be expressed in a cell, can be done by one of several methods, which as such are known to the person skilled in the art, also dependent on the format of the nucleic acid to be introduced. Said methods include but are not limited to transfection, infection, injection, transformation, and the like.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Culturing can be done for instance in dishes, roller bottles or in bioreactors, using batch, fed-batch, continuous systems such as perfusion systems, and the like. In order to achieve large scale (continuous) production of recombinant proteins through cell culture it is preferred in the art to have cells capable of growing in suspension, and it is preferred to have cells capable of being cultured in the absence of animal- or human-derived serum or animal- or human-derived serum components.

The conditions for growing or multiplying cells (see e.g. Tissue Culture, Academic Press, Kruse and Paterson, editors (1973)) and the conditions for expression of the recombinant product are known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991). In a preferred embodiment, the expressed protein is collected (isolated), either from the cells or from the culture medium or from both. It may then be further purified using known methods, e.g. filtration, column chromatography, etc, by methods generally known to the person skilled in the art.

### Novel selection method

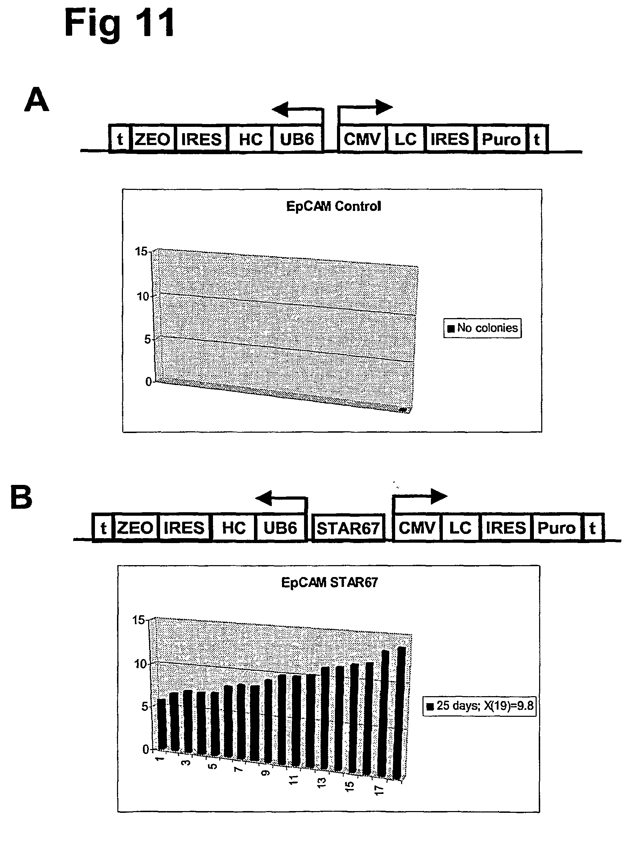

The present invention discloses a novel method for generating host cells expressing two polypeptides of interest, which method gives a surprisingly good result, in that almost no or no colonies appear without the use of an anti-repressor sequence, while the presence of an anti-repressor sequence does lead to clones surviving the selection, and these clones in general express the two polypeptides of interest at high levels (example 7, Fig. 11). The invention therefore provides a method for generating a host cell expressing two polypeptides of interest, the method comprising: a) introducing into a host cell one or more nucleic acid molecules, the nucleic acid molecule or molecules together comprising: (i) a promoter functionally linked to a sequence encoding a first polypeptide of interest and a first selectable marker gene, and (ii) a promoter functionally linked to a sequence encoding a second polypeptide of interest and a second selectable marker, and (iii) at least one anti-repressor sequence, chosen from the group consisting of (a) SEQ. ID. NO. 66, (b) fragments of SEQ. ID. NO. 66, said fragments having anti-repressor activity, (c) sequences that are at least 70% identical to (a) or (b) and having anti-repressor activity, and (d) the complement of any one of (a)-(c); b) selecting a host cell by essentially simultaneously selecting for expression of said first and second selectable marker genes. The selected host cells can be conveniently used for the expression of said two polypeptides, by culturing said selected host cells. In one embodiment, two nucleic acid molecules, one containing (i) and the other containing (ii), are used for introduction into the host cell. In this embodiment, each nucleic acid molecule preferably contains at least one said anti-repressor sequence. In another embodiment, a single nucleic acid molecule containing both (i) and (ii) is used, which must then contain at least one said anti-repressor sequence. One advantage of having the coding sequences for both polypeptides on a single nucleic acid molecule is that only one nucleic acid preparation is required before the nucleic acid is introduced into the cells. Furthermore, in such an embodiment a single integration event is sufficient for the coding sequences of both polypeptides, thereby eliminating the possibility that the nucleic acid encoding the first polypeptide is integrated on a different location or with a different copy number than that encoding the second polypeptide. In preferred embodiments, said two polypeptides may form part of a multimeric protein, such as an immunoglobulin. The method is therefore particularly suited for the expression of antibodies. The two promoters may be the same or different, and could be any promoters as described supra. When the embodiment where a single nucleic acid molecule contains both (i) and (ii) is used, the direction of the two transcription units on the single nucleic acid molecule may for instance be both the same, or in opposite directions facing each other, or in opposite directions each pointing outward. In the latter case, the anti-repressor sequence may be placed between the two promoters (e.g. see Fig. 11B). Furthermore, anti-repressor sequences may be added to flank one or both transcription units. The marker genes must each be different, and may for instance be selected from the marker genes as described supra. In certain embodiments, one selectable marker is zeocin, and the other selectable marker is for instance puromycin. However, it will be clear that other combinations may be used, and are within the scope of this aspect of the invention. Expression of the markers should preferably be dependent from the expression of the polypeptides of interest. This can be established by linking the expression of the marker genes to that of the polypeptides of interest for instance by using multicistronic genes, e.g. with IRES sequences, as discussed supra. "Essentially simultaneous" selection means that at least part of the time, preferably at least one day, more preferably at least two days, still more preferably at least 5 days, both selection agents are present, which can be brought about by having both selection agents added to the culture medium at the same time or adding culture medium comprising both selection agents (e.g. example 7). It will be clear that adding the second selection agent only after a few hours or days to medium wherein the first selection agent is already present, will still result in essentially simultaneous selection within the meaning of the invention. This situation is distinguished from the situation where cells are first selected with the first selection agent, and once colonies are formed the cells are selected on new medium whereto the second selection agent and not the first selection agent has been added (consecutive selection, e.g. example 5 and WO 03/106684). By selecting for both selectable markers essentially simultaneously, it is disclosed herein surprisingly that a fast and strong selection is provided, whereby many low-producing clones are eliminated, resulting in a strongly decreased workload for finding a clone with desirable expression characteristics. This is particularly advantageous for the biotechnological industry, where many, usually hundreds or even thousands, of colonies have to be screened before a desired clone having high expression levels is identified.

The practice of this invention will employ, unless otherwise indicated, conventional techniques of immunology, molecular biology, microbiology, cell biology, and recombinant DNA, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

The invention is further explained in the following examples. The examples do not limit the invention in any way. They merely serve to clarify the invention.

### Examples

### Example 1: Construction of STAR67 vectors

A novel anti-repressor sequence was isolated using a genetic screen as described in WO 03/004704, and this novel sequence was coined STAR67 (SEQ. ID. NO. 66). The effects of STAR67 on expression of transgenes in mammalian cell lines were tested. Here we describe the construction of the various constructs.

### Materials and Methods

Three plasmids were created (Fig. 1):
A) CMV-d2EGFP-ires-Zeo (CMV Control),
B) STAR67-CMV-d2EGFP-ires-Zeo (CMV-STAR67),
C) STAR7-STAR67-CMV-d2EGFP-ires-Zeo-STAR7 (CMV-STAR67 7/7)

The construction of construct A is described below. Plasmid pd2EGFP (Clontech 6010-1) was modified by insertion of a linker at the *BsiWI* site to yield pd2EGFP-link. The linker, made by annealing oligonucleotides GTACGGATATCAGATCTTTAATTAAG (SEQ. ID. NO. 67) and GTACCTTAATTAAAGATCTGATAT (SEQ. ID. NO. 68), introduced sites for the *Pac*I*, Bgl*II, and *Eco*RV restriction endonucleases. This created the multiple cloning site MCSII for insertion of STAR elements. Then primers
GATCAGATCTGGCGCGCCATTTAAATCGTCTCGCGCGTTTCGGTGATGACG G (SEQ. ID. NO. 69) and
(AGGCGGATCCGAATGTATTTAGAAAAATAAACAAATAGGGG (SEQ. ID. NO. 70) were used to amplify a region of 0.37 kb from pd2EGFP, which was inserted into the *Bgl*II site of pIRES (Clontech 6028-1) to yield pIRES-stuf. This introduced sites for the *Asc*I and *Swa*I restriction endonucleases at MCSI, and acts as a "stuffer fragment" to avoid potential interference between STAR elements and adjacent promoters, pIRES-stuf was digested with *Bgl*II and *Fsp*I to liberate a DNA fragment composed of the stuffer fragment, the CMV promoter, the IRES element (flanked by multiple cloning sites MCS A and MCS B), and the SV40 polyadenylation signal. This fragment was ligated with the vector backbone of pd2EGFP-link produced by digestion with *Bam*HI and *Stu*I, to yield pIRES-link.

The open reading frame of the zeocin-resistance gene was inserted into *Bam*HI/*Not*I sites downstream of the pIRES as follows: the zeocin-resistance ORF was amplified by PCR with primers
GATCGGATCCTTCGAAATGGCCAAGTTGACCAGTGC (SEQ. ID. NO. 71) and AGGCGCGGCCGCAATTCTCAGTCCTGCTCCTC (SEQ. ID. NO. 72) from plasmid pCMV/zeo (Invitrogen, cat.no. V50120), digested with *Bam*HI and *Not*I*,* and ligated with *Bam*HI/*Not*I-digested pIRES-link to yield pIRES-link-zeo. The d2EGFP reporter ORF was introduced into pIRES-link-zeo by amplification of pd2EGFP (Clontech 6010-1) with primers
GATCGAATTCTCGCGAATGGTGAGCAAGCAGATCCTGAAG (SEQ. ID. NO. 73) and
AGGCGAATTCACCGGTGTTTAAACTTACACCCACTCGTGCAGGCTGCCCA GG (SEQ. ID. NO. 74), and insertion of the *Eco*RI-digested d2EGFP cassette into the *Eco*RI site in the pIRES-link-zeo plasmid. This created construct A, CMV-d2EGFP-IRES-Zeo (CMV Control).

STAR67 was cloned upstream of the CMV promoter, in the AscI site (about 15 nt remaining between STAR67 and the promoter). This created construct B, STAR67-CMV-d2EGFP-ires-Zeo (CMV-STAR67).

STAR67 was also tested in the context of a cassette that contains also the STAR7 (SEQ. ID. NO. 7), cloned directionally in the 5' Sall and XbaI sites and 3' BglII and PacI sites to flank the entire cassette with STAR7. This is construct C (CMV-STAR67 7/7).

### Example 2: STAR67 enhances the expression level from CMV, EF1α and UB6 promoters in stably transfected CHO cells.

We tested whether the presence of STAR67 adjacent to the CMV, EF1α and UB6 promoters influences the expression level of these promoters in CHO cells. The constructs A and B (Fig. 1) described in Example 1 are used for this purpose, modified for the respective promoters:
1 CMV-d2EGFP-ires-Zeo (CMV Control)
2 STAR67-CMV-d2EGFP-ires-Zeo (CMV-STAR67)
3 EF 1 α-d2EGFP-ires-Zeo (EF 1 α Control)
4 STAR67-EF1α-d2EGFP-ires-Zeo (EF1α-STAR67)
5 UB6-d2EGFP-ires-Zeo (UB6 Control)
6 STAR67-UB6-d2EGFP-ires-Zeo (UB6-STAR67).

### Materials and Methods

The UB6 and EF1 α promoters were exchanged for the CMV promoter in the plasmids described in Fig. 1. The UB6 promoter was cloned as follows. A DNA 0.37 kb stuffer from the pd2EGFP plasmid was amplified by PCR, as described in example 1, using primers identified by SEQ. ID. NOs. 69 and 70. The resulting DNA stuffer was cloned in the BglII site of pUB6/V5-His [Invitrogen V250-20], creating pUB6-stuf. From pUB6-stuf an AscI-SacI fragment was cloned into CMV-d2EGFP-IRES-Zeo, from which the CMV promoter was removed.

The EF1α promoter was amplified by PCR with pEF1α/V5-His [Invitrogen V920-20] as template, using primers
GATCGGCGCGCCATTTAAATCCGAAAAGTGCCACCTCGACG (SEQ. ID. NO. 79) and
AGGCGGGACCCCCTCACGACACCTGAAATGGAAG (SEQ. ID. NO. 80). The PCR fragment was cloned in the AscI and PpuMI sites of CMV-d2EGFP-IRES-Zeo, from which the CMV promoter was removed.

The Chinese Hamster Ovary cell line CHO-K1 (ATCC CCL-61) was cultured in HAMS-F12 medium + 10% Fetal Calf Serum containing 2 mM glutamine, 100 U/ml penicillin, and 100 microgams/ml streptomycin at 37°C/5% CO₂. Cells were transfected with the plasmids using SuperFect (QIAGEN) as described by the manufacturer. Briefly, cells were seeded to culture vessels and grown overnight to 70-90% confluence. SuperFect reagent was combined with plasmid DNA at a ratio of 6 microliters per microgram (e.g. for a 10 cm Petri dish, 20 micrograms DNA and 120 microliters SuperFect) and added to the cells. After overnight incubation the transfection mixture was replaced with fresh medium, and the transfected cells were incubated further. After overnight cultivation, cells were trypsinized and seeded into fresh culture vessels with fresh medium. After another overnight incubation zeocin was added to a concentration of 50 µg/ml and the cells were cultured further. After another three days the medium was replaced by fresh medium containing zeocin (100 µg/ml) and cultured further. When individual colonies became visible (approximately ten days after transfection) medium was removed and replaced with fresh medium without zeocin. Individual clones were isolated and transferred to 24-well plates in medium without zeocin. One day after isolation of the colonies zeocin was added to the medium. Expression of the d2EGFP reporter gene was assessed approximately 3 weeks after transfection. d2EGFP expression levels in the colonies were measured after periods of two weeks. After the initial two weeks after transfection when the first d2EGFP measurements were performed, the colonies were cultured in medium without zeocin or other antibiotics. This continued for the remainder of the experiment.

### Results

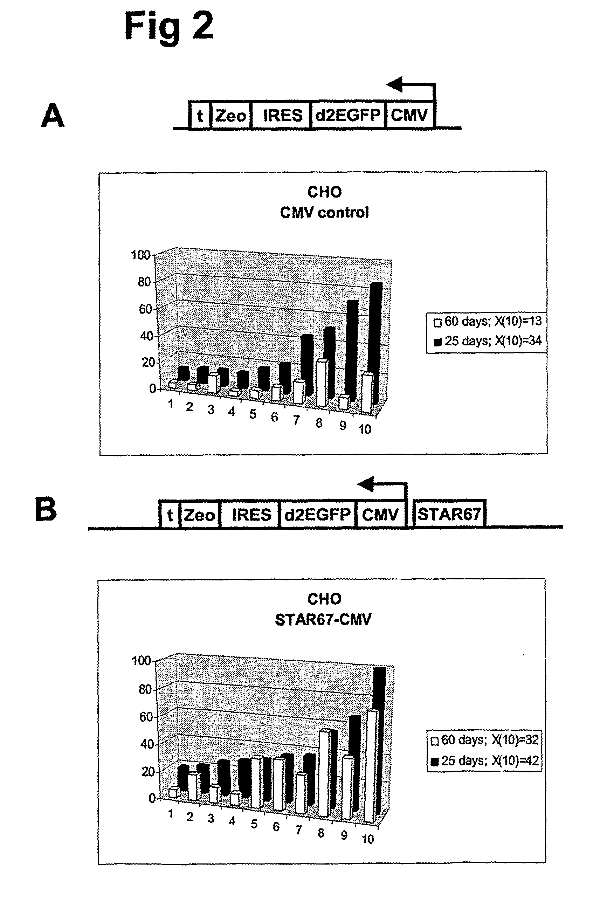

Fig. 2 shows that transfection of the construct that contains STAR67 cloned upstream of the CMV promoter resulted in a number of CHO colonies that express significantly higher levels of d2EGFP protein, as compared to the "empty" control without STAR67, CMV Control. The average of the d2EGFP signal in the 10 colonies transfected with the CMV Control plasmid was 34, when measured 25 days after transfection. 60 days after transfection the average of the d2EGFP signal in these 10 colonies was reduced to 13, indicating that expression is not stable over time. In comparison, the average of the d2EGFP signal in the 10 colonies transfected with the STAR67-CMV plasmid was 42 when measured after 25 days and 32 measured 60 days after transfection. Hence 60 days after transfection, a STAR67-encompassing CMV construct conveyed a factor 2.5 higher CMV promoter driven expression level of the reporter protein in stably transfected clones. Importantly, 25 days after transfection, after the first measurement, selection pressure was removed by culturing the colonies in medium without zeocin. Hence, colonies containing the STAR67 construct are more stable over time in the absence of selection pressure than colonies that do not contain a STAR67 construct.

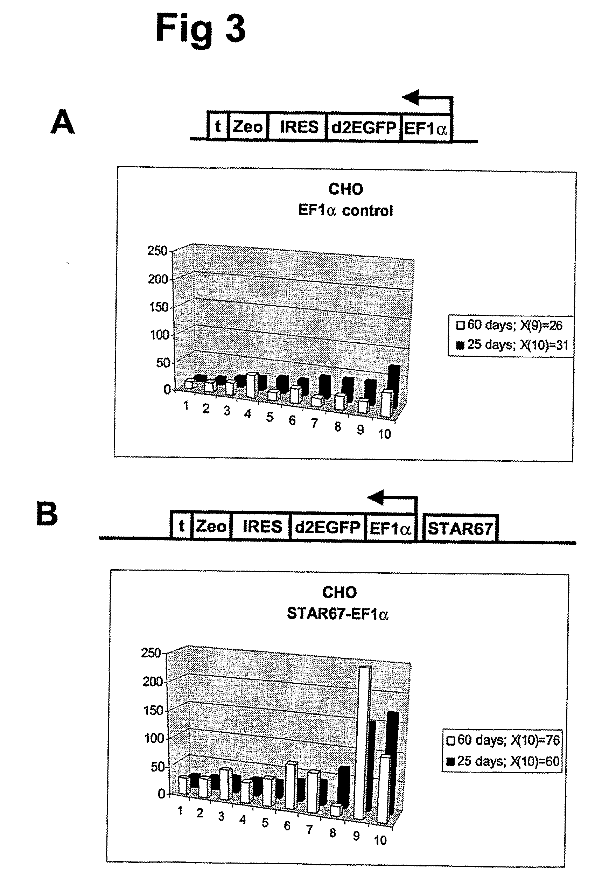

Fig. 3 shows that transfection of the construct that contains STAR67 cloned upstream of the EF1α- promoter resulted in a number of CHO colonies that express significantly higher levels of d2EGFP protein, as compared to the "empty" control without STAR67, EF1α Control. The average of the d2EGFP signal in the 10 colonies transfected with the EF1α Control plasmid was 31, when measured 25 days after transfection. 60 days after transfection the average of the d2EGFP signal in these 10 colonies was 26. In comparison, the average of the d2EGFP signal in the 10 colonies transfected with the EFIα-STAR67 plasmid was 60 when measured after 25 days and 76 measured 60 days after transfection. Hence, both after 25 and 60 days after transfection, a STAR67-encompassing EF1α construct conveyed a factor 2.9 higher EF1α promoter driven expression level of the reporter protein in stably transfected clones.

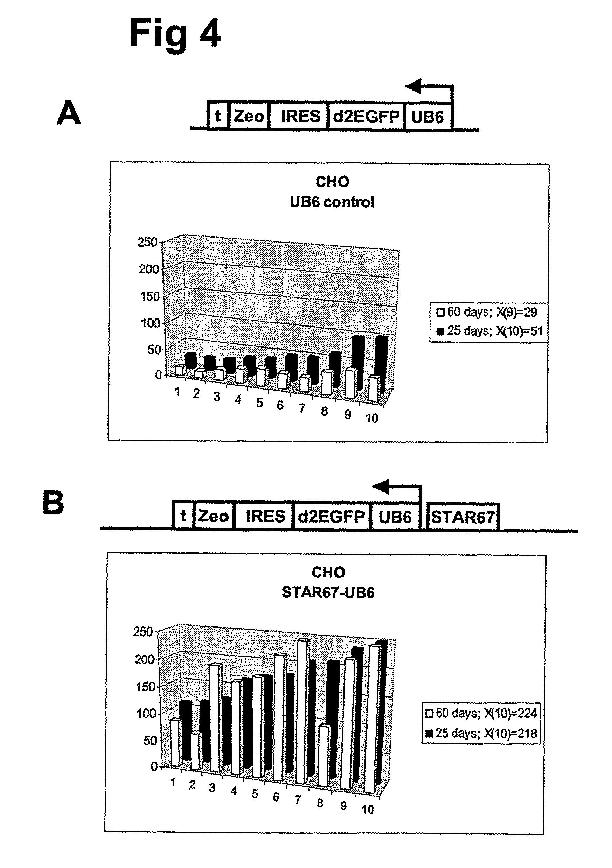

Fig. 4 shows that transfection of the construct that contains STAR67 cloned upstream of the UB6 promoter resulted in a number of CHO colonies that express significantly higher levels of d2EGFP protein, as compared to the "empty" control without STAR67, UB6 Control. The average of the d2EGFP signal in the 10 colonies transfected with the UB6 Control plasmid was 51, when measured 25 days after transfection. 60 days after transfection the average of the d2EGFP signal in these 10 colonies was 29, indicating that the expression was not stable over time. In comparison, the average of the d2EGFP signal in the 10 colonies transfected with the UB6-STAR67 plasmid was 218 when measured after 25 days and 224 measured 60 days after transfection. Hence, 25 days after transfection, a STAR67-encompassing UB6 construct conveyed a factor 4.3 higher UB6 promoter driven expression level of the reporter protein in stably transfected clones. After 60 days this factor was 7.7, due to instability of expression in the control colonies and stability in the UB6-STAR67 colonies. Importantly, 25 days after transfection, after the first measurement, selection pressure was removed by culturing the colonies in medium without zeocin. Hence, colonies containing the STAR67 construct are more stable over time in the absence of selection pressure than colonies that do not contain a STAR67 construct.

In conclusion, STAR67 increases expression from three different, unrelated promoters.

### Example 3: STAR67 enhances the expression level from CMV, EF1α and UB6 promoters in stably transfected PER.C6 cells.

We tested whether the presence of STAR67 adjacent of the CMV, EF1α and UB6 promoters influences the expression level of these promoters in another cell type than CHO cells, namely human PER.C6 cells. The same constructs as in example 1 were used.

### Materials and Methods

### Transfection, culturing and analysis of PER. C6 cells

PER.C6® cells were cultured in DMEM medium + pyridoxine + 9% Foetal Bovine Serum (Non-Heat Inactivated), 8.9 mM MgCl₂ 100 U/ml penicillin, and 100 mierograms/ml streptomycin at 37°C/10% CO₂. Cells were transfected with the plasmids using Lipofectamine 2000 (Invitrogen) as described by the manufacturer. Briefly, cells were seeded to 6-wells and grown overnight to 70-90% confluence. Lipofectamine reagent was combined with plasmid DNA at a ratio of 15 microliters per 3 microgram (e.g. for a 10 cm Petri dish, 20 micrograms DNA and 120 microliters Lipofectamine) and added after 30 minutes incubation at 25°C to the cells. After 6-hour incubation the transfection mixture was replaced with fresh medium, and the transfected cells were incubated further. After overnight cultivation, cells were trypsinized and seeded (1:15, 1:30, 1:60, 1:120 dilutions) into fresh petri dishes (90 mm) with fresh medium with zeocin added to a concentration of 100 µg/ml and the cells were cultured further. When colonies became visible, individual clones were isolated by scraping and transferred to 24-well plates in medium with zeocin. When grown to ∼70% confluence, cells were transferred to 6-well plates. Stable colonies were expanded for 2 weeks in 6-well plates before the d2EGFP signal was determined on a XL-MCL Beckman Coulter flowcytometer. The mean of the d2EGFP signal was taken as measure for the level of d2EGFP expression. Colonies were measured for a second time after 2 weeks. Thereafter colonies were further cultured in the absence of zeocin.

### Results

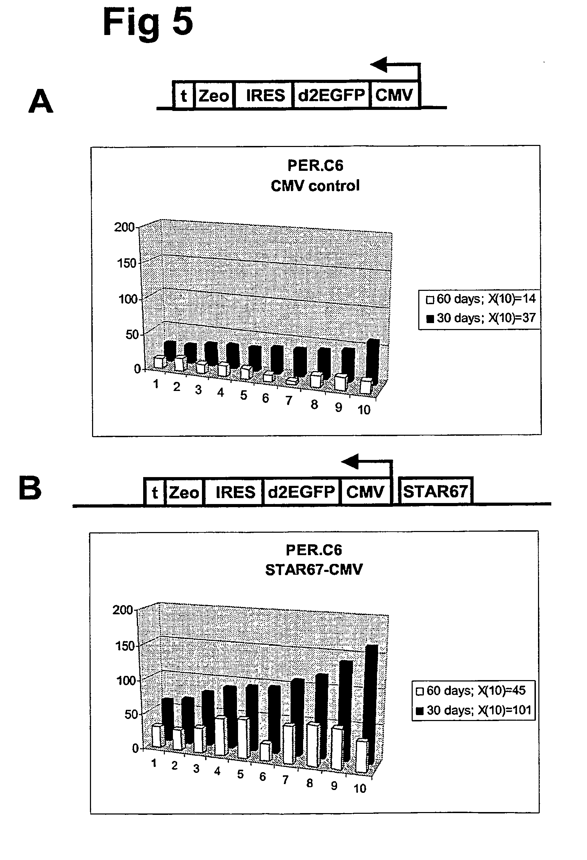

Fig. 5 shows that transfection of the construct that contains STAR67 cloned upstream of the CMV promoter resulted in a number of PER.C6 colonies that express significantly higher levels of d2EGFP protein, as compared to the "empty" control without STAR67, CMV Control. The average of the d2EGFP signal in the 10 colonies transfected with the CMV Control plasmid was 37, when measured 30 days after transfection. 60 days after transfection the average of the d2EGFP signal in these 10 colonies was reduced to 14, indicating that expression was not stable over time. In comparison, the average of the d2EGFP signal in the 10 colonies transfected with the STAR67-CMV plasmid was 101 when measured after 30 days and 45 measured 60 days after transfection. Hence 60 days after transfection, a STAR67-encompassing CMV construct conveyed a factor 3.2 higher CMV promoter driven expression level of the reporter protein in stably transfected clones.

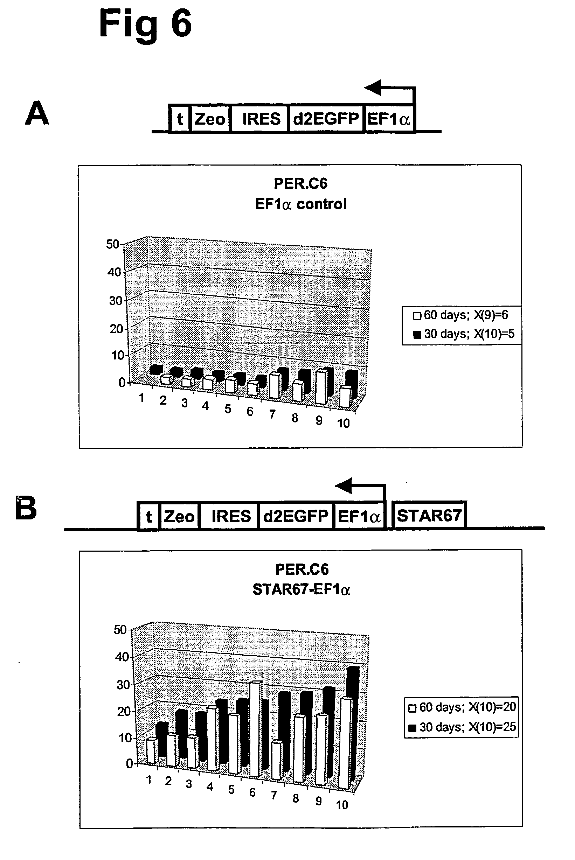

Fig. 6 shows that transfection of the construct that contains STAR67 cloned upstream of the EF1α- promoter resulted in a number of PER.C6 colonies that express significantly higher levels of d2EGFP protein, as compared to the "empty" control without STAR67, EF1α Control. The average of the d2EGFP signal in the 10 colonies transfected with the BF1α Control plasmid was 5, when measured 30 days after transfection. 60 days after transfection the average of the d2EGFP signal in these 10 colonies was 6. In comparison, the average of the d2EGFP signal in the 10 colonies transfected with the EF1α-STAR67 plasmid was 25 when measured after 30 days and 20 measured 60 days after transfection. Hence, both after 30 and 60 days after transfection, a STAR67-encompassing EF1α construct conveyed a factor 4 higher EF1α promoter driven expression level of the reporter protein in stably transfected clones.

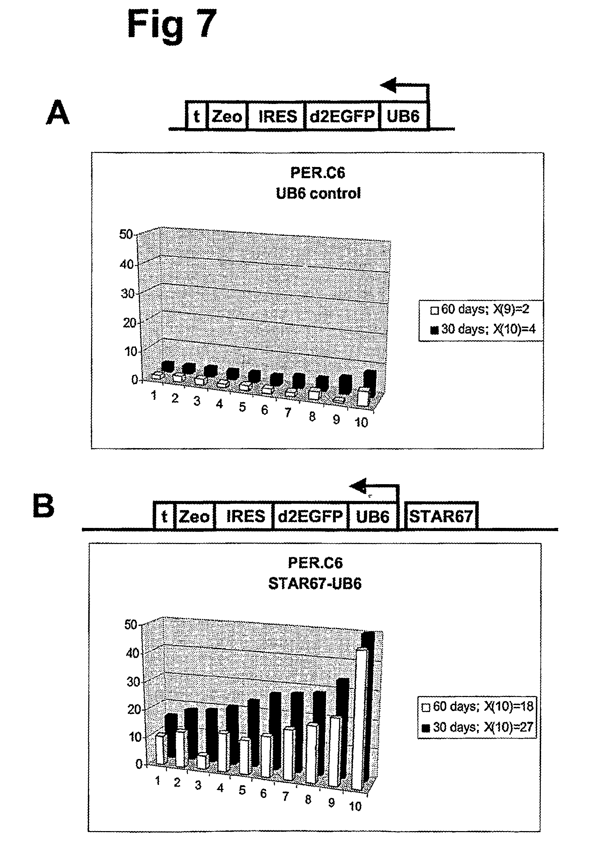

Fig. 7 shows that transfection of the construct that contains STAR67 cloned upstream of the UB6 promoter resulted in a number of PER.C6 colonies that express significantly higher levels of d2EGFP protein, as compared to the "empty" control without STAR67, UB6 Control. The average of the d2EGFP signal in the 10 colonies transfected with the UB6 Control plasmid was 4, when measured 30 days after transfection. 60 days after transfection the average of the d2EGFP signal in these 10 colonies was 2. In comparison, the average of the d2EGFP signal in the 10 colonies transfected with the UB6-STAR67 plasmid was 27 when measured after 30 days and 18 measured 60 days after transfection. Hence, both after 30 and 60 days after transfection, a STAR67-encompassing UB6 construct conveyed a factor 7 to 9 fold higher UB6 promoter driven expression level of the reporter protein in stably transfected clones.

Hence placing STAR67 upstream of the promoter resulted in significantly higher protein expression levels in comparison with STAR67-less constructs, also in PER.C6 cells. Hence STAR67 functions in different, unrelated cell types.

### Example 4: Novel configuration of STAR67 combined with other anti-repressor elements to enhance the SV40 promoter in CHO cells.

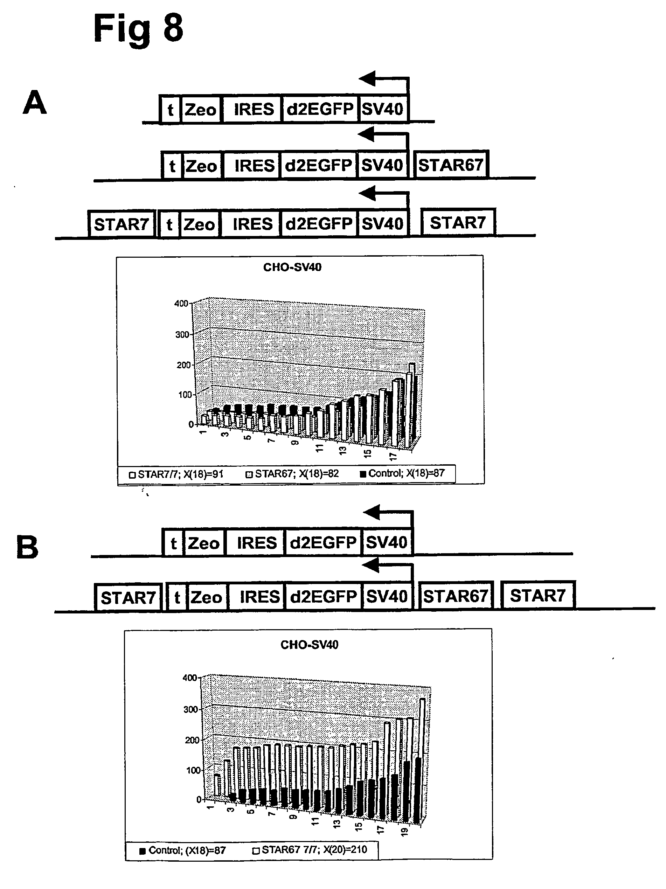

We tested whether the presence of STAR67 adjacent of the SV40 promoter influences the expression level of this promoter, either alone or in combination with another anti-repressor element, in this example STAR7. The constructs that were used for this purpose (see Fig. 8), are:
1 SV40-d2EGFP-ires-Zeo (SV40 Control)
2 STAR67-SV40-d2EGFP-ires-Zeo (SV40-STAR67)
3 STAR7-SV40-d2EGFP-ires-Zeo-STAR7 (SV40-STAR7/7)
4 STAR7- STAR67-SV40--d2EGFP-ires-Zeo-STAR7 (SV40-STAR67 7/7)

### Materials and Methods

The SV40 promoter was amplified by PCR with pIRES as template using primers TTGGTTGGGGCGCGCCGCAGCACCATGGrCCTCAAATAACCTCTGAAAGAG G (SEQ. ID. NO. 81) and
TTGGTTGGGAGCTCAAGCTTTTTGCAAAAGCCTAGGCCTCCAAAAAAGCC TCCTC (SEQ. ID. NO. 82). The PCR fragment was cloned in the AscI and SacI sites of CMV-d2EGFP-IRES-Zeo, from which the CMV promoter was removed.

CHO cells were transfected, colonies were isolated and propagated and analysed as in Example 2.

### Results

Fig. 8 shows that transfection of the construct that either contains STAR67 cloned upstream of the SV40 promoter (SV40-STAR67) or STAR7 cloned to flank the entire construct (SV40-STAR 7/7) did not result in CHO colonies that express significantly higher levels of d2EGFP protein, as compared to the "empty" control without anti-repressor elements (SV40 Control). The average of the d2EGFP signal in the 18 colonies transfected with the SV40 Control plasmid was 86, when measured 40 days after transfection. In comparison, the average of the d2EGFP signal in the 18 colonies transfected with the SV40-STAR67 plasmid was 82 when measured after 40 days and the average of the d2EGFP signal in the 18 colonies transfected with the SV40-STAR 7/7 plasmid was 91 when measured after 40 days. Hence, no significant effect of these anti-repressor elements on the SV40 promoter in CHO cells was observed.

It appears that the expression levels from the SV40 promoter in these cells are already quite high, and even much higher than those observed with the CMV promoter, which was considered to be a very strong promoter. This high background expression in the absence of an anti-repressor element using the SV40 promoter in CHO cells, may explain why no significant effect of STAR67 alone, or STAR7 flanking the transgene, was observed.

However, the average of the d2EGFP signal in the 18 colonies transfected with the SV40-STAR67 7/7 plasmid was 209 when measured after 40 days colonies, which is a factor 2.4 higher than the average of the 18 control colonies (86). Hence, when the STAR67 element is used in combination with another anti-repressor element, this results in a number of stably transfected CHO colonies that show significantly higher d2EGFP expression levels.

Therefore in this novel configuration (5'- STAR sequence A - STAR sequence C - promoter - nucleic acid encoding a protein of interest - STAR sequence B-3', wherein in the present example STAR sequences A and B are STAR 7 and STAR sequence C is STAR67) STAR elements appear to function even better than in hitherto disclosed configurations.

We have done experiments in which the flanking STAR7 elements were replaced by flanking STAR6 (SEQ. ID. NO. 6) elements or by flanking STAR4 (SEQ. ID. NO. 4) elements, in combination with STAR67 upstream of the SV40 promoter (SV40-STAR67 6/6 and SV40-STAR67 4/4, respectively, using the same nomenclature as above), and observed improved expression also with these combinations. This proves that the flanking STAR7 elements can be exchanged for other STAR sequences, and still the improvement of the novel configuration of the expression cassette with the STAR sequences is observed.

### Example 5: A combination of STAR67 and STAR7 enhance UB6-driven antibody expression levels in stably transfected CHO cells.

In example 4 we showed that the combination of STAR67 and STAR7 enhanced the expression levels of d2EGFP protein in CHO cells. Here we tested whether the combination of STAR67 and STAR7 could be used for the production of an antibody. We chose an antibody against the EpCAM molecule (Huls et al, 1999) as test protein and used the UB6 promoter.

### Materials and Methods

### Plasmids

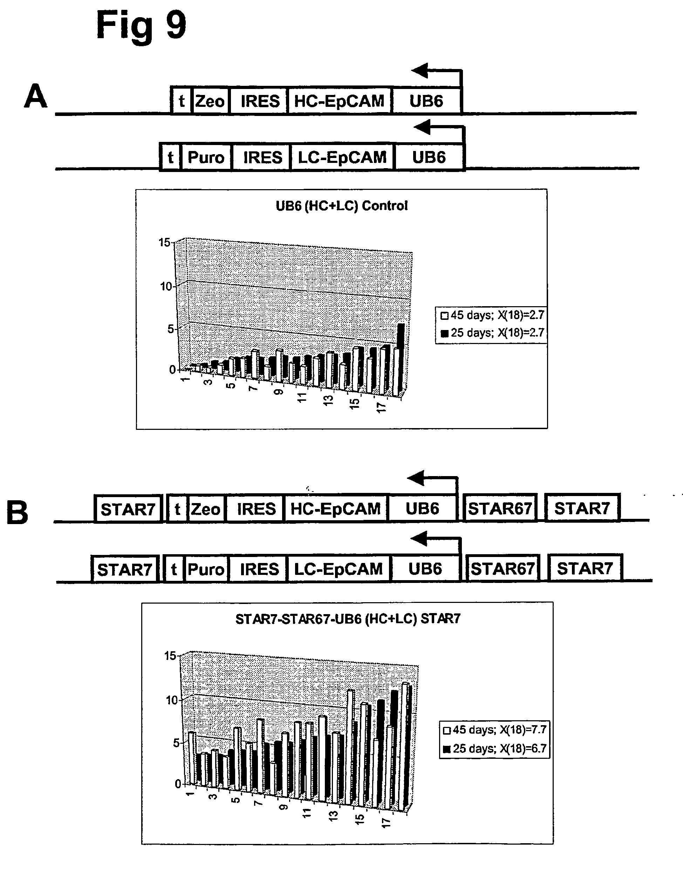

The heavy chain cDNA (HC-EpCAM) was cloned in a construct encompassing the UB6 promoter. The HC-EpCAM was coupled to the Zeocin resistance gene by an IRES sequence. The light chain cDNA (LC-EpCAM) was also cloned in a construct encompassing the UB6 promoter. The LC-EpCAM was coupled to the puromycin resistance gene by an IRES sequence. Together these two plasmids represent the UB6 (HC+LC) Control (Fig. 9)

To test the effects of STAR67 and STAR7, STAR67 was cloned in both HC+LC constructs, upstream of the UB6 promoters. STAR7 was cloned to flank the entire cassettes, both at the 5' and 3' end (Fig. 9). These two plasmids represent STAR7-STAR67-UB6 (HC+LC) STAR7.

### Transfection and culture of CHO cells

The Chinese Hamster Ovary cell line CHO-K1 (ATCC CCL-61) was transfected and cultured as in example 2, using zeocin (100 µg/ml) and puromycin (2.5 µg/ml) for selection. One day after transfection, zeocin was added to the culture medium. When first colonies became visible (approximately 7 days after addition of zeocin) the culture medium was removed and replaced with culture medium containing puromycin. After approximately 7 days, colonies were isolated and transferred to 24 wells plates, in culture medium containing zeocin only.

### Results

Fig. 9 shows that that transfection of antibody constructs that contain STAR67 cloned upstream of the UB6 promoter and two STAR7 elements cloned to flank the entire cassettes resulted in a number of CHO colonies that express significantly higher levels of EpCAM antibody (measured by ELISA using an anti-human IgG antibody) as compared to the "empty" control without STAR67 and STAR7, UB6 (HC+LC) Control. The average of the EpCAM production in the 18 colonies transfected with the UB6 (HC+LC) Control plasmid was 2.7 pg/cell/day, when measured 25 days after transfection. Selection agents zeocin and puromycin were removed after 25 days. 45 days after transfection the average of the EpCAM production in these 18 colonies was 2.7 pg/cell/day. In comparison, the average of the EpCAM production signal in the 18 colonies transfected with the STAR7-STAR67-UB6 (HC+LC)-STAR7 plasmid was 6.7 when measured after 25 days and 7.7 pg/cell/day, when measured 45 days after transfection. Hence, both after 30 and 45 days after transfection, a STAR67/STAR7-encompassing UB6 construct conveyed a factor 2.5 to 2.9 fold higher UB6 promoter driven EpCAM expression level in stably transfected CHO clones.

Hence placing STAR67 upstream of the promoter and two STAR7 elements to flank the cassettes resulted in significantly higher EpCAM antibody expression levels in CHO cells, in comparison with STAR67/STAR7-less constructs.

### Example 6: STAR67 is not an enhancer blocker, whereas STAR6 and STAR7 are.

All hitherto known STAR elements that were tested for that property, including STAR6 and STAR7, are enhancer blockers (WO 03/004704, Kwaks et al, 2003). Enhancer blocker activity is tested by placing a STAR element between a strong enhancer and a promoter. Here we tested whether also STAR67 is an enhancer blocker.

### Materials and Methods

The d2EGFP gene was PCR-amplified using primers TTGGTTGGTCATGAATGGTGAGCAACrGGCGAGGAGCTGTTC (SEQ.ID.NO. 75) and ATTCTCTAGACTACACATTGATCCTAGCAGAAGCAC (SEQ.ID.NO. 76) and cloned into plasmid pGL3-promoter (Promega) using the NcoI and XbaI restriction sites to replace the Luciferase gene to create plasmid pGL3-promoter-GFP. A linker (created by annealing oligo's
CGATATCTTGGAGATCTACTAGTGGCGCGCCTTGGGrCTAGCT (SEQ.ID.NO. 77) and
GATCAGCTAGCCCAAGGCGCGCCACTAGTAGATCTCCAAGATATCGAGCT (SEQ.ID.NO. 78), was cloned in the SacI and BglII sites to create multiple cloning sites. The original BglII site was destroyed upon ligation of the linker, creating a new unique BglII site within the linker DNA. The SV40-enhancer was cut from plasmid pGL3-basic (Promega) using BsaBI and BamHI and cloned into the pGL3-linker-promoter-GFP using the EcoRV and BglII sites creating plasmid pGL3-enhancer-promoter-GFP. The STAR40 element (SEQ. ID. NO. 40) was placed upstream of the SV40 enhancer using KpnI and SacI sites to prevent action of the enhancer on upstream sequences. Finally, anti-repressor elements STAR6, STAR7 and STAR67 were placed in between the SV40 enhancer and the SV40 minimal promoter using the SpeI and AscI restriction sites.

### Transfection and culture of CHO cells

The Chinese Hamster Ovary cell line CHO-K1 (ATCC CCL-61) is transfected as in example 2. One day after the transfection, d2EGFP levels are measured on an Epics XL flowcytometer (Beckman Coulter)

### Results

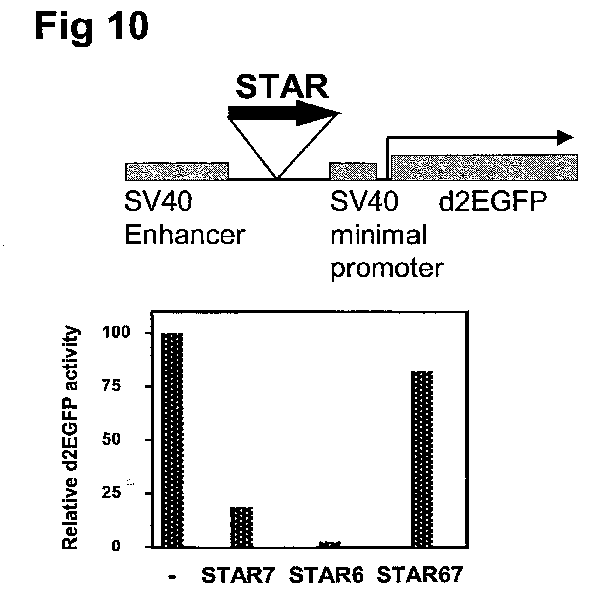

Fig. 10 shows that STAR67 is not an enhancer blocker, whereas STAR6 and STAR7 act enhancer blockers in the same assay. STAR6, STAR7 and STAR67 were cloned between the SV40 enhancer and a minimal SV40-promoter upstream of the d2EGFP gene. When no STAR element was cloned between the enhancer and the promoter strong transcriptional activation occurred (arbitrarily set at 100%). When STAR6 or STAR7 was placed between the enhancer and the promoter, transcription dropped to background levels, indicating that STAR6 and STAR7 are potent enhancer blockers. In contrast, when STAR67 was cloned between the enhancer and the promoter relative transcription levels were still 80% of the control, indicating that STAR67 is not a good enhancer blocker, this in contrast with STAR6 and STAR 7, as well as other anti-repressor elements, as previously described (WO 03/004704, Kwaks et al, 2003).

### Example 7: STAR67 enhances UB6 and CMV-driven antibody expression levels in stably transfected CHO cells.

In example 5 we showed that the combination of STAR67 and STAR7 enhanced the expression levels of EpCAM antibody in CHO cells, in the context of two distinct plasmids, which contained the heavy and light chains. In this example we tested whether STAR67 could be used for the production of EpCAM antibody when both heavy and light chains are placed on one plasmid. We used simultaneous selection for each selectable marker.

### Materials and Methods

### Plasmids

The heavy chain cDNA (HC-EpCAM) is under the control of the UB6 promoter and coupled to the Zeocin resistance gene by an IRES sequence. The light chain cDNA (LC-EpCAM) is under control of the CMV promoter and coupled to the puromycin resistance gene by an IRES sequence. Basically these are the constructs used in example 5. These two expression cassettes were placed on one plasmid, in such a manner that transcription of the two expression units had opposite directions. In the control plasmid the UB6 and CMV promoters were separated by a stuffer of 500 bp (EpCAM Control) (Fig. 10). In another plasmid STAR67 was placed between the UB6 and CMV promoter (EpCAM STAR67) (Fig. 10).

### Transfection and culture of CHO cells

The Chinese Hamster Ovary cell line CHO-K1 (ATCC CCL-61) was transfected and cultured as in example 2, using zeocin (100 µg/ml) and puromycin (2.5 µg/ml) for selection. In example 5, consecutive selection for both selection markers was used. In contrast, here both selection agents were present in the culture medium simultaneously. One day after transfection, zeocin and puromycin were added to the culture medium. The selection medium was present until colonies were isolated (approximately 14 days after transfection). After colonies were isolated and transferred to 24-wells plates, the cells were cultured in the presence of zeocin and puromycin.

### Results

Fig. 11 shows that that transfection of the antibody construct that contains STAR67 cloned between the UB6 and CMV promoters resulted in a number of CHO colonies that express EpCAM antibody (measured by ELISA using an anti-human IgG antibody). The average of the EpCAM production in the 19 colonies transfected with the EpCAM STAR67 plasmid was 9.8 pg/cell/day, when measured 25 days after transfection.

In contrast, surprisingly no colonies survived of the transfection with the EpCAM Control plasmid. When selection was performed with either zeocin or puromycin alone, EpCAM Control colonies survived. However, when the selection pressure was increased by placing selection pressure on both the heavy and light chain, these conditions allowed only colonies to survive that have a STAR67 present in the transfected plasmid.

The results also show that incorporation of STAR67 has a beneficial effect on two promoters, the UB6 and CMV promoters, that are placed upstream and downstream of one STAR67 element. This indicates that STAR67 may operate in a bi-directional fashion.

The difference between the EpCAM control and EpCAM STAR67 plasmid is black-white in the sense that only transfection of EpCAM STAR67 results in the establishment of colonies, when the selection pressure is high. This opens an opportunity to use this plasmid configuration for identifying the region in STAR67 that is responsible for mediating this effect. Smaller, overlapping portions of STAR67 are placed between the UB6 and CMV promoters, driving the EpCAM molecule. When a portion of STAR67 is functional, colonies will survive when both zeocin and puromycin are simultaneously used as selection agent. When a portion of STAR67 is not functional, no colonies will survive under identical selection conditions.

Hence placing STAR67 upstream of the promoter resulted in significantly higher EpCAM antibody expression levels in CHO cells, in comparison with constructs lacking such anti-repressor elements.
A similar experiment is performed with the SV40 promoter to drive expression of the heavy and light chains of the anti-EpCAM antibody. In other experiments, STAR67 is exchanged for other STAR sequences. In further experiments, the orientation of the two expression units encoding the heavy and light chains is changed such that both are in the same direction. In another experiment, the expression units for the heavy and light chains are placed on different nucleic acid molecules respectively (as in example 5), and the resulting clones are simultaneously selected for both selectable markers. In another experiment, the host cell type is varied. Combinations of these variations are made.

### DESCRIPTION OF FIGURES

**Fig. 1.** Schematic diagram of the invention.
   A) bicistronic gene containing (from 5' to 3') a transgene (encoding for example the d2EGFP gene), an IRES, and a selectable marker (zeo, conferring zeocin resistance) under control of the CMV promoter. The expression unit has the SV40 transcriptional terminator at its 3' end (t). The name of the construct is CMV-d2EGFP-ires-Zeo (CMV Control).
   B) construct as in A, but now STAR 67 is cloned upstream of the CMV promoter. The name of the construct is STAR67-CMV-d2EGFP-ires-Zeo (CMV-STAR67).
   C) construct as in B, but upstream and downstream STAR7 elements are cloned to flank the entire construct. The name of the construct is STAR7-STAR67-CMV-d2EGFP-ires-Zeo-STAR7 (CMV-STAR67 7/7)
**Fig. 2.** STAR67 improves CMV driven d2EGFP expression in CHO cells. The mean of the d2EGFP signal for 10 independent stable colonies is plotted for the indicated constructs in CHO cells. A) CMV Control; B) STAR67-CMV. X(10): average d2EGFP expression levels of the 10 colonies. See example 2 for details.
**Fig. 3.** STAR67 improves EF1 α driven d2EGFP expression in CHO cells. Same as Fig. 2, but now with EF1α promoter. A) EF1α Control; B) STAR67- EF1α.
**Fig. 4.** STAR67 improves UB6 driven d2EGrFP expression in CHO cells. Same as Figs. 2 and 3, but now with UB6 promoter. A) UB6 Control; B) STAR67-UB6.
**Fig. 5.** STAR67 improves CMV driven d2EGFP expression in PER.C6 cells. Same as Fig. 2, but now in PER.C6 cells. A) CMV Control; B) STAR67-CMV.
**Fig. 6.** STAR67 improves EF1α driven d2EGFP expression in PER.C6 cells. Same as Fig. 3, but now in PER.C6 cells. A) EF1α Control; B) STAR67- EF1α.
**Fig. 7.** STAR67 improves UB6 driven d2EGFP expression in PER.C6 cells. Same as Fig. 4, but now in PER.C6 cells. A) UB6 Control; B) STAR67-UB6.
**Fig. 8.** STAR67 improves SV40 driven d2EGFP expression in CHO-K1 cells in combination with another STAR element. Similar as Fig. 2, but now using the SV40 promoter in CHO cells, and the indicated constructs. The mean of the d2EGFP signal is plotted for 18-20 independent stable colonies 60 days after transfection. A) SV40 Control, SV40-STAR67, SV40-STAR7/7; B) SV40 Control and SV40-STAR67 7/7. See example 4 for details.
**Fig. 9.** STAR67 improves UB6 driven expression levels of the EpCAM antibody in CHO-K1 cells. See example 5 for details. A) constructs without STAR elements; B) constructs with STAR67 upstream of promoter and flanking STAR7 elements. The anti-EpCAM antibody concentration is presented as pg/cell/day. X(18): average production level of the 18 colonies.
**Fig. 10.** STAR67 is not an enhancer blocker, whereas STAR 6 and 7 are. See example 6 for details.
**Fig. 11.** STAR67 enhances UB6 and CMV-driven antibody expression levels in stably transfected CHO cells. See example 7 for details. A) single DNA molecule containing anti-EpCAM heavy chain (HC) and light chain (LC), each behind a promoter, and each linked to a different selectable marker gene (simultaneous selection was used for both markers): construct without STAR elements. No colonies were found; B) same construct with STAR67 between the two promoters. The anti-EpCAM antibody concentration is presented as pg/cell/day. X(19): average production level of the 19 colonies.

### References

Boshart, M, Weber, F, Jahn, G, Dorsch-Hasler, K, Fleckenstein, B, and Schaffner, W. (1985) A very strong enhancer is located upstream of an immediate early gene of human cytomegalovirus Cell 41, 521-530.
Chung JH, Whiteley M, and Felsanfeld G. (1993) A 5' element of the chicken beta-globin domain serves as an insulator in human erythroid cells and protects against position effect in Drosophila. Cell 74: 505-514.
Chung JH, Bell AC, Felsenfeld G. (1997). Characterization of the chicken beta-globin insulator. Proc Natl Acad Sci USA 94: 575-580.
Das, GC, Niyogi, SK, and Salzman, NP. (1985) SV40 promoters and their regulation Prog Nucleic Acid Res Mol Biol 32, 217-236.
Gill DR, Smyth SE, Goddard CA, Pringle IA, Higgins CF, Colledge WH, and Hyde SC. (2001) Increased persistence of lung gene expression using plasmids containing the ubiquitin C or elongation factor 1α promoter. Gene Therapy 8: 1539-1546.
Gossen M, and Bujard H. (1992) Tight control of gene expression in mammalian cells by tetracycline-responsive promoters. Proc Natl Acad Sci USA 89: 5547-5551.
Graham FO, Smiley J, Russell W and Nairn R. (1977). Characteristics of a human cell line transformed by DNA from human adenovirus type 5. J. Gen. Virol. 36, 59-72.
Huls GA, Heijnen IAFM, Cuomo ME, Koningsberger JC, Wiegman L, Boel E, van der Vuurst-de Vries A-R, Loyson SAJ, Helfrich W, van Berge Henegouwen GP, van Meijer M, de Kruif J, Logtenberg T. (1999). A recombinant, fully human monoclonal antibody with antitumor activity constructed from phage-displayed antibody fragments. Nat Biotechnol. 17, 276-281.
Jones D, Kroos N, Anema R, Van Montfort B, Vooys A, Van Der Kraats S, Van Der Helm E, Smits S, Schouten J, Brouwer K, Lagerwerf F, Van Berkel P, Opstelten D-J, Logtenberg T, Bout A (2003) High-level expression of recombinant IgG in the human cell line PER.C6. Biotechnol. Prog. 19: 163-168.
Kaufman, RJ. (2000) Overview of vector design for mammalian gene expression Mol Biotechnol 16, 151-160.
Kaufman, RJ, and Sharp, PA. (1982) Construction of a modular dihydrofolate reductase cDNA gene: analysis of signals utilized for efficient expression Mol Cell Biol 2, 1304-1319.
Kellum R, and Schedl P. (1991) A position-effect assay for boundaries of higher order chromosomal domains. Cell 64: 941-950.
Kwaks TH, Barnett P, Hemrika W, Siersma T, Sewalt RG, Satijn DP, Brons JF, van Blokland R, Kwakman P, Kruckeberg AL, Kelder A, Otte AP. (2003) Identification of anti-repressor elements that confer high and stable protein production in mammalian cells. Nat Biotechnol 21, 553-558. Erratum in: Nat Biotechnol 21, 822 (2003).
Phi-Van L, Von Kreis JP, Ostertag W, and Strätling WH. (1990) The chicken lysozyme 5' matrix attachment region increases transcription from a heterologous promoter in heterologous cells and dampens position effects on the expression of transfected genes. Mol. Cell. Biol. 10: 2302-2307.
Lopez de Quinto, S, and Martinez-Salas, E. (1998) Parameters influencing translational efficiency in aphthovirus IRES- based bicistronic expression vectors Gene 217, 51-56.
Martin, DI, and Whitelaw, E. (1996) The vagaries of variegating transgenes Bioessays 18, 919-923.
Martinez-Salas, E. (1999) Internal ribosome entry site biology and its use in expression vectors Curr Opin Biotechnol 10, 458-464.
McBurney, MW, Mai, T, Yang, X, and Jardine, K. (2002) Evidence for repeat-induced gene silencing in cultured Mammalian cells: inactivation of tandem repeats of transfected genes Exp Cell Res 274, 1-8.
Migliaccio, AR, Bengra, C, Ling, J, Pi, W, Li, C, Zeng, S, Keskintepe, M, Whitney, B, Sanchez, M, Migliaccio, G, and Tuan, D. (2000) Stable and unstable transgene integration sites in the human genome: extinction of the Green Fluorescent Protein transgene in K562 cells Gene 256, 197-214.
Mizuguchi, H, Xu, Z, Ishii-Watabe, A, Uchida, E, and Hayakawa, T. (2000) IRES-dependent second gene expression is significantly lower than cap- dependent first gene expression in a bicistronic vector Mol Ther 1, 376-382.
Rees, S, Coote, J, Stables, J, Goodson, S, Harris, S, and Lee, MG. (1996) Bicistronic vector for the creation of stable mammalian cell lines that predisposes all antibiotic-resistant cells to express recombinant protein Biotechniques 20, 102-104, 106, 108-110.
Schorpp, M, Jager, R, Schellander, K, Schenkel, J, Wagner, EF, Weiher, H, and Angel, P. (1996) The human ubiquitin C promoter directs high ubiquitous expression of transgenes in mice Nucleic Acids Res 24, 1787-8.
Strutzenberger, K, Borth, N, Kunert, R, Steinfellner, W, and Katinger, H. (1999) Changes during subclone development and ageing of human antibody- producing recombinant CHO cells J Biotechnol 69, 215-26.
Van der Vlag, J, den Blaauwen, JL, Sewalt, RG, van Driel, R, and Otte, AP. (2000) Transcriptional repression mediated by polycomb group proteins and other chromatin-associated repressors is selectively blocked by insulators J Biol Chem 275, 697-704.
Whitelaw, E, Sutherland, H, Kearns, M, Morgan, H, Weaving, L, and Garrick, D. (2001) Epigenetic effects on transgene expression Methods Mol Biol 158, 351-68.

## Claims

1. A recombinant nucleic acid molecule comprising a nucleic acid sequence having anti-repressor activity selected from the group consisting of:
a) SEQ. ID. NO. 66,
b) fragments of SEQ. ID. NO. 66 wherein said fragment has anti-repressor activity,
c) sequences that are at least 70% identical in nucleotide sequence to a) or b) wherein said sequences have anti-repressor activity; and
d) the complement to any one of a) to c);
said recombinant nucleic acid molecule further comprising an expression cassette, said expression cassette comprising a heterologous promoter linked to a nucleic acid of interest, and wherein said nucleic acid of interest preferably encodes all or part of a protein of interest.

2. A molecule according to claim 1, wherein said nucleic acid sequence having anti-repressor activity is situated upstream of said promoter in said expression cassette.

3. A molecule according to claim 2, wherein said sequence having anti-repressor activity and said promoter are separated by less than 2 kb.

4. A molecule according to any one of the preceding claims, wherein said nucleic acid encoding a protein of interest is present in a multicistronic gene further encoding a selectable marker gene.

5. A molecule according to any one of the preceding claims, further comprising at least one other sequence having anti-repressor activity, said at least one other sequence being chosen from
a) any of SEQ. ID. NOs. 1-65,
b) fragments of any of SEQ. ID. NOs. 1-65, wherein said fragments have anti-repressor activity, and
c) sequences that are at least 70% identical in nucleotide sequence to a) or b) wherein said sequences have anti-repressor activity, and
d) the complement to any one of a) to c).

6. A recombinant nucleic acid molecule comprising an expression cassette comprising: 5'- anti-repressor sequence A - promoter - nucleic acid encoding all or part of a protein of interest - anti-repressor sequence B-3' wherein anti-repressor sequences A and B may be the same or different and are chosen from the group consisting of
(i) any of SEQ. ID. NOs. 1-65,
(ii) fragments of any of SEQ. ID. NOs. 1-65, wherein said fragments have anti-repressor activity,
(iii) sequences that are at least 70% identical in nucleotide sequence to a) or b) wherein said sequences have anti-repressor activity, and
(iv) the complement to any one of (i) to (iii), **characterized in that** said expression cassette further comprises between said anti-repressor sequences A and B a sequence having anti-repressor activity chosen from the group consisting of
a) SEQ. ID. NO. 66,
b) fragments of SEQ. ID. NO. 66 wherein said fragment has anti-repressor activity,
c) sequences that are at least 70% identical in nucleotide sequence to a) or b) wherein said sequences have anti-repressor activity; and
d) the complement to any one of a) to c).

7. A cell comprising a molecule according to any one of claims 1-6.

8. A cell according to claim 7, which is a mammalian cell.

9. A cell according to claim 8, which is a CHO cell.

10. A method for producing a protein of interest, comprising culturing a cell comprising a recombinant nucleic acid molecule encoding the protein of interest to express said nucleic acid encoding the protein of interest in said cell,
**characterized in that**
said recombinant nucleic acid molecule comprises a nucleic acid sequence having anti-repressor activity selected from the group consisting of:
a) SEQ. ID. NO. 66,
b) fragments of SEQ. ID. NO. 66 wherein said fragment has anti-repressor activity,
c) sequences that are at least 70% identical in nucleotide sequence to a) or b) wherein said sequences have anti-repressor activity; and
d) the complement to any one of a) to c).

11. A method according to claim 10, further comprising isolating said protein of interest.

12. A method according to claim 10 or 11, wherein said nucleic acid sequence having anti-repressor activity is situated upstream of a promoter that controls expression of the protein of interest.

13. A method according to claim 12, wherein said sequence having anti-repressor activity and said promoter are separated by less than 2 kb.

14. A method according to any one of claims 10 to 13, wherein said nucleic acid encoding a protein of interest is present in a multicistronic gene further encoding a selectable marker gene.

15. A method according to any one of claims 10 to 14, wherein said cell is a mammalian cell.

16. A method according to claim 15, wherein said cell is a CHO cell.

17. Use of a nucleic acid sequence having anti-repressor activity selected from the group consisting of:
a) SEQ. ID. NO. 66,
b) fragments of SEQ. ID. NO. 66 wherein said fragment has anti-repressor activity,
c) sequences that are at least 70% identical in nucleotide sequence to a) or b) wherein said sequences have anti-repressor activity; and
d) the complement to any one of a) to c),
for increasing expression of a nucleic acid of interest.

18. A method for generating a host cell expressing two polypeptides of interest, the method comprising:
a) introducing into a host cell one or more nucleic acid molecules, the nucleic acid molecule or molecules together comprising:
(i) a promoter functionally linked to a sequence encoding a first polypeptide of interest and a first selectable marker gene, and
(ii) a promoter functionally linked to a sequence encoding a second polypeptide of interest and a second selectable marker gene, and
(iii) at least one sequence having anti-repressor activity, chosen from the group consisting of
(a) SEQ. ID. NO. 66,
(b) fragments of SEQ. ID. NO. 66, said fragments having anti-repressor activity,
(c) sequences that are at least 70% identical to (a) or (b) and having anti-repressor activity, and
(d) the complement of any one of (a)-(c);
b) selecting a host cell by selecting essentially simultaneously for expression of said first and second selectable marker genes.

19. A method for expressing two polypeptides of interest, the method comprising:
culturing a host cell obtained by the method according to claim 18 to express said first and second polypeptides, and optionally isolating said polypeptides.

20. A method according to claim 18 or 19, wherein said two polypeptides are part of a multimeric protein.

## Patentansprüche

1. Rekombinantes Nucleinsäuremolekül, umfassend eine Nucleinsäuresequenz, die Anti-Reprossor-Aktivität hat und ausgewählt ist aus der Gruppe bestehend aus:
a) SEQ ID NO: 66,
b) Fragmenten von SEQ ID NO: 66, wobei das Fragment Anti-Repressor-Aktivität hat,
c) Sequenzen, deren Nucleotidsequenz zu mindestens 70% identisch mit a) oder b) ist, wobei die Sequenzen Anti-Repressor-Aktivität haben; und
d) dem Komplement zu einer der Sequenzen von a) bis c),
wobei das rekombinante Nucleinsäuremolekül des weiteren eine Expressionskassette umfasst, wobei die Expressionskassette einen heterologen Promoter umfasst, der mit einer Nucleinsäure von Interesse verbunden ist, und wobei die Nucleinsäure von Interesse vorzugsweise alles oder einen Teil eines Proteins von Interesse codiert.

2. Molekül nach Anspruch 1, wobei die Nucleinsäuresequenz, die Anti-Repressor-Aktivität hat, stromaufwärts von dem Promoter in der Expressionskassette gelegen ist.

3. Molekül nach Anspruch 2, wobei die Sequenz, die Anti-Repressor-Aktivität hat, und der Promoter durch weniger als 2 kb voneinander getrennt sind.

4. Molekül nach einem der vorangegangenen Ansprüche, wobei die Nucleinsäure, die ein Protein von Interesse codiert, in einem multicistronischen Gen vorliegt, das des weiteren ein selektierbares Markergen codiert.

5. Molekül nach einem der vorangegangenen Ansprüche, des Weiteren umfassend mindestens eine andere Sequenz, die Anti-Repressor-Aktivität hat,
wobei die mindestens eine andere Sequenz ausgewählt ist aus
a) irgendeiner der SEQ ID NOs: 1 - 65,
b) Fragmenten irgendeiner der SEQ ID NOs: 1 - 65, wobei die Fragmente Anti-Repressor-Aktivität haben, und
c) Sequenzen, deren Nucleotidsequenz zu mindestens 70% identisch mit a) oder b) ist, wobei die Sequenzen Anti-Repressor-Aktivität haben, und
d) dem Komplement zu einer der Sequenzen von a) bis c).

6. Rekombinantes Nucleinsäuremolekül, umfassend eine Expresstonskassette umfassend
5'- Anti-Repressor-Sequenz A - Promotor - Nucleinsäure, die alles oder einen Teil eines Proteins von Interesse codiert- Anti-Reprossor-Sequenz B - 3', wobei die Anti-Repressor-Sequenzen A und B gleich oder unterschiedlich sein können und ausgewählt sind aus der Gruppe bestehend aus
(i) einer der SEQ ID NOs: 1 - 65.
(ii) Fragmenten irgendeiner der SEQ ID NOs: 1 - 65, wobei die Fragmente Anti-Repressor-Aktivität haben,
(iii) Sequenzen, deren Nucleotidsequenz zu mindestens 70% Identisch mit a) oder b) ist, wobei die Sequenzen Anti-Repressor-Aktivität haben, und
(iv) dem Komplement zu einer der Sequenzen von (i) bis (iii);
**dadurch gekennzeichnet, dass** die Expressionskassette des weiteren zwischen den Anti-Repressor-Sequenzen A und B eine Sequenz umfasst, die Anti-Repressor-Aktivität hat und ausgewählt ist aus der Gruppe bestehend aus
a) SEQ ID NO: 66,
b) Fragmenten von SEQ ID NO: 66, wobei das Fragment Anti-Repressor-Aktivität hat,
c) Sequenzen, deren Nucleotidsequenz zu mindestens 70% identisch mit a) oder b) ist, wobei die Sequenzen Anti-Repressor-Aktivität haben, und
d) dem Komplement zu einer der Sequenzen von a) bis c).

7. Zelle, umfassend ein Molekül nach einem der Ansprüche 1 - 6.

8. Zelle nach Anspruch 7, die eine Säugerzelle ist.

9. Zelle nach Anspruch 8, die eine CHO-Zelle ist.

10. Verfahren zum Herstellen eines Proteins von Interesse, umfassend das Züchten einer Zelle, die ein das Protein von Interesse codierendes rekombinantes Nucleinsäuremolekül umfasst, um die Nucleinsäure, die das Protein von Interesse codiert, in der Zelle zu exprimieren,
**dadurch gekennzeichnet, dass**
das rekombinante Nucleinsäuremolekül eine Nucleinsäuresequenz umfasst, die Anti-Repressor-Aktivität hat und ausgewählt Ist aus der Gruppe bestehend aus:
a) SEQ ID NO: 66,
b) Fragmenten von SEQ ID NO: 66, wobei das Fragment Anti-Repressor-Aktivität hat,
c) Sequenzen, deren Nucleotidsequenz zu mindestens 70% identisch mit a) oder b) ist, wobei die Sequenzen Anti-Repressor-Aktivität haben, und
d) dem Komplement zu einer der Sequenzen von a) bis c).

11. Verfahren nach Anspruch 10, des Weiteren umfassend das Isolieren des Proteins von Interesse.

12. Verfahren nach Anspruch 10 oder 11, wobei die Nucleinsäuresequenz, die Anti-Repressor-Aktivität hat, stromaufwärts von einem Promotor, der die Expression des Proteins von Interesse kontrolliert, gelegen ist,

13. Verfahren nach Anspruch 12, wobei die Sequenz, die Anti-Repressor-Aktivität hat, und der Promoter durch weniger als 2 kb voneinander getrennt sind.

14. Verfahren nach einem der Ansprüche 10 bis 13, wobei die Nucleinsäure, die ein Protein von Interesse codiert, in einem multicistronischen Gen vorliegt, das des Weiteren ein selektierbares Markargen codiert.

15. Verfahren nach einem der Ansprüche 10 bis 14, wobei die Zelle eine Säugerzelle ist.

16. Verfahren nach Anspruch 15, wobei die Zelle eine CHO-Zelle ist.

17. Verwendung einer Nucleinsäuresequenz, die Anti-Repressor-Aktivität hat und ausgewählt ist aus der Gruppe bestehend aus:
a) SEQ ID NO: 66,
b) Fragmenten von SEQ ID NO: 66, wobei das Fragment Anti-Rapressor-Aktivität hat,
c) Sequenzen, deren Nucleotidsequenz zu mindestens 70% Identisch mit a) oder b) ist, wobei die Sequenzen Anti-Repressor-Aktivität haben, und
d) dem Komplement zu einer der Sequenzen von a) bis c),
zum Erhöhen der Expression einer Nucleinsäure von Interesse.

18. Verfahren zum Erzeugen einer Wirtszelle, die zwei Polypeptide von interesse exprimiert, wobei das Verfahren umfasst:
a) Einbringen eines oder mehrerer Nucleinsäuremoleküle in eine Wirtszelle, wobei das Nucleinsäuremolekül oder die Nucleinsäuremoleküle zusammen umfassen:
(i) einen Promotor, der funktionell verbunden ist mit einer Sequenz, die ein erstes Polypeptid von Interesse und ein erstes selektierbares Markergen codiert, und
(ii) einen Promotor, der funktionell verbunden ist mit einer Sequenz, die ein zweites Polypeptid von Interesse und ein zweites selektierbares Markergen codiert, und
(iii) mindestens eine Sequenz, die Anti-Repressor-Aktivität hat, ausgewählt aus der Gruppe bestehend aus
(a) SEQ ID NO: 66,
(b) Fragmenten von SEQ ID NO: 66, wobei die Fragmente Anti-Repressor-Aktivität haben,
(c) Sequenzen, deren Nucleotidsequenz zu mindestens 70% identisch mit a) oder b) ist, wobei die Sequenzen Anti-Repressor-Aktivität haben, und
(d) dem Komplement zu einer der Sequenzen von a) bis c).
b) Selektieren einer Wirtszelle durch im Wesentlichen gleichzeitiges Selektieren auf Expression des ersten und zweiten selektierbaren Markergens.

19. Verfahren zum Exprimieren zweier Polypeptide von Interesse, wobei das Verfahren umfasst:
Züchten einer Wirtszelle, die mit dem Verfahren nach Anspruch 18 erhalten wurde, um das erste und zweite Polypeptide zu exprimieren, und gegebenenfalls Isolieren der Polypeptide.

20. Verfahren nach Anspruch 18 oder 19, wobei die zwei Polypeptide Teil eines multimeren Proteins sind.

## Revendications

1. Molécule d'acide nucléique recombinant comprenant une séquence d'acides nucléique possédant une activité antirépresseur, choisie parmi le groupe constitué par :
a) SEQ ID NO: 66,
b) des fragments de SEQ ID NO: 66, lesdits fragments possédant une activité antirépresseur,
c) des séquences possédant une identité de séquence nucléotidique à concurrence d'au moins 70 % par rapport à a) ou à b), lesdites séquences possédant une activité antirépresseur ; et
d) le complément de l'un quelconque des éléments a) à c) ;
ladite molécule d'acide nucléique recombinant comprenant en outre une cassette d'expression, ladite cassette d'expression comprenant un promoteur hétérologue lié à un acide nucléique d'intérêt, ledit acide nucléique d'intérêt encodant de préférence la totalité ou une partie d'une protéine d'intérêt.

2. Molécule selon la revendication 1, dans laquelle ladite séquence d'acides nucléiques possédant une activité antirépresseur est située en amont dudit promoteur dans ladite cassette d'expression.

3. Molécule selon la revendication 2, dans laquelle ladite séquence possédant une activité antirépresseur et ledit promoteur sont séparés de moins de 2 kb.

4. Molécule selon l'une quelconque des revendications précédentes, dans laquelle ledit acide nucléique encodant une protéine d'intérêt est présent dans un gène polycistronique encodant en outre un gène marqueur sélectionnable.

5. Molécule d'acide nucléique recombinant selon l'une quelconque des revendications précédentes, comprenant en outre au moins une autre séquence possédant une activité antirépresseur, ladite au moins une autre séquence étant choisie parmi :
a) l'une quelconque des SEQ ID NO: 1 à 65,
b) des fragments de l'une quelconque des SEQ ID NO: 1 - 65, lesdits fragments possédant une activité antirépresseur, et
c) des séquences possédant une identité de séquence nucléotidique à concurrence d'au moins 70 % par rapport à a) ou à b), lesdites séquences possédant une activité antirépresseur ; et
d) le complément de l'un quelconque des éléments a) à c).

6. Molécule d'acide nucléique recombinant comprenant une cassette d'expression comprenant : 5' - séquence antirépresseur A - promoteur - acide nucléique encodant la totalité ou une partie d'une protéine d'intérêt - séquence antirépresseur B - 3', les séquences antirépresseur A et B pouvant être identiques ou différentes et étant choisies parmi le groupe constitué par :
(i) l'une quelconque des SEQ ID NO: 1 à 65,
(ii) des fragments de l'une quelconque des SEQ ID NO: 1 - 65, lesdits fragments possédant une activité antirépresseur,
(iii) des séquences possédant une identité de séquence nucléotidique à concurrence d'au moins 70 % par rapport à a) ou à b), lesdites séquences possédant une activité antirépresseur ; et
(iv) le complément de l'un quelconque des éléments (i) à (iii), **caractérisée en ce que** ladite cassette d'expression comprend en outre, entre lesdites séquences antirépresseur A et B, une séquence possédant une activité antirépresseur, choisie parmi le groupe constitué par :
a) SEQ ID NO: 66,
b) des fragments de SEQ ID NO: 66, lesdits fragments possédant une activité antirépresseur,
c) des séquences possédant une identité de séquence nucléotidique à concurrence d'au moins 70 % par rapport à a) ou à b), lesdites séquences possédant une activité antirépresseur ; et
d) le complément de l'un quelconque des éléments a) à c).

7. Cellule comprenant une molécule selon l'une quelconque des revendications 1 à 6.

8. Cellule selon la revendication 7, qui est une cellule mammalienne.

9. Cellule selon la revendication 8, qui est une cellule CHO.

10. Procédé pour produire une protéine d'intérêt, comprenant la mise en culture d'une cellule comprenant une molécule d'acide nucléique recombinant encodant la protéine d'intérêt pour exprimer ledit acide nucléique encodant la protéine d'intérêt dans ladite cellule,
**caractérisé en ce que**
ladite molécule d'acide nucléique recombinant comprend une séquence d'acides nucléiques possédant une activité antirépresseur, choisie parmi le groupe constitué par :
a) SEQ ID NO: 66,
b) des fragments de SEQ ID NO: 66, lesdits fragments possédant une activité antirépresseur,
c) des séquences possédant une identité de séquence nucléotidique à concurrence d'au moins 70 % par rapport à a) ou à b), lesdites séquences possédant une activité antirépresseur ; et
d) le complément de l'un quelconque des éléments a) à c).

11. Procédé selon la revendication 10, comprenant en outre l'isolation de ladite protéine d'intérêt.

12. Procédé selon la revendication 10 ou 11, dans lequel ladite séquence d'acides nucléiques possédant une activité antirépresseur est située en amont d'un promoteur qui contrôle l'expression de la protéine d'intérêt.

13. Procédé selon la revendication 12, dans lequel ladite séquence possédant une activité antirépresseur et ledit promoteur sont séparés de moins de 2 kb.

14. Procédé selon l'une quelconque des revendications 10 à 13, dans lequel ledit acide nucléique encodant une protéine d'intérêt est présent dans un gène polycistronique encodant en outre un gène marqueur sélectionnable.

15. Procédé selon l'une quelconque des revendications 10 à 14, dans lequel ladite cellule est une cellule mammalienne.

16. Procédé selon la revendication 15, dans lequel ladite cellule est une cellule CHO.

17. Utilisation d'une séquence d'acides nucléiques possédant une activité antirépresseur, choisie parmi le groupe constitué par :
a) SEQ ID NO: 66,
b) des fragments de SEQ ID NO: 66, lesdits fragments possédant une activité antirépresseur,
c) des séquences possédant une identité de séquence nucléotidique à concurrence d'au moins 70 % par rapport à a) ou à b), lesdites séquences possédant une activité antirépresseur ; et
d) le complément de l'un quelconque des éléments a) à c),
pour augmenter l'expression d'un acide nucléique d'intérêt.

18. Procédé pour générer une cellule hôte exprimant deux polypeptides d'intérêt, procédé comprenant :
a) l'introduction dans une cellule hôte d'une ou de plusieurs molécules d'acides nucléiques, la molécule ou les molécules d'acides nucléiques comprenant ensemble :
(i) un promoteur lié de manière fonctionnelle à une séquence encodant un premier polypeptide d'intérêt et un premier gène marqueur sélectionnable, et
(ii) un promoteur lié de manière fonctionnelle à une séquence encodant un deuxième polypeptide d'intérêt et un deuxième gène marqueur sélectionnable, et
(iii) au moins une séquence possédant une activité antirépresseur, choisie parmi le groupe constitué par :
(a) SEQ ID NO: 66,
(b) des fragments de SEQ ID NO: 66, lesdits fragments possédant une activité antirépresseur,
(c) des séquences possédant une identité à concurrence d'au moins 70 % par rapport à (a) ou à (b) et possédant une activité antirépresseur ; et
(d) le complément de l'un quelconque des éléments (a) à (c) ;
b) la sélection d'une cellule hôte en procédant à une sélection essentiellement simultanée pour l'expression dudit premier et dudit deuxième gène marqueur sélectionnable.

19. Procédé pour l'expression de deux polypeptides d'intérêt, le procédé comprenant :
la mise en culture d'une cellule hôte obtenue via le procédé selon la revendication 18 pour exprimer ledit premier et ledit deuxième polypeptide, et facultativement l'isolation desdits polypeptides.

20. Procédé selon la revendication 18 ou 19, dans lequel lesdits deux polypeptides font partie d'une protéine multimérique.


### SEQUENCE LISTING

<110> ChromaGenics B.V.
   Otte, Arie P.
   Kwaks, Theodorus H.J.
   Sewalt, Richard G.A.B.
   van Blokland, Rik
<120> Novel sequence for improving expression of nucleic acid
<130> 0111 WO 00 ORD
<150> PCT/EP2004/051405
   <151> 2004-07-08
<160> 82
<170> PatentIn version 3.2
<210> 1
   <211> 749
   <212> DNA
   <213> Homo sapiens
<220>
   <221> misc_feature
   <223> sequence of STAR1
<400> 1
   atgcggtggg ggcgcgccag agactcgtgg gatccttggc ttggatgttt ggatctttct     60
   gagttgcctg tgccgcgaaa gacaggtaca tttctgatta ggcctgtgaa gcctcctgga    120
   ggaccatctc attaagacga tggtattgga gggagagtca cagaaagaac tgtggcccct    180
   ccctcactgc aaaacggaag tgattttatt ttaatgggag ttggaatatg tgagggctgc    240
   aggaaccagt ctccctcctt cttggttgga aaagctgggg ctggcctcag agacaggttt    300
   tttggccccg ctgggctggg cagtctagtc gaccctttgt agactgtgca cacccctaga    360
   agagcaacta cccctataca ccaggctggc tcaagtgaaa ggggctctgg gctccagtct    420
   ggaaaatctg gtgtcctggg gacctctggt cttgcttctc tcctcccctg cactggctct    480
   gggtgcttat ctctgcagaa gcttctcgct agcaaaccca cattcagcgc cctgtagctg    540
   aacacagcac aaaaagccct agagatcaaa agcattagta tgggcagttg agcgggaggt    600
   gaatatttaa cgcttttgtt catcaataac tcgttggctt tgacctgtct gaacaagtcg    660
   agcaataagg tgaaatgcag gtcacagcgt ctaacaaata tgaaaatgtg tatattcacc    720
   ccggtctcca gccggcgcgc caggctccc                                      749
<210> 2
   <211> 883
   <212> DNA
   <213> Homo sapiens
<220>
   <221> misc_feature
   <223> sequence of STAR2
<400> 2
   gggtgcttcc tgaattcttc cctgagaagg atggtggccg gtaaggtccg tgtaggtggg     60
   gtgcggctcc ccaggccccg gcccgtggtg gtggccgctg cccagcggcc cggcaccccc    120
   atagtccatg gcgcccgagg cagcgtgggg gaggtgagtt agaccaaaga gggctggccc    180
   ggagttgctc atgggctcca catagctgcc ccccacgaag acggggcttc cctgtatgtg    240
   tggggtccca tagctgccgt tgccctgcag gccatgagcg tgcgggtcat agtcgggggt    300
   gccccctgcg cccgcccctg ccgccgtgta gcgcttctgt gggggtggcg ggggtgcgca    360
   gctgggcagg gacgcagggt aggaggcggg gggcagcccg taggtaccct gggggggctt    420
   ggagaagggc gggggcgact ggggctcata cgggacgctg ttgaccagcg aatgcataga    480
   gttcagatag ccaccggctc cggggggcac ggggctgcga cttggagact ggcccccgga     540
   tgacgttagc atgcccttgc ccttctgatc ctttttgtac ttcatgcggc gattctggaa    600
   ccagatcttg atctggcgct cagtgaggtt cagcagattg gccatctcca cccggcgcgg    660
   ccggcacagg tagcggttga agtggaactc tttctccagc tccaccagct gcgcgctcgt    720
   gtaggccgtg cgcgcgcgct tggacgaagc ctgccccggc gggctcttgt cgccagcgca    780
   gctttcgcct gcgaggacag agagaggaag agcggcgtca ggggctgccg cggccccgcc    840
   cagcccctga cccagcccgg cccctccttc caccaggccc caa                      883
<210> 3
   <211> 2126
   <212> DNA
   <213> Homo sapiens
<220>
   <221> misc_feature
   <223> sequence of STAR3
<400> 3
   atctcgagta ctgaaatagg agtaaatctg aagagcaaat aagatgagcc agaaaaccat     60
   gaaaagaaca gggactacca gttgattcca caaggacatt cccaaggtga gaaggccata    120
   tacctccact acctgaacca attctctgta tgcagattta gcaaggttat aaggtagcaa    180
   aagattagac ccaagaaaat agagaacttc caatccagta aaaatcatag caaatttatt    240
   gatgataaca attgtctcca aaggaacaag gcagagtcgt gctagcagag gaagcacgtg    300
   agctgaaaac agccaaatct gctttgtttt catgacacag gagcataaag tacacaccac    360
   caactgacct attaaggctg tggtaaaccg attcatagag agaggttcta aatacattgg    420
   tccctcacag gcaaactgca gttcgctccg aacgtagtcc ctggaaattt gatgtccagt    480
   atagaaaagc agagcagtca aaaaatatag ataaagctga accagatgtt gcctgggcaa    540
   tgttagcagc accacactta agatataacc tcaggctgtg gactccctcc ctggggagcg    600
   gtgctgccgg cggcgggcgg gctccgcaac tccccggctc tctcgcccgc cctcccgttc 660
   tcctcgggcg gcggcggggg ccgggactgc gccgctcaca gcggcggctc ttctgcgccc 720
   ggcctcggag gcagtggcgg tggcggccat ggcctcctgc gttcgccgat gtcagcattt 780
   cgaactgagg gtcatctcct tgggactggt tagacagtgg gtgcagccca cggagggcga 840
   gttgaagcag ggtggggtgt cacctccccc aggaagtcca gtgggtcagg gaactccctc 900
   ccctagccaa gggaggccgt gagggactgt gcccggtgag agactgtgcc ctgaggaaag 960
   gtgcactctg gcccagatac tacacttttc ccacggtctt caaaacccgc agaccaggag 1020
   attccctcgg gttcctacac caccaggacc ctgggtttca accacaaaac cgggccattt 1080
   gggcagacac ccagctagct gcaagagttg tttttttttt tatactcctg tggcacctgg 1140
   aacgccagcg agagagcacc tttcactccc ctggaaaggg ggctgaaggc agggaccttt 1200
   agctgcgggc taggggtttt ggggttgagt gggggagggg agagggaaaa ggcctcgtca 1260
   ttggcgtcgt ctgcagccaa taaggctacg ctcctctgct gcgagtagac ccaatccttt 1320
   cctagaggtg gagggggcgg gtaggtggaa gtagaggtgg cgcggtatct aggagagaga 1380
   aaaagggctg gaccaatagg tgcccggaag aggcggaccc agcggtctgt tgattggtat 1440
   tggcagtgga ccctcccccg gggtggtgcc ggaggggggg atgatgggtc gaggggtgtg 1500
   tttatgtgga agcgagatga ccggcaggaa cctgccccaa tgggctgcag agtggttagt 1560
   gagtgggtga cagacagacc cgtaggccaa cgggtggcct taagtgtctt tggtctcctc 1620
   caatggagca gcggcggggc gggaccgcga ctcgggttta atgagactcc attgggctgt 1680
   aatcagtgtc atgtcggatt catgtcaacg acaacaacag ggggacacaa aatggcggcg 1740
   gcttagtcct acccctggcg gcggcggcag cggtggcgga ggcgacggca ctcctccagg 1800
   cggcagccgc agtttctcag gcagcggcag cgcccccggc aggcgcggtg gcggtggcgc 1860
   gcagccaggt ctgtcaccca ccccgcgcgt tcccaggggg aggagactgg gcgggagggg 1920
   ggaacagacg gggggggatt caggggcttg cgacgcccct cccacaggcc tctgcgcgag 1980
   ggtcaccgcg gggccgctcg gggtcaggct gcccctgagc gtgacggtag ggggcggggg 2040
   aaaggggagg agggacaggc cccgcccctc ggcagggcct ctagggcaag ggggcggggc 2100
   tcgaggagcg gaggggggcg gggcgg 2126
<210> 4
   <211> 1625
   <212> DNA
   <213> Homo sapiens
<220>
   <221> misc_feature
   <223> sequence of STAR4
<400> 4
   gatctgagtc atgttttaag gggaggattc ttttggctgc tgagttgaga ttaggttgag 60
   ggtagtgaag gtaaaggcag tgagaccacg taggggtcat tgcagtaatc caggctggag 120
   atgatggtgg ttcagttgga atagcagtgc atgtgctgta acaacctcag ctgggaagca 180
   gtatatgtgg cgttatgacc tcagctggaa cagcaatgca tgtggtggtg taatgacccc 240
   agctgggtag ggtgcatgtg gtgtaacgac ctcagctggg tagcagtgtg tgtgatgtaa 300
   caacctcagc tgggtagcag tgtacttgat aaaatgttgg catactctag atttgttatg 360
   agggtagtgc cattaaattt ctccacaaat tggttgtcac gtatgagtga aaagaggaag 420
   tgatggaaga cttcagtgct tttggcctga ataaatagaa gacgtcattt ccagttaatg 480
   gagacaggga agactaaagg tagggtggga ttcagtagag caggtgttca gttttgaata 540
   tgatgaactc tgagagagga aaaacttttt ctacctctta gtttttgtga ctggacttaa 600
   gaattaaagt gacataagac agagtaacaa gacaaaaata tgcgaggtta tttaatattt 660
   ttacttgcag aggggaatct tcaaaagaaa aatgaagacc caaagaagcc attagggtca 720
   aaagctcata tgccttttta agtagaaaat gataaatttt aacaatgtga gaagacaaag 780
   gtgtttgagc tgagggcaat aaattgtggg acagtgatta agaaatatat gggggaaatg 840
   aaatgataag ttattttagt agatttattc ttcatatcta ttttggcttc aacttccagt 900
   ctctagtgat aagaatgttc ttctcttcct ggtacagaga gagcaccttt ctcatgggaa 960
   attttatgac cttgctgtaa gtagaaaggg gaagatcgat ctcctgtttc ccagcatcag 1020
   gatgcaaaca tttccctcca ttccagttct caaccccatg gctgggcctc atggcattcc 1080
   agcatcgcta tgagtgcacc tttcctgcag gctgcctcgg gtagctggtg cactgctagg 1140
   tcagtctatg tgaccaggag ctgggcctct gggcaatgcc agttggcagc ccccatccct 1200
   ccactgctgg gggcctccta tccagaaggg cttggtgtgc agaacgatgg tgcaccatca 1260
   tcattcccca cttgccatct ttcaggggac agccagctgc tttgggcgcg gcaaaaaaca 1320
   cccaactcac tcctcttcag gggcctctgg tctgatgcca ccacaggaca tccttgagtg 1380
   ctgggcagtc tgaggacagg gaaggagtga tgaccacaaa acaggaatgg cagcagcagt 1440
   gacaggagga agtcaaaggc ttgtgtgtcc tggccctgct gagggctggc gagggccctg 1500
   ggatggcgct cagtgcctgg tcggctgcaa gaggccagcc ctctgcccat gaggggagct 1560
   ggcagtgacc aagctgcact gccctggtgg tgcatttcct gccccactct ttccttctaa 1620
   gatcc 1625
<210> 5
   <211> 1571
   <212> DNA
   <213> Homo sapiens
<220>
   <221> misc_feature
   <223> sequence of STAR5
<400> 5
   cacctgattt aaatgatctg tctggtgagc tcactgggtc tttactcgca tgctgggtcc     60
   acagctccac tgtcctgcag ggtccgtgag tgtgggcccc ttatctattt catcatcata    120
   accctgcgtg tcctcaactc ctggcacata ttgggtggcc ccatccacac acggttgttg    180
   agtgaatcca tgagatgaca aaggctatga tgtagactat atcatgagcc agaaccaggc    240
   tttcctacct ccagacaatc aagggccttg atttgggatt gagggagaaa ggagtagaag    300
   ccaggaagga gaagagattg aggtttacca agggtgcaaa gtcctggccc ctgactgtag    360
   gctgaaaact atagaaatga tagaacaatt ttgcaatgaa atgcagaaga ccctgcatca    420
   actttaggtg ggacttcggg tatttttatg gccacagaac atcctcccat ttacctgcat    480
   ggcccagaca cagacttcaa aacagttgag gccagcaggc tccaggtaag tggtaggatt    540
   ccagaatgcc ctcagagtgt tgtgggaggc agcaggcgat tttcctggac ttctgagttt    600
   atgagaaccc caaaccccaa ttggcattaa cattgaggtc tcaatgtatc atggcaggaa    660
   gcttccgagt ggtgaaaagg aaagtgaaca tcaaagctcg gaagacaaga gggtggagtg    720
   atggcaacca agagcaagac ccttccctct cctgtgatgg ggtggctcta tgtgaagccc    780
   ccaaactgga cacaggtctg gcagaatgag gaacccactg agatttagcg ccaacatcca    840
   gcataaaagg gagactgaca tagaatttga gttagttaaa aataaggcac aatgcttttc    900
   atgtattcct gagttttgtg gactggtgtt caatttgcag cattcttagt tgattaaatc    960
   tgagatgaag aaagagtgtc caacactttc accttggaaa gctctggaaa agcaaaaggg   1020
   agagacaatt agcttcatcc attaactcac ttagtcatta tgcattcatt catgtaacta   1080
   ccaaacacgt actgagtgcc taacactcct gagacactga gaagtttctt gggaatacaa   1140
   agatgaataa aaaccacgcc aggcaggagt tggaggaagg ttctggatgc caccacgctc   1200
   tacctcctgg ctggacacca ggcaatgttg gtaaccttct gcctccaatt tctgcaaata   1260
   cataattaat aaacacaagg ttatcttcta aacagttctt aaaatgagtc aactttgttt   1320
   aaacttgttc tttttagaga aaaatgtatt tttgaaagag ttggttagtg ctaggggaaa   1380
   tgtctgggca cagctcagtc tggtgtgaga gcaggaagca gctctgtgtg tctggggtgg   1440
   gtacgtatgt aggacctgtg ggagaccagg ttgggggaag gcccctcctc atcaagggct   1500
   cctttgcttt ggtttgcttt ggcgtgggag gtgctgtgcc acaagggaat acgggaaata   1560
   agatctctgc t                                                        1571
<210> 6
   <211> 1173
   <212> DNA
   <213> Homo sapiens
<230>
   <221> misc_feature
   <223> sequence of STAR6
<400> 6
   tgacccacca cagacatccc ctctggcctc ctgagtggtt tcttcagcac agcttccaga      60
   gccaaattaa acgttcactc tatgtctata gacaaaaagg gttttgacta aactctgtgt     120
   tttagagagg gagttaaatg ctgttaactt tttaggggtg ggcgagaggg atgacaaata     180
   acaacttgtc tgaatgtttt acatttctcc ccactgcctc aagaaggttc acaacgaggt     240
   catccatgat aaggagtaag acctcccagc cggactgtcc ctcggccccc agaggacact     300
   ccacagagat atgctaactg gacttggaga ctggctcaca ctccagagaa aagcatggag     360
   cacgagcgca cagagcaggg ccaaggtccc agggacagaa tgtctaggag ggagattggg     420
   gtgagggtaa tctgatgcaa ttactgtggc agctcaacat tcaagggagg gggaagaaag     480
   aaacagtccc tgtcaagtaa gttgtgcagc agagatggta agctccaaaa tttgaaactt     540
   tggctgctgg aaagttttag ggggcagaga taagaagaca taagagactt tgagggttta     600
   ctacacacta gacgctctat gcatttattt atttattatc tcttatttat tactttgtat     660
   aactcttata ataatcttat gaaaacggaa accctcatat acccatttta cagatgagaa     720
   aagtgacaat tttgagagca tagctaagaa tagctagtaa gtaaaggagc tgggacctaa     780
   accaaaccct atctcaccag agtacacact cttttttttt ttccagtgta atttttttta      840
   atttttattt tactttaagt tctgggatac atgtgcagaa ggtatggttt gttacatagg     900
   tatatgtgtg ccatagtgga ttgctgcacc tatcaacccg tcatctaggt ttaagcccca     960
   catgcattag ctatttgtcc tgatgctctc cctcccctcc ccacaccaga caggccttgg    1020
   tgtgtgatgt tcccctccct gtgtccatgt gttctcactg ttcagctccc acttatgagt    1080
   gagaacgtgt ggtatttggt tttctgttcc tgtgttagtt tgctgaggat gatggcttcc    1140
   agcttcatcc atgtccctgc aaaggacacg atc                                 1173
<210> 7
   <211> 2101
   <212> DNA
   <213> Homo sapiens
<220>
   <221> misc_feature
   <223> sequence of STAR7
<400> 7
   aggtgggtgg atcacccgag gtcaggagtt caagaccagc ctggccaaca tggtaaaacc      60
   tcgtctctac taaaaaatac gaaaaattag ctggttgtgg tggtgcgtgc ttgtaatccc     120
   agctactcgg gaggctgagg caggagaatc acttgaatct gggaggcaga ggttgcagtg     180
   agctgagata gtgccattgc actccagcct gggcaacaga cggagactct gtctccaaaa    240
   aaaaaaaaaa aaatcttaga ggacaagaat ggctctctca aacttttgaa gaaagaataa    300
   ataaattatg cagttctaga agaagtaatg gggatatagg tgcagctcat gatgaggaag    360
   acttagctta actttcataa tgcatctgtc tggcctaaga cgtggtgagc tttttatgtc    420
   tgaaaacatt ccaatataga atgataataa taatcacttc tgacccccct tttttttcct    480
   ctccctagac tgtgaagcag aaaccccata tttttcttag ggaagtggct acgcactttg    540
   tatttatatt aacaactacc ttatcaggaa attcatattg ttgccctttt atggatgggg    600
   aaactggaca agtgacagag caaaatccaa acacagctgg ggatttccct cttttagatg    660
   atgattttaa aagaatgctg ccagagagat tcttgcagtg ttggaggaca tatatgacct    720
   ttaagatatt ttccagctca gagatgctat gaatgtatcc tgagtgcatg gatggacctc    780
   agttttgcag attctgtagc ttatacaatt tggtggtttt ctttagaaga aaataacaca    840
   tttataaata ttaaaatagg cccaagacct tacaagggca ttcatacaaa tgagaggctc    900
   tgaagtttga gtttgttcac tttctagtta attatctcct gcctgtttgt cataaatgcg    960
   tttagtaggg agctgctaat gacaggttcc tccaacagag tgtggaagaa ggagatgaca   1020
   gctggcttcc cctctgggac agcctcagag ctagtgggga aactatgtta gcagagtgat   1080
   gcagtgacca agaaaatagc actaggagaa agctggtcca tgagcagctg gtgagaaaag   1140
   gggtggtaat catgtatgcc ctttcctgtt ttatttttta ttgggtttcc ttttgcctct    1200
   caattccttc tgacaataca aaatgttggt tggaacatgg agcacctgga agtctggttc   1260
   attttctctc agtctcttga tgttctctcg ggttcactgc ctattgttct cagttctaca   1320
   cttgagcaat ctcctcaata gctaaagctt ccacaatgca gattttgtga tgacaaattc   1380
   agcatcaccc agcagaactt aggttttttt ctgtcctccg tttcctgacc tttttcttct   1440
   gagtgcttta tgtcacctcg tgaaccatcc tttccttagt catctaccta gcagtcctga   1500
   ttcttttgac ttgtctccct acaccacaat aaatcactaa ttactatgga ttcaatccct   1560
   aaaatttgca caaacttgca aatagattac gggttgaaac ttagagattt caaacttgag   1620
   aaaaaagttt aaatcaagaa aaatgacctt taccttgaga gtagaggcaa tgtcatttcc   1680
   aggaataatt ataataatat tgtgtttaat atttgtatgt aacatttgaa taccttcaat   1740
   gttcttattt gtgttatttt aatctcttga tgttactaac tcatttggta gggaagaaaa   1800
   catgctaaaa taggcatgag tgtcttatta aatgtgacaa gtgaatagat ggcagaaggt   1860
   ggattcatat tcagttttcc atcaccctgg aaatcatgcg gagatgattt ctgcttgcaa   1920
   ataaaactaa cccaatgagg ggaacagctg ttcttaggtg aaaacaaaac aaacacgcca   1980
   aaaaccttta ttctctttat tatgaatcaa atttttcctc tcagataatt gttttattta   2040
   tttattttta ttattattgt tattatgtcc agtctcactc tgtcgcctaa gctggcatga 2100
   t 2101
<210> 8
   <211> 1821
   <212> DNA
   <213> Homo sapiens
<220>
   <221> misc_feature
   <223> sequence of STAR8
<400> 8
   gagatcacct cgaagagagt ctaacgtccg taggaacgct ctcgggttca caaggattga 60
   ccgaacccca ggatacgtcg ctctccatct gaggcttgct ccaaatggcc ctccactatt 120
   ccaggcacgt gggtgtctcc cctaactctc cctgctctcc tgagcccatg ctgcctatca 180
   cccatcggtg caggtccttt ctgaagagct cgggtggatt ctctccatcc cacttccttt 240
   cccaagaaag aagccaccgt tccaagacac ccaatgggac attccccttc cacctccttc 300
   tccaaagttg cccaggtgtt catcacaggt tagggagaga agcccccagg tttcagttac 360
   aaggcatagg acgctggcat gaacacacac acacacacac acacacacac acacacacac 420
   acacgactcg aagaggtagc cacaagggtc attaaacact tgacgactgt tttccaaaaa 480
   cgtggatgca gttcatccac gccaaagcca agggtgcaaa gcaaacacgg aatggtggag 540
   agattccaga ggctcaccaa accctctcag gaatattttc ctgaccctgg gggcagaggt 600
   tggaaacatt gaggacattt cttgggacac acggagaagc tgaccgacca ggcattttcc 660
   tttccactgc aaatgaccta tggcgggggc atttcacttt cccctgcaaa tcacctatgg 720
   cgaggtacct ccccaagccc ccacccccac ttccgcgaat cggcatggct cggcctctat 780
   ccgggtgtca ctccaggtag gcttctcaac gctctcggct caaagaagga caatcacagg 840
   tccaagccca aagcccacac ctcttccttt tgttataccc acagaagtta gagaaaacgc 900
   cacactttga gacaaattaa gagtccttta tttaagccgg cggccaaaga gatggctaac 960
   gctcaaaatt ctctgggccc cgaggaaggg gcttgactaa cttctatacc ttggtttagg 1020
   aaggggaggg gaactcaaat gcggtaattc tacagaagta aaaacatgca ggaatcaaaa 1080
   gaagcaaatg gttatagaga gataaacagt tttaaaaggc aaatggttac aaaaggcaac 1140
   ggtaccaggt gcggggctct aaatccttca tgacacttag atataggtgc tatgctggac 1200
   acgaactcaa ggctttatgt tgttatctct tcgagaaaaa tcctgggaac ttcatgcact 1260
   gtttgtgcca gtatcttatc agttgattgg gctcccttga aatgctgagt atctgcttac 1320
   acaggtcaac tccttgcgga agggggttgg gtaaggagcc cttcgtgtct cgtaaattaa 1380
   ggggtcgatt ggagtttgtc cagcattccc agctacagag agccttattt acatgagaag 1440
   caaggctagg tgattaaaga gaccaacagg gaagattcaa agtagcgact tagagtaaaa 1500
   acaaggttag gcatttcact ttcccagaga acgcgcaaac attcaatggg agagaggtcc 1560
   cgagtcgtca aagtcccaga tgtggcgagc ccccgggagg aaaaaccgtg tcttccttag 1620
   gatgcccgga acaagagcta ggcttccgga gctaggcagc catctatgtc cgtgagccgg 1680
   cgggagggag accgccggga ggcgaagtgg ggcggggcca tccttctttc tgctctgctg 1740
   ctgccgggga gctcctggct ggcgtccaag cggcaggagg ccgccgtcct gcagggcgcc 1800
   gtagagtttg cggtgcagag t 1821
<210> 9
   <211> 1929
   <212> DNA
   <213> Homo sapiens
<220>
   <221> misc_feature
   <223> sequence of STAR9
<400> 9
   cacttcctgg gagtggagca gaggctctgc gtggagcatc catgtgcagt actcttaggt 60
   acggaaggga ttgggctaaa ccatggatgg gagctgggaa gggaagggac caacttcagg 120
   ccccactggg acactggagc tgccaccctt tagagccctc ctaaccctac accagaggct 180
   gagggggacc tcagacatca cacacatgct ttcccatgtt ttcagaaatc tggaaacgta 240
   gaacttcagg ggtgagagtg cctagatatt gaatacaagg ctagattggg cttctgtaat 300
   atcccaaagg accctccagc tttttcacca gcacctaatg cccatcagat accaaagaca 360
   cagcttagga gaggttcacc ctgaagctga ggaggaggca gccggattag agttgactga 420
   gcaaggatga ctgccttctc cacctgacga tttcagctgc tgcccttttc ttttcctggg 480
   aatgcctgtc gccatggcct tctgtgtcca caggagagtt tgacccagat actcatggac 540
   caggcaaagg tgctgttcct cccagcccag ggcccaccat gaagcatgcc tgggagcctg 600
   gtaaggaccc agccactcct gggctgttga cattggcttc tcttgcccag cattgtagcc 660
   acgccactgc attgtactgt gagataagtc aaggtgggct caccaggacc tgcactaaat 720
   tgtgaaattc agctccaaag aactttggaa attacccatg catttaagca aaatgaatga 780
   tacctgagca aaccctttca cattggcaca agttacaatc ctgtctcatc ctcttgatta 840
   caaattccat ccaggcaaga gctgtatcac cctgaggtct ccccattcat gttttggtca 900
   ataatattta gtttcctttt gaaaatagat ttttgtgtta ctccattatg atgggcagag 960
   gccagatgct tatattctat ttaaatgact atgtttttct atctgtaact gggtttgtgt 1020
   tcaggtggta aatgcttttt ttttgcagtc agaagattcc tggaaggcga ccagaaatta 1080
   gctggccgct gtcagacctg aagttacttc taaagggcct ttagaaatga attctttttt 1140
   atgccttctc tgaattctga gaagtaggct tgacttcccc taagtgtgga gttgggagtc   1200
   aactcttctg aaaagaaagt ttcagagcat tttccaaagc catggtcagc tgtgggaagg   1260
   gaagacgatg gatagtacag ttgccggaaa acactgatgg aggcggatgc tccagctcag   1320
   ccaaagacct ttgttctgcc caccccagaa atgccccttc ctcaatcgca gaaacgttgc   1380
   cccatggctc ctgatactca gaatgcagcc tctgaccagg accatctgca tcctccagga   1440
   gctcgtaaga aatgcagcat cgtgggacct gctggcacct ggtgaaccca aacctgcagg   1500
   gctcctgggt gtgcttgggg cggctgcagg ggaagaggga gtcagcagcc tcctcctgac   1560
   cttcccgggg gctgcttttc tgaggggcca gaatgcaccg gttgaccttg ttgcatcact   1620
   ggcccatgac tggctgcttt ggtcaggtgt aaaaaggtgt ttccagaggg tctgctcctc   1680
   tcactatcgg accaggtttc catggagagc tcagcctccc agcaaggata gagaacttca   1740
   aatggctcaa agaactgaga ggccacacat gtgtgacctg aatagtctct gctgcaaaac   1800
   aaagggtttc ttaatgtaaa acgttctctt cctcacagag gggttcccag ctgctagtgg    1860
   gcatgttgca ggcatttcct gggctgcatc aggttgtcat aagccagagg atcatttttg   1920
   ggggctcat                                                            1929
<210> 10
   <211> 1167
   <212> DNA
   <213> Homo sapiens
<220>
   <221> misc_feature
   <223> sequence of STAR10
<220>
   <221> misc_feature
   <222> (452)..(1143)
   <223> n is a, c, g, or t on various positions
<400> 10
   aggtcaggag ttcaagacca gcctggccaa catggtgaaa ccctgtccct acaaaaaata     60
   caaaaattag ccgggcgtgg tggggggcgc ctataatccc agctactcag gatgctgaga    120
   caggagaatt gtttgaaccc gggaggtgga ggttgcagtg aactgagatc gcgccactgc    180
   actccagcct ggtgacagag agagactccg tctcaacaac agacaaacaa acaaacaaac    240
   aacaacaaaa atgtttactg acagctttat tgagataaaa ttcacatgcc ataaaggtca    300
   ccttctacag tatacaattc agtggattta gtatgttcac aaagttgtac gttgttcacc    360
   atctactcca gaacatttac atcaccccta aaagaagctc tttagcagtc acttctcatt    420
   ctccccagcc cctgccaacc acgaatctac tntctgtctc tattctgaat atttcatata    480
   aaggagtcct atcatatggg ccttttacgt ctaccttctt tcacttagca tcatgttttt    540
   aagattcatc cacagtgtag cacgtgtcag ttaattcatt tcatcttatg gctggataat    600
   gctctattgt atgcatatcc ctcactttgc ttatccattc atcaactgat tgacatttgg    660
   gttatttcta ctttttgact attatgagta atgctgctat gaacattcct gtaccaatcg    720
   ttacgtggac atatgctttc aattctcctg agtatgtaac tagggttgga gttgctgggt    780
   catatgttaa ctcagtgttt catttttttg aagaactacc aaatggtttt ccaaagtgga    840
   tgcaacactt tacattccca ccagcaagat atgaaggttc caatgtctct acatttttgc    900
   caacacttgt gattttcttt tatttattta tttatttatt tatttttgag atggagtctc    960
   actctgtcac ccaggctgga gtgcagtggc acaatttcag ctcactgcaa tctccacctc   1020
   tcgggctcaa gcgatactcc tgcctcaacc tcccgagtaa ctgggattac aggcgcccac   1080
   caccacacca agctaatttt ttgtattttt agtagagacg gggtttcatc atgtcggcca    1140
   ggntgtactc gaactctgac ctcaagt                                       1167
<210> 11
   <211> 1377
   <212> DNA
   <213> Homo sapiens
<220>
   <221> misc_feature
   <223> sequence of STAR11
<400> 11
   aggatcactt gagcccagga gttcaagacc agcctgggca acatagcgag aacatgtctc      60
   aaaaaggaaa aaaatggggg aaaaaaccct cccagggaca gatatccaca gccagtcttg     120
   ataagctcca tcattttaaa gtgcaaggcg gtgcctccca tgtggatgat tatttaatcc     180
   tcttgtactt tgtttagtcc tttgtggaaa tgcccatctt ataaattaat agaattctag     240
   aatctaatta aaatggttca actctacatt ttactttagg ataatatcag gaccatcaca     300
   gaatgtctga gatgtggatt taccctatct gtagctcact tcttcaacca ttcttttagc     360
   aaggctagtt atcttcagtg acaacccctt gctgccctct actatctcct ccctcagatg     420
   gactactctg attaagcttg agctagaata agcatgttat cccgggattt catatggaat     480
   attttataca tgagtgagcc attatgagtt gtttgaaaat ttattatgtt gagggagggt     540
   aaccgctgta acaaccatca ccaaatctaa tcgactgaat acatttgacg tttatttctt     600
   gttcacctga cagttcagtg ttacctaaat ttacatgaag acccagaggc ccacgctcct     660
   tcattttggg ctccaccgac ctccaaggtt tcagggccct ctgccccgcc ttctgcaccc     720
   acaggggaag agagtggagg atgcacacgc ccaggcctgg aagtgacgca tgtggcttcc     780
   ccgtccacag acttcaccca cagtccattg gccttcttaa gtcatggact cctgctgagc     840
   tgccagggtg catgggaaat ccatgtgact gtgtgccctg gaggaagggg agcgtttcgg     900
   tgagcacaca ggagtctttg ccactagacg ctgatgagga ttccccacag gcgatgaagc     960
   atggagactc atcttgtaac aaacagatga gttgttgaca tctcttaagt ttactttgtg    1020
   tgcagttttt attcagatag gaaaggctgt taaaatctta acacctaact ggaagaaggg    1080
   ttttagagaa gtgtggtttt cagtaagcca gttctttcca caatccaaga aacgaaataa    1140
   atttccagca tggagcagtt ggcaggtaag gtttttgttg tggtctcgcc caggcttgag    1200
   tgtaaccggt gtggtcatag ctcactacat tctcaaactc ctggccttaa gtcatcctcc    1260
   tgcctcagcc tcccaaaggc aagtaaggtt aagaataggg gaaaggtgaa gtttcacagc    1320
   ttttctagaa ttctttttat tcaagggact ctcagatcat caaacccacc cagaatc       1377
<210> 12
   <211> 1051
   <212> DNA
   <213> Homo sapiens
<220>
   <221> misc_feature
   <223> sequence of STAR12
<400> 12
   atcctgcttc tgggaagaga gtggcctccc ttgtgcaggt gactttggca ggaccagcag      60
   aaacccaggt ttcctgtcag gaggaagtgc tcagcttatc tctgtgaagg gtcgtgataa     120
   ggcacgagga ggcaggggct tgccaggatg ttgcctttct gtgccatatg ggacatctca     180
   gcttacgttg ttaagaaata tttggcaaga agatgcacac agaatttctg taacgaatag     240
   gatggagttt taagggttac tacgaaaaaa agaaaactac tggagaagag ggaagccaaa     300
   caccaccaag tttgaaatcg attttattgg acgaatgtct cactttaaat ttaaatggag     360
   tccaacttcc ttttctcacc cagacgtcga gaaggtggca ttcaaaatgt ttacacttgt     420
   ttcatctgcc tttttgctaa gtcctggtcc cctacctcct ttccctcact tcacatttgt     480
   cgtttcatcg cacacatatg ctcatcttta tatttacata tatataattt ttatatatgg     540
   cttgtgaaat atgccagacg agggatgaaa tagtcctgaa aacagctgga aaattatgca     600
   acagtgggga gattgggcac atgtacattc tgtactgcaa agttgcacaa cagaccaagt     660
   ttgttataag tgaggctggg tggtttttat tttttctcta ggacaacagc ttgcctggtg     720
   gagtaggcct cctgcagaag gcattttctt aggagcctca acttccccaa gaagaggaga     780
   gggcgagact ggagttgtgc tggcagcaca gagacaaggg ggcacggcag gactgcagcc     840
   tgcagagggg ctggagaagc ggaggctggc acccagtggc cagcgaggcc caggtccaag     900
   tccagcgagg tcgaggtcta gagtacagca aggccaaggt ccaaggtcag tgagtctaag     960
   gtccatggtc agtgaggctg agacccaggg tccaatgagg ccaaggtcca gagtccagta    1020
   aggccgagat ccagggtcca gggaggtcaa g                                   1051
<210> 13
   <211> 1291
   <212> DNA
   <213> Homo sapiens
<220> .
   <221> misc_feature
   <223> sequence of STAR13
<400> 13
   agccactgag gtcctaactg cagccaaggg gccgttctgc acatgtcgct caccctctgt     60
   gctctgttcc ccacagagca aacgcacatg gcaacgttgg tccgctcagc cactggttct    120
   gtggtggaac ggtggatgtc tgcactgtga catcagctga gtaagtaaca acgactgagg    180
   atgccgctga cccagggctg gggaagggga ctcccagctc agacaggctt ggctgtggtt    240
   tgctttggga ggagagtgaa catcacaggg aatggctcat gtcagcccca ggagggtggg    300
   ctggcccctg gtccccgggc tccttctggc cctgcaggcg atagagagcc tcaacctgct    360
   gccgcttctc cttggcccgg gtgatggccg tctggaagag cctgcagtag aggtgcacag    420
   ccagcggaga gtcgtcattg ccgggtacag ggtaggtgat gaggcagggg ttgcagttgg    480
   tgtccacgat gcccactgtg gggatgttca tcttggctgc gtctctcacg gccacgtgtg    540
   gctcaaagat gttgttgagc gtgtgcagga agatgatgag gtccggcagg cggaccgtgg    600
   ggccaaagag gaggcgcgcg ttggtcagca tgccgcccct gaagtagcga gtgtgggcgt    660
   actcgccaca gtcacgggcc atgttctcaa tcaggtacga gaactgccgg ttgcggctta    720
   taaacaagat gatgcccttg cggtaggcca tgtgggcggt gaagttcaag gccagctgga    780
   ggtgcgtggc tgtctgttcc aggtcgatga tgtcgtggtc caggcggctc ccaaagatgt    840
   acggctccat aaacctgcca gagaccccac caaggcaagg gggatgagag ttcacggggc    900
   catctccact ggctccttgc aggaacacag acgcccacca gggactcccg ggctcctctg    960
   tgggggcact atgggctggg aagcacaatt tgcaacgctc cccgtgtgca tggacagcag   1020
   tgcagaccca tccaggccac ccctctgcat gcctcgtctc gtggcttaac ccctcctacc   1080
   ctctacctct tcccgaagga atcctaatag aactgacccc atatggatgt gtggacatcc   1140
   aacatgacgc caaaaggaca ttctgccccg tgcagctcac agggcagccg cctccgtcac   1200
   tgtcctcttc ccgaggcttt gcggatgagg cccctctggg gttggactta gcggggtgct   1260
   ctgggccaaa agcattaagg gatcagggca g                                   1291
<210> 14
   <211> 711
   <212> DNA
   <213> Homo sapiens
<220>
   <221> misc_feature
   <223> sequence of STAR14
<400> 14
   ccctggacca gggtccgtgg tcttggtggg cactggcttc ttcttgctgg gtgttttcct      60
   gtgggtctct ggcaaggcac tttttgtggc gctgcttgtg ctgtgtgcgg gaggggcagg     120
   tgctctttcc tcttggagct ggaccctctg gggcgggtcc ccgtcggcct ccttgtgtgt     180
   tttctgcacc tggtacagct ggatggcctc ctcaatgccg tcgtcgctgc tggagtcgga     240
   cgcctcgggc gcctgtacgg cgctcgtgac tcgctttccc ctccttgcgg tgctggcgtt     300
   ccttttaatc ccacttttat tctgtactgc ttctgaaggg cggtgggggt tgctggcttt     360
   gtgctgccct ccttctcctg cgtggtcgtg gtcgtgacct tggacctgag gcttctgggc     420
   tgcacgtttg tctttgctaa ccgggggagg tctgcagaag gcgaactcct tctggacgcc     480
   catcaggccc tgccggtgca ccacctttgt agccggctct tggtgggatt tcgagagtga     540
   cttcgccgaa ttttcatgtg tgtctggttt cttctccact gacccatcac atttttgggt     600
   ctcatgctgt cttttctcat tcagaaactg ttctatttct gccctgatgc tctgctcaaa     660
   ggagtctgct ctgctcatgc tgactgggga ggcagagccc tggtccttgc t              711
<210> 15
   <211> 1876
   <212> DNA
   <213> Homo sapiens
<220>
   <221> misc_feature
   <223> sequence of STAR15
<400> 15
   gagtccaaga tcaaggtgcc agcatcttgt gagggccttc ttgttacgtc actccctagc      60
   gaaagggcaa agagagggtg agcaagagaa aggggggctg aactcgtcct tgtagaagag     120
   gcccattccc gagacaatgg cattcatcca ttcactccac cctcatggcc tcaccacctc     180
   tcatgaggct ccacctccca gccctggttt gttggggatt aaatttccaa cacatgcctt     240
   ttgggggaca tgttaaaatt atagcacccc aaatgttaca ctatcttttg atgagcggta     300
   gttctgattt taagtctagc tggcctactt tttcttgcac gtgggatgct ttctgcctgt     360
   tccagggcag gcagctcttc tctgtccctc tgctggcccc acctcatcct ctgttgtcct     420
   cttccctcct tctgtgccct ggggtcctgg tgggggtgtg actgtcaact gcgttgggct     480
   aacttttttc cctgctggtg gcccgtaatg aaagaaagct tcttgctccc aagttcctta     540
   aatccaagct catagacaac gcggtctcac agcaggcctg gggccagcct cacgtgagcc     600
   ccttccctgg tgtagtcact ggcatggggg aatgggattt cctgttgccc tactgtgtgg     660
   ctgaggtggg ggttgcttcc tggagccagg ccttgtggaa gggcagtgcc cactgcagtg     720
   gatgctgggc cctgaatctg accccagtgt tcattggctc tgtgagaccc agtgagggca     780
   gggagggaag tggagctggg gtgagaagta gaggccctgc agggcccacg tgccagccac     840
   caggcctcag actaggctca gatgacggag agctgcacac ctgcccaacc caggccctgc     900
   agtgcccaca tgccagccgc tggggcccag acttgctcca gagggcggag agctttacac     960
   cggcccaacc caggccatgg ctccaaatgc gtgacagttt tgctgttgct tcttttagtc    1020
   attgtcaagt tgatgcttgt tttgcagagg accaaggctt tatgaaccta ttaccctgtg    1080
   tgaagagttt caccaggtta tggaaatttc tttaaaacca taccacagtt ttttcattat    1140
   tcatgtatat ttttaaaaat aattactgca ctcagtagaa taacatgaaa atgttgcctg    1200
   ttagcccttt tccagtttgc cccgagaata ctgggggcac ttgtggctgc aatgtttatc    1260
   ctgcggcagc tttgccatga agtatctcac ttttattatt atttttgcat tgctcgagta    1320
   tattgacttt ggaaacaaaa gacatcattc tatttatagc attatgtttt tagtagtggt    1380
   atttccatat acaagataca gtaattttcc gtcaatgaaa atgtcaaatt ctagaaaatg    1440
   taacattcct atgcgtggtg ttaacatcgt tctctaacag ttgttggccg aagattcgtt    1500
   tgatgaatcc gatttttcca aaatagccga ttctgatgat tcagacgatt ctgatgttct    1560
   gtttagaaat aattccaaga acagttttta cattttattt tcacattgaa aatcagtcag    1620
   atttgcttca gcctcaaaga gcacgtttat gtaaaattaa atgagtgctg gcagccagct    1680
   gcgctttgtt tttctaaatg ggaaaagggt taaatttcac tcagctttta aatgacagcg    1740
   cacagcctgt gtcatagagg gttggaggag atgactttaa ctgcctgtgg ttaggatccc    1800
   tttcccccag gaatgtctgg gagcccactg ccgggtttgc tgtccgtctc gtttggactc    1860
   agttctgcat gtactg                                                    1876
<210> 16
   <211> 1282
   <212> DNA
   <213> Homo sapiens
<220>
   <221> misc_feature
   <223> sequence of STAR16
<400> 16
   cgcccacctc ggctttccaa agtgctggga ttacaggcat gagtcactgc gcccatcctg      60
   attccaagtc tttagataat aacttaactt tttcgaccaa ttgccaatca ggcaatcttt     120
   gaatctgcct atgacctagg acatccctct ccctacaagt tgccccgcgt ttccagacca     180
   aaccaatgta catcttacat gtattgattg aagttttaca tctccctaaa acatataaaa     240
   ccaagctata gtctgaccac ctcaggcacg tgttctcagg acctccctgg ggctatggca     300
   tgggtcctgg tcctcagatt tggctcagaa taaatctctt caaatatttt ccagaatttt     360
   actcttttca tcaccattac ctatcaccca taagtcagag ttttccacaa ccccttcctc     420
   agattcagta atttgctaga atggccacca aactcaggaa agtattttac ttacaattac 480
   caatttatta tgaagaactc aaatcaggaa tagccaaatg gaagaggcat agggaaaggt 540
   atggaggaag gggcacaaag cttccatgcc ctgtgtgcac accaccctct cagcatcttc 600
   atgtgttcac caactcagaa gctcttcaaa ctttgtcatt taggggtttt tatggcagtt 660
   ccactatgta ggcatggttg ataaatcact ggtcatcggt gatagaactc tgtctccagc 720
   tcctctctct ctcctcccca gaagtcctga ggtggggctg aaagtttcac aaggttagtt 780
   gctctgacaa ccagccccta tcctgaagct attgaggggt cccccaaaag ttaccttagt 840
   atggttggaa gaggcttatt atgaataaca aaagatgctc ctatttttac cactagggag 900
   catatccaag tcttgcggga acaaagcatg ttactggtag caaattcata caggtagata 960
   gcaatctcaa ttcttgcctt ctcagaagaa agaatttgac caagggggca taaggcagag 1020
   tgagggacca agataagttt tagagcagga gtgaaagttt attaaaaagt tttaggcagg 1080
   aatgaaagaa agtaaagtac atttggaaga gggccaagtg ggcgacatga gagagtcaaa 1140
   caccatgccc tgtttgatgt ttggcttggg gtcttatatg atgacatgct tctgagggtt 1200
   gcatccttct cccctgattc ttcccttggg gtgggctgtc cgcatgcaca atggcctgcc 1260
   agcagtaggg aggggccgca tg 1282
<210> 17
   <211> 793
   <212> DNA
   <213> Homo sapiens
<220>
   <221> misc_feature
   <223> sequence of STAR17
<400> 17
   atccgagggg aggaggagaa gaggaaggcg agcagggcgc cggagcccga ggtgtctgcg      60
   agaactgttt taaatggttg gcttgaaaat gtcactagtg ctaagtggct tttcggattg     120
   tcttatttat tactttgtca ggtttcctta aggagagggt gtgttggggg tgggggagga     180
   ggtggactgg ggaaacctct gcgtttctcc tcctcggctg cacagggtga gtaggaaacg     240
   cctcgctgcc acttaacaat ccctctatta gtaaatctac gcggagactc tatgggaagc     300
   cgagaaccag tgtcttcttc cagggcagaa gtcacctgtt gggaacggcc cccgggtccc     360
   cctgctgggc tttccggctc ttctaggcgg cctgatttct cctcagccct ccacccagcg     420
   tccctcaggg acttttcaca cctccccacc cccatttcca ctacagtctc ccagggcaca     480
   gcacttcatt gacagccaca cgagccttct cgttctcttc tcctctgttc cttctctttc     540
   tcttctcctc tgttccttct ctttctctgt cataatttcc ttggtgcttt cgccacctta     600
   aacaaaaaag agaaaaaaat aaaataaaaa aaacccattc tgagccaaag tattttaaga     660
   tgaatccaag aaagcgaccc acatagccct ccccacccac ggagtgcgcc aagacgcacc     720
   caggctccat cacagggccg agagcagcgc cactctggtc gtacttttgg gtcaagagat     780
   cttgcaaaag agg                                                        793
<210> 18
   <211> 492
   <212> DNA
   <213> Homo sapiens
<220>
   <221> misc_feature
   <223> sequence of STAR18
<400> 18
   atctttttgc tctctaaatg tattgatggg ttgtgttttt tttcccacct gctaataaat      60
   attacattgc aacattcttc cctcaacttc aaaactgctg aactgaaaca atatgcataa     120
   aagaaaatcc tttgcagaag aaaaaaagct attttctccc actgattttg aatggcactt     180
   gcggatgcag ttcgcaaatc ctattgccta ttccctcatg aacattgtga aatgaaacct     240
   ttggacagtc tgccgcattg cgcatgagac tgcctgcgca aggcaagggt atggttccca     300
   aagcacccag tggtaaatcc taacttatta ttcccttaaa attccaatgt aacaacgtgg     360
   gccataaaag agtttctgaa caaaacatgt catctttgtg gaaaggtgtt tttcgtaatt     420
   aatgatggaa tcatgctcat ttcaaaatgg aggtccacga tttgtggcca gctgatgcct     480
   gcaaattatc ct                                                         492
<210> 19
   <211> 1840
   <212> DNA
   <213> Homo sapiens
<220>
   <221> misc_feature
   <223> sequence of STAR19
<400> 19
   tcacttcctg atattttaca ttcaaggcta gctttatgca tatgcaacct gtgcagttgc     60
   acagggcttt gtgttcagaa agactagctc ttggtttaat actctgttgt tgccatcttg    120
   agattcatta taatataatt tttgaatttg tgttttgaac gtgatgtcca atgggacaat    180
   ggaacattca cataacagag gagacaggtc aggtggcagc ctcaattcct tgccaccctt    240
   ttcacataca gcattggcaa tgccccatga gcacaaaatt tgggggaacc atgatgctaa    300
   gactcaaagc acatataaac atgttacctc tgtgactaaa agaagtggag gtgctgacag    360
   cccccagagg ccacagttta tgttcaaacc aaaacttgct tagggtgcag aaagaaggca    420
   atggcagggt ctaagaaaca gcccatcata tccttgttta ttcatgttac gtccctgcat    480
   gaactaatca cttacactga aaatattgac agaggaggaa atggaaagat agggcaaccc    540
   atagttcttt ttccttttag tctttcctta tcagtaaacc aaagatagta ttggtaaaat 600
   gtgtgtgagt taattaatga gttagtttta ggcagtgttt ccactgttgg ggtaagaaca 660
   aaatatatag gcttgtattg agctattaaa tgtaaattgt ggaatgtcag tgattccaag 720
   tatgaattaa atatccttgt atttgcattt aaaattggca ctgaacaaca aagattaaca 780
   gtaaaattaa taatgtaaaa gtttaatttt tacttagaat gacattaaat agcaaataaa 840
   agcaccatga taaatcaaga gagagactgt ggaaagaagg aaaacgtttt tattttagta 900
   tatttaatgg gactttcttc ctgatgtttt gttttgtttt gagagagagg gatgtggggg 960
   cagggaggtc tcattttgtt gcccaggctg gacttgaact cctgggctcc agctatcctg 1020
   ccttagcttc ttgagtagct gggactacag gcacacacca cagtgtctga cattttctgg 1080
   attttttttt tttttttatt ttttttgtga gacaggttct ggctctgtta ctcaggttgc 1140
   agtgcagtgg catgatagcg gctcactgca gcctcaacct cctcagctta agctactctc 1200
   ccacttcagc ctcctgagta gccaggacta cagttgtgtg ccaccacacc tgtggctaat 1260
   ttttgtagag atggggtctc tccacgttgc cgaggctggt ctccaactcc tggtctcaag 1320
   cgaacctcct gacttggcct cccgaagtgc tgggattaca ggcttgagcc actgcatcca 1380
   gcctgtcctc tgtgttaaac ctactccaat ttgtctttca tctctacata aacggctctt 1440
   ttcaaagttc ccatagacct cactgttgct aatctaataa taaattatct gccttttctt 1500
   acatggttca tcagtagcag cattagattg ggctgctcaa ttcttcttgg tatattttct 1560
   tcatttggct tctggggcat cacactctct ttgagttact cattcctcat tgatagcttc 1620
   ttcctagtct tctttactgg ttcttcctct tctccctgac tccttaatat tgtttttctc 1680
   cccaggcttt agttcttagt cctcttctgt tatctattta cacccaattc tttcagagtc 1740
   tcatccagag tcatgaactt aaacctgttt ctgtgcagat aattcacatt attatatctc 1800
   cagcccagac tctcccgcaa actgcagact gatcctactg 1840
<210> 20
   <211> 780
   <212> DNA
   <213> Homo sapiens
<220>
   <221> misc_feature
   <223> sequence of STAR20
<400> 20
   gatctcaagt ttcaatatca tgttttggca aaacattcga tgctcccaca tccttaccta      60
   aagctaccag aaaggctttg ggaactgtca acagagctac agaaaagtca gtaaagacca     120
   atggacccct caaacaaaaa cagccaagct tttctgccaa aaagatgact gagaagactg     180
   ttaaagcaaa aaactctgtt cctgcctcag atgatggcta tccagaaata gaaaaattat     240
   ttcccttcaa tcctctaggc ttcgagagtt ttgacctgcc tgaagagcac cagattgcac     300
   atctcccctt gagtgaagtg cctctcatga tacttgatga ggagagagag cttgaaaagc     360
   tgtttcagct gggcccccct tcacctttga agatgccctc tccaccatgg aaatccaatc     420
   tgttgcagtc tcctttaagc attctgttga ccctggatgt tgaattgcca cctgtttgct     480
   ctgacataga tatttaaatt tcttagtgct ttagagtttg tgtatatttc tattaataaa     540
   gcattatttg tttaacagaa aaaaagatat atacttaaat cctaaaataa aataaccatt     600
   aaaaggaaaa acaggagtta taactaataa gggaacaaag gacataaaat gggataataa     660
   tgcttaatcc aaaataaagc agaaaatgaa gaaaaatgaa atgaagaaca gataaataga     720
   aaacaaatag caatatgaaa gacaaacttg accgggtgtg gtggctgatg cctgtaatcc     780
<210> 21
   <211> 607
   <212> DNA
   <213> Homo sapiens
<220>
   <221> misc_feature
   <223> sequence of STAR21
<400> 21
   gatcaataat ttgtaatagt cagtgaatac aaaggggtat atactaaatg ctacagaaat      60
   tccattcctg ggtataaatc ctagacatat ttatgcatat gtacaccaag atatatctgc     120
   aagaatgttc acagcaaatc tctttgtagt agcaaaaggc caaaaggtct atcaacaaga     180
   aaattaatac attgtggcac ataatggcat ccttatgcca ataaaaatgg atgaaattat     240
   agttaggttc aaaaggcaag cctccagata atttatatca tataattcca tgtacaacat     300
   tcaacaacaa gcaaaactaa acatatacaa atgtcaggga aaatgatgaa caaggttaga     360
   aaatgattaa tataaaaata ctgcacagtg ataacattta atgagaaaaa aagaaggaag     420
   ggcttaggga gggacctaca gggaactcca aagttcatgg taagtactaa atacataatc     480
   aaagcactca aaatagaaaa tattttagta atgttttagc tagttaatat cttacttaaa     540
   acaaggtcta ggccaggcac ggtggctcac acctgtaatc ccagcacttt gggaggctga     600
   ggcgggt                                                                607
<210> 22
   <211> 1380
   <212> DNA
   <213> Homo sapiens
<220>
   <221> misc_feature
   <223> sequence of STAR22
<400> 22
   cccttgtgat ccacccgcct tggcctccca aagtgctggg attacaggcg tgagtcacta     60
   cgcccggcca ccctccctgt atattatttc taagtatact attatgttaa aaaaagttta    120
   aaaatattga tttaatgaat tcccagaaac taggatttta catgtcacgt tttcttatta    180
   taaaaataaa aatcaacaat aaatatatgg taaaagtaaa aagaaaaaca aaaacaaaaa    240
   gtgaaaaaaa taaacaacac tcctgtcaaa aaacaacagt tgtgataaaa cttaagtgcc     300
   tgaaaattta gaaacatcct tctaaagaag ttctgaataa aataaggaat aaaataatca    360
   catagttttg gtcattggtt ctgtttatgt gatggattat gtttattgat ttgtgtatgt    420
   tgaacttatc tcaatagatg cagacaaggc cttgataaaa gtttttaaca ccttttcatg    480
   ttgaaaactc tcaatagact aggtattgat gaaacatatc tcaaaataat agaagctatt    540
   tatgataaac ccatagccaa tatcatactg agtgggcaaa agctggaagc attccctttg    600
   aaaactggca caagacaagg atgccctctc tcaccactcc tattaaatgt agtattggaa    660
   gttctggcca gagcaatcag gcaggagaaa gaaaaggtat taaaatagga agagaggaag    720
   tcaaattgtc tctgtttgca gtaaacatga ttgtatattt agaaaacccc attgtctcat    780
   cctaaaaact ccttaagctg ataaacaact tcagcaaagt ctcaggatac aaaatcaatg    840
   tgcaaaaatc acaagcattc ctatacaccg ataatagaca gcagagagcc aaatcatgag    900
   tgaagtccca ttcacaattg cttcaaagaa aataaaatac ttaggaatac aactttcacg    960
   ggacatgaag gacattttca aggacaacta aaaaccactg ctcaaggaaa tgagagagga   1020
   cacaaagaaa tggaaaaaca ttccatgctc atggaagaat caatatcatg aaaatggcca   1080
   tactgcccaa agtaatttat agattcaatg ctaaccccat caagccacca ttgactttct   1140
   tcacagaact agaaaaaaac tattttaaaa ctcatatgta gtcaaaaaga gtcggtatag   1200
   ccaagacaat cctaagcata aagaacaaag ctggatgcat cacgctgact tcaaaccata   1260
   ctacaaggct acagtaacca aaacagcatg gtactggtac caaaacagat agatagaccg   1320
   atagaacaga acagaggcct cggaaataac accacacatc tacaaccctt tgatcttcaa   1380
<210> 23
   <211> 1246
   <212> DNA
   <213> Homo sapiens
<220>
   <221> misc_feature
   <223> sequence of STAR23
<400> 23
   atcccctcat ccttcagggc agctgagcag ggcctcgagc agctggggga gcctcactta      60
   atgctcctgg gagggcagcc agggagcatg gggtctgcag gcatggtcca gggtcctgca    120
   ggcggcacgc accatgtgca gccgccccca cctgttgctc tgcctccgcc acctggccat    180
   gggcttcagc agccagccac aaagtctgca gctgctgtac atggacaaga agcccacaag    240
   cagctagagg accttgtgtt ccacgtgccc agggagcatg gcccacagcc caaagaccag    300
   tcaggagcag gcaggggctt ctggcaggcc cagctctacc tctgtcttca cacagatggg    360
   agatttctgt tgtgattttg agtgatgtgc ccctttggtg acatccaaga tagttgctga    420
   agcaccgctc taacaatgtg tgtgtattct gaaaacgaga acttctttat tctgaaataa    480
   ttgatgcaaa ataaattagt ttggatttga aattctattc atgtaggcat gcacacaaaa    540
   gtccaacatt gcatatgaca caaagaaaag aaaaagcttg cattccttaa atacaaatat    600
   ctgttaacta tatttgcaaa tatatttgaa tacacttcta ttatgttaca tataatatta    660
   tatgtatatg tatatataat atacatatat atgttacata taatatactt ctattatgtt    720
   acatataata tttatctata agtaaataca taaatataaa gatttgagta gctgtagaac    780
   attgtcttat gtgttatcag ctactactac aaaaatatct cttccactta tgccagtttg    840
   ccatataaat atgatcttct cattgatggc ccagggcaag agtgcagtgg gtacttattc    900
   tctgtgagga gggaggagaa aagggaacaa ggagaaagtc acaaagggaa aactctggtg    960
   ttgccaaaat gtcaagtttc acatattccg agacggaaaa tgacatgtcc cacagaagga   1020
   ccctgcccag ctaatgtgtc acagatatct caggaagctt aaatgatttt tttaaaagaa   1080
   aagagatggc attgtcactt gtttcttgta gctgaggctg tgggatgatg cagatttctg   1140
   gaaggcaaag agctcctgct ttttccacac cgagggactt tcaggaatga ggccagggtg   1200
   ctgagcacta caccaggaaa tccctggaga gtgtttttct tactta                  1246
<210> 24
   <211> 939
   <212> DNA
   <213> Homo sapiens
<220>
   <221> misc_feature
   <223> sequence of STAR24
<400> 24
   acgaggtcac gagttcgaga ccagcctggc caagatggtg aagccctgtc tctactaaaa 60
   atacaacaag tagccgggcg cggtgacggg cgcctgtaat cccagctact caggaggctg 120
   aagcaggaga atctctagaa cccaggaggc ggaggtgcag tgagctgaga ctgccccgct 180
   gcactctagc ctgggcaaca cagcaagact ctgtctcaaa taaataaata aataaataaa 240
   taaataaata aataaataaa tagaaaggga gagttggaag tagatgaaag agaagaaaag 300
   aaatcctaga tttcctatct gaaggcacca tgaagatgaa ggccacctct tctgggccag 360
   gtcctcccgt tgcaggtgaa ccgagttctg gcctccattg gagaccaaag gagatgactt 420
   tggcctggct cctagtgagg aagccatgcc tagtcctgtt ctgtttgggc ttgatcctgt 480
   atcacttgat tgtctctcct ggactttcca tggattccag ggatgcaact gagaagttta 540
   tttttaatgc acttacttga agtaagagtt attttaaaac attttagcaa aggaaatgaa 600
   ttctgacagg ttttgcactg aagacattca catgtgagga aaacaggaaa accactatgc 660
   tagaaaaagc aaatgctgtt gagattgtct cacaaacaca aattgcgtgc cagcaggtag 720
   gtttgagcct caggttgggc acattttacc ttaagcgcac tgttggtgga acttaaggtg 780
   actgtaggac ttatatatac atacatacat ataatatata tacatattta tgtgtatata 840
   cacacacaca cacacacaca cacacagggt cttgctatct tgcccagggt ggtctccaac 900
   tctgggtctc aagcgatcct ctgcctcccc ttcccaaag 939
<210> 25
   <211> 1067
   <212> DNA
   <213> Homo sapiens
<220>
   <221> misc_feature
   <223> sequence of STAR25
<400> 25
   cagcccctct tgtgtttttc tttatttctc gtacacacac gcagttttaa gggtgatgtg     60
   tgtataatta aaaggaccct tggcccatac tttcctaatt ctttagggac tgggattggg    120
   tttgactgaa atatgttttg gtggggatgg gacggtggac ttccattctc cctaaactgg    180
   agttttggtc ggtaatcaaa actaaaagaa acctctggga gactggaaac ctgattggag    240
   cactgaggaa caagggaatg aaaaggcaga ctctctgaac gtttgatgaa atggactctt    300
   gtgaaaatta acagtgaata ttcactgttg cactgtacga agtctctgaa atgtaattaa    360
   aagtttttat tgagcccccg agctttggct tgcgcgtatt tttccggtcg cggacatccc    420
   accgcgcaga gcctcgcctc cccgctgccc tcagcctccg atgacttccc cgcccccgcc    480
   ctgctcggtg acagacgttc tactgcttcc aatcggaggc acccttcgcg ggagcggcca    540
   atcgggagct ccggcaggcg gggaggccgg gccagttaga tttggaggtt caacttcaac    600
   atggccgaag caagtagcgc caatctaggc agcggctgtg aggaaaaaag gcatgagggg    660
   tcgtcttcgg aatctgtgcc acccggcact accatttcga gggtgaagct cctcgacacc    720
   atggtggaca cttttcttca gaagctggtc gccgccggca ggtaaagtgg acgcagccgc    780
   ggtgggagtg tttgttggca ccgaagctca aatcccgcga ggtcaggacg gccgcaggct    840
   ggcgcgcggt gacgtgggtc cgcgttgggg gcggggcagt cggacgaggc gacccagtca    900
   aatcctgagc cttaggagtc agggtattca cgcactgata acctgtagcg gaccgggata    960
   gctagctact ccttcctaca ggaagccccg ttttcactaa aatttcaggt ggttgggagg   1020
   aaagatagag cctttgcaaa ttagagcagg gttttttatt tttttat                1067
<210> 26
   <211> 540
   <212> DNA
   <213> Homo sapiens
<220>
   <221> misc_feature
   <223> sequence of STAR26
<400> 26
   ccccctgaca agccccagtg tgtgatgttc cccactctgt gtccatgcat tctcattgtt      60
   caactcccat ctgtgagtga gaacatgcag tgtttggttt tctgtccttg agatagtttg     120
   ctgagaatga tggtttccag cttcatccat gtccttgcaa aggaagtgaa cttatccttt     180
   tttatggctt catagtattc catggcacat atgtgccaca tttttttaat ccagtctatc    240
   attgatggac atttgggttg gttccaagtc tttgctattg tgaatagcac cacaattaac     300
   atatgtgtgc atgtatacat ctttatagta gcatgattta taatccttcg ggtatatacc     360
   ctgtaatggg atcgctgggt caaatggtat ttctagttct agatccttga ggaatcacca     420
   cactgctttc cacaatggtt gaactaattt acgctcccac cagcagtgta aaagcattcc     480
   tatttctcca cgtcctctcc agtatctgtt gtttcctgac tttttaatga tcatcattct     540
<210> 27
   <211> 1520
   <212> DNA
   <213> Homo sapiens
<220>
   <221> misc_feature
   <223> sequence of STAR27
<400> 27
   cttggccctc acaaagcctg tggccaggga acaattagcg agctgcttat tttgctttgt      60
   atccccaatg ctgggcataa tgcctgccat tatgagtaat gccggtagaa gtatgtgttc     120
   aaggaccaaa gttgataaat accaaagaat ccagagaagg gagagaacat tgagtagagg     180
   atagtgacag aagagatggg aacttctgac aagagttgtg aagatgtact aggcaggggg     240
   aacagcttaa ggagagtcac acaggaccga gctcttgtca agccggctgc catggaggct     300
   gggtggggcc atggtagctt tcccttcctt ctcaggttca gagtgtcagc cttgaacttc     360
   taattcccag aggcatttat tcaatgtttt cttctagggg catacctgcc ctgctgtgga     420
   agactttctt ccctgtgggt cgccccagtc cccagatgag acggtttggg tcagggccag     480
   gtgcaccgtt gggtgtgtgc ttatgtctga tgacagttag ttactcagtc attagtcatt     540
   gagggaggtg tggtaaagat ggagatgctg ggtcacatcc ctagagaggt gttccagtat     600
   gggcacatgg gagggctgga aggataggtt actgctagac gtagagaagc cacatccttt     660
   aacaccctgg cttttcccac tgccaagatc cagaaagtcc ttgtggtttc gctgctttct     720
   cctttttttt tttttttttt tttctgagat ggagtctggc tctgtcgccc aggctggagt    780
   gcagtggcac gatttcggct cactgcaagt tccgcctcct aggttcatac cattctccca    840
   cctcagcctc ccgagtagct gggactacag gcgccaccac acccagctaa ttttttgtat    900
   ttttagtaga gacggcgttt caccatgtta gccaggatgg tcttgatccg cctgcctcag    960
   cctcccaaag tgctgggatt acaggcgtga gccaccgcgc ccggcctgct ttcttctttc   1020
   atgaagcatt cagctggtga aaaagctcag ccaggctggt ctggaactct tgacctcaag   1080
   tgatctgcct gcctcagcct cccaaagtgc tgagattaca ggcatgagcc agtccgaatg   1140
   tggctttttt tgttttgttt tgaaacaagg tctcactgtt gcccaggctg cagtgcagtg   1200
   gcatacctca gctccactgc agcctcgacc tcctgggctc aagcaatcct cccaactgag   1260
   cctccccagt agctggggct acaagcgcat gccaccacgc ctggctattt tttttttttt   1320
   tttttttttt gagaaggagt ttcattcttg ttgcccaggc tggagtgcaa tggcacagtc   1380
   tcagctcact gcagcctccg cctcctgggt tcaagcgatt ctcctgcctc agcctcccga   1440
   gtagctggga ttataggcac ctgccaccat gcctggctaa tttttttgta tttttagtag   1500
   ggatggggtt tcaccatgtt                                               1520
<210> 28
   <211> 961
   <212> DNA
   <213> Homo sapiens
<220>
   <221> misc_feature
   <223> sequence of STAR28
<400> 28
   aggaggttat tcctgagcaa atggccagcc tagtgaactg gataaatgcc catgtaagat     60
   ctgtttaccc tgagaagggc atttcctaac tctccctata aaatgccaag tggagcaccc    120
   cagatgaaat agctgatatg ctttctatac aagccatcta ggactggctt tatcatgacc    180
   aggatattca cccactgaat atggctatta cccaagttat ggtaaatgct gtagttaagg    240
   gggtcccttc cacatggaca ccccaggtta taaccagaaa gggttcccaa tctagactcc    300
   aagagagggt tcttagacct catgcaagaa agaacttggg gcaagtacat aaagtgaaag    360
   caagtttatt aagaaagtaa agaaacaaaa aaatggctac tccataagca aagttatttc    420
   tcacttatat gattaataag agatggatta ttcatgagtt ttctgggaaa ggggtgggca    480
   attcctggaa ctgagggttc ctcccacttt tagaccatat agggtatctt cctgatattg    540
   ccatggcatt tgtaaactgt catggcactg atgggagtgt cttttagcat tctaatgcat    600
   tataattagc atataatgag cagtgaggat gaccagaggt cacttctgtt gccatattgg    660
   tttcagtggg gtttggttgg cttttttttt tttttaacca caacctgttt tttatttatt    720
   tatttattta tttatttatt tatatttttt attttttttt agatggagtc ttgctctgtc    780
   acccaggtta gagtgcagtg gcaccatctc ggctcactgc aagctctgcc tccttggttc    840
   acgccattct gctgcctcag cctcccgagt agctgggact acaggtgcct gccaccatac    900
   ccggctaatt ttttctattt ttcagtagag acggggtttc accgtgttag ccaggatggt    960
   c                                                                    961
<210> 29
   <211> 2233
   <212> DNA
   <213> Homo sapiens
<220>
   <221> misc_feature
   <223> sequence of STAR29
<400> 29
   agcttggaca cttgctgatg ccactttgga tgttgaaggg ccgccctctc ccacaccgct     60
   ggccactttt aaatatgtcc cctctgccca gaagggcccc agaggagggg ctggtgaggg    120
   tgacaggagt tgactgctct cacagcaggg ggttccggag ggaccttttc tccccattgg    180
   gcagcataga aggacctaga agggccccct ccaagcccag ctgggcgtgc agggccagcg    240
   attcgatgcc ttcccctgac tcaggtggcg ctgtcctaaa ggtgtgtgtg ttttctgttc    300
   gccagggggt ggcggataca gtggagcatc gtgcccgaag tgtctgagcc cgtggtaagt    360
   ccctggaggg tgcacggtct cctccgactg tctccatcac gtcaggcctc acagcctgta    420
   ggcaccgctc ggggaagcct ctggatgagg ccatgtggtc atccccctgg agtcctggcc    480
   tggcctgaag aggaggggag gaggaggcca gcccctccct agccccaagg cctgcgaggc    540
   tgcaagcccg gccccacatt ctagtccagg cttggctgtg caagaagcag attgcctggc    600
   cctggccagg cttcccagct aggatgtggt atggcagggg tgggggacat tgaggggctg    660
   ctgtagcccc cacaacctcc ccaggtaggg tggtgaacag taggctggac aagtggacct    720
   gttcccatct gagattcaag agcccacctc tcggaggttg cagtgagccg agatccctcc    780
   actgcactcc agcctgggca acagagcaag actctgtctc aaaaaaacag aacaacgaca    840
   acaaaaaacc cacctctggc ccactgccta actttgtaaa taaagtttta ttggcacata    900
   gacacaccca ttcatttaca tactgctgcg gctgcttttg cattaccctt gagtagacga    960
   cagaccacgt ggccatggaa gccaaaaata tttactgtct ggccctttac agaagtctgc   1020
   tctagaggga gaccccggcc catggggcag gaccactggg cgtgggcaga agggaggcct   1080
   cggtgcctcc acgggcctag ttgggtatct cagtgcctgt ttcttgcatg gagcaccagg   1140
   ggtcagggca agtacctgga ggaggcaggc tgttgcccgc ccagcactgg gacccaggag   1200
   accttgagag gctcttaacg aatgggagac aagcaggacc agggctccca ttggctgggc   1260
   ctcagtttcc ctgcctgtaa gtgagggagg gcagctgtga aggtgaactg tgaggcagag   1320
   cctctgctca gccattgcag gggcggctct gccccactcc tgttgtgcac ccagagtgag   1380
   gggcacgggg tgagatgtca ccatcagccc ataggggtgt cctcctggtg ccaggtcccc   1440
   aagggatgtc ccatcccccc tggctgtgtg gggacagcag agtccctggg gctgggaggg   1500
   ctccacactg ttttgtcagt ggtttttctg aactgttaaa tttcagtgga aaattctctt   1560
   tcccctttta ctgaaggaac ctccaaagga agacctgact gtgtctgaga agttccagct   1620
   ggtgctggac gtcgcccaga aagcccaggt actgccacgg gcgccggcca ggggtgtgtc   1680
   tgcgccagcc atgggcacca gccaggggtg tgtctacgcc ggccaggggt aggtctccgc   1740
   cggcctccgc tgctgcctgg ggagggccgt gcctgacact gcaggcccgg tttgtccgcg   1800
   gtcagctgac ttgtagtcac cctgcccttg gatggtcgtt acagcaactc tggtggttgg   1860
   ggaaggggcc tcctgattca gcctctgcgg acggtgcgcg agggtggagc tcccctccct   1920
   ccccaccgcc cctggccagg gttgaacgcc cctgggaagg actcaggccc gggtctgctg   1980
   ttgctgtgag cgtggccacc tctgccctag accagagctg ggccttcccc ggcctaggag   2040
   cagccgggca ggaccacagg gctccgagtg acctcagggc tgcccgacct ggaggccctc   2100
   ctggcgtcgc ggtgtgactg acagcccagg agcgggggct gttgtaattg ctgtttctcc   2160
   ttcacacaga accttttcgg gaagatggct gacatcctgg agaagatcaa gaagtaagtc   2220
   ccgcccccca ccc                                                      2233
<210> 30
   <211> 1851
   <212> DNA
   <213> Homo sapiens
<220>
   <221> misc_feature
   <223> sequence of STAR30
<400> 30
   gggtgcattt ccacccaggg gacacttggc aatggtggga gacattgctt gttgtcacaa 60
   ctgggcatgg gagtgctgct gcgtctagtg ggtagaggcc agagatgctc ctaatatcct 120
   acaaggcaca gaacagcccc ccacaacaga gaattatcca gcctgaaaat gtccacagtg 180
   ctgaggttgg gaaaccctat tctagagcca acaggctgtg aagcttgact catggttcca 240
   tcaccaatag ctgcgtgacc ttggtgagtt ccttagctgc tctgtgcctc ggattcatgg 300
   taggttttcc ttgttaggtt taaatgagtg aagttataca gagggcctga agtctcatgg 360
   tattttacta gagcctcatt gtgttttagt tataattaga aattgggtaa ggtaaggaca 420
   cagaagaagc catctgatct gggggcttca cacttagaag tgacctcgga gcaattgtat 480
   tggggtggaa agggactaac agccaggagc agagggcaca ttggaattgg ggccagaggg 540
   cacagactgc cttgtccatc aggcatagca atggacagag gaaggggaat gactagttat 600
   ggctgcaagg ccaagtacag gggacttatt tctcatatct atctatctat ctacctaccg 660
   tctatttatc tatcatctat ctacttattt atctatctat ttatgcatgt gtaccaaccg 720
   aaagttttag taaatgcaca aactgcgata taatgaaaat ggaaattttc aaaagaagag 780
   aaatcacctg ccacctgact accttaacaa atgagtggtt ttcatctctc cttccaggcc 840
   tgtcattttt acagtgcttt agtcataaaa caggtcctct attctattgt tttatgtcac 900
   atgaaattgt accataagca ttttccatga tgtgactcca ctgtttcatt ttccattttt 960
   ttccagaatg aagataacct cattgttttt ttcctgattg taaaaatgct ctgtgctctt 1020
   tttttttttt tttaacaatg caggcagtac caaaaagtat gaagaagaat gtaatagttc 1080
   ccatttccca tctcactctt taaggccagc attttggtga acatccatcc gaacaaatct 1140
   ccacgcgttt atcaatttgt tgacttactc cttcttttat gtaaatatga acatgattta 1200
   actgccagtc catttggaac cttaaagtga aggtttttta ttgttggggt ttgctatggt 1260
   ctgaatatgt gtgtcccccc aaaatttatg ttgaatccta acgcccaatg cgattaggag 1320
   gtggggccat taggaggtga ttaagtcatg aagtcatcag ccctaatgaa tgggatttgt 1380
   ggccttgaaa agggacccca gagagctgcc ttgccccttc tgccatgtaa ggacacagtg 1440
   aggagctagg aagggggcct cagcagagac caaatgtgat ggtgcctcga tattggactt 1500
   cccagcctcc agaatgtgag aaatgaattt ctgttgttta taagtcaccc agtctatagt 1560
   attttgttct agcagcccaa acagactaag tcagggttgt tgttttagga agtggggaat 1620
   ggggccatgc atgggtgtac gccagaacaa aggaagccag caagtcctga aagatactgg 1680
   aaaagggaat agtgggcacg tgcagtgtgt tagtttcctg aggctgctat aacaaagcac 1740
   cacaggttgg gtggcttaaa taacagaaat tcattctccc atcattctgg ggaccagacg 1800
   tctgaaatca agactcctat gccatgctcc ttctgaaggc tccaggggag g 1851
<210> 31
   <211> 1701
   <212> DNA
   <213> Homo sapiens
<220>
   <221> misc_feature
   <223> sequence of STAR31
<220>
   <221> misc_feature
   <222> (159)..(1696)
   <223> n is a, c, g, or t on various positions
<400> 31
   cacccgcctt ggccccccag agtgctggga ttacaagtgt aaaccaccat tcctggctag 60
   atttaatttt ttaaaaaata aagagaagta ggaatagttc attttaggga gagcccctta     120
   actgggacag gggcaggaca ggggtgaggc ttcccttant tcaagctcac ctcaaaccca     180
   cccaggactg tgtgtcacat tctccaataa aggaaaggtt gctgcccccg cctgtgagtg     240
   ctgcagtgga gggtagaggg ccgtgggcag agtgcttcat ggactgctca tcaagaaagg     300
   cttcatgaca atcggcccag ctgctgtcat cccacattct acttccagct aggagaaggc     360
   ggcttgccca cagtcaccca gccggcaagt gtcacccctg ggttggaccc agagctatga     420
   tcctgcccag gggtccagct gagaatcagg cccacgttct aggcagaggg gctcacctac      480
   tgggactcca gtagctgtag tgcatggagg catcatggct gcagcagcct ggacctggtc     540
   tcacactggc tgtccctgtg ggcaggccat cctcaatgcc aggtcaggcc caagcatgta     600
   tcccagacaa tgacaatggg gtggaatcct ctcttgtccc agaagccact cctcactgtt     660
   ctacctgagg aaggcagggg catggtggaa tcctgaagcc tgctgtgagg gtctccagcg     720
   aacttgcaca tggtcagccc tgccttctcc tccctgaact agattgagcg agagcaagaa     780
   ggacattgaa ccagcaccca aagaattttg gggaacggcc tctcatccag gtcaggctca     840
   cctccttttt aaaatttaat taattaatta attaattttt ttttagagac agagtcttac     900
   tgtgtggccc aggctgtagt gcagtggcac aatcatagtt cactgcagcc tcaaactccc     960
   cacctcagcc tctggattag ctgagactac aggtgcacca ccaccacacc cagctaatat    1020
   ttttattttt gtagagagag ggtttcacca tcttgcccag gctggtctca aactcctggg    1080
   ctcaagtgat cccgcccagg tctgaaagcc cccaggctgg cctcagactg tggggttttc    1140
   catgcagcca cccgagggcg cccccaagcc agttcatctc ggagtccagg cctggccctg    1200
   ggagacagag tgaaaccagt ggtttttatg aacttaactt agagtttaaa agatttctac    1260
   tcgatcactt gtcaagatgc gccctctctg gggagaaggg aacgtgactg gattccctca    1320
   ctgttgtatc ttgaataaac gctgctgctt catcctgtgg gggccgtggc cctgtccctg    1380
   tgtgggtggg gcctcttcca tttccctgac ttagaaacca cagtccacct agaacagggt    1440
   ttgagaggct tagtcagcac tgggtagcgt tttgactcca ttctcggctt tcttcttttt    1500
   ctttccagga tttttgtgca gaaatggttc ttttgttgcc gtgttagtcc tccttggaag    1560
   gcagctcaga aggcccgtga aatgtcgggg gacaggaccc ccagggaggg aaccccaggc    1620
   tacgcacttt agggttcgtt ctccagggag ggcgacctga cccccgnatc cgtcggngcg    1680
   cgnngnnacn aannnnttcc c                                              1701
<210> 32
   <211> 771
   <212> DNA
   <213> Homo sapiens
<220>
   <221> misc_feature
   <223> sequence of STAR32
<400> 32
   gatcacacag cttgtatgtg ggagctagga ttggaacccc agaagtctgg ccccaggttc      60
   atgctctcac ccactgcata caatggcctc tcataaatca atccagtata aaacattaga     120
   atctgcttta aaaccataga attagtagcg taagtaataa atgcagagac catgcagtga     180
   atggcattcc tggaaaaagc ccccagaagg aattttaaat cagctttcgt ctaatcttga     240
   gcagctagtt agcaaatatg agaatacagt tgttcccaga taatgcttta tgtctgacca     300
   tcttaaactg gcgctgtttt tcaaaaactt aaaaacaaaa tccatgactc ttttaattat     360
   aaaagtgata catgtctact tgggaggctg aggtggtggg aggatggctt gagtttgagg     420
   ctgcagtatg ctactatcat gcctataaat agccgctgca ttccagcttg ggcaacatac     480
   ccaggcccta tctcaaaaaa ataaaaagta atacatctac attgaagaaa attaatttta     540
   ttgggttttt ttgcattttt attatacaca gcacacacag cacatatgaa aaaatgggta     600
   tgaactcagg cattcaactg gaagaacagt actaaatcaa tgtccatgta gtcagcgtga     660
   ctgaggttgg tttgtttttt cttttttctt ctcttctctt ctcttttctt tttttttgag      720
   acggagcttt gctctttttg cccaggcttg attgcaatgg cgtgatctca g               771
<210> 33
   <211> 1368
   <212> DNA
   <213> Homo sapiens
<220>
   <221> misc_feature
   <223> sequence of STAR33
<400> 33
   gcttttatcc tccattcaca gctagcctgg cccccagagt acccaattct ccctaaaaaa      60
   cggtcatgct gtatagatgt gtgtggcttg gtagtgctaa agtggccaca tacagagctc     120
   tgacaccaaa cctcaggacc atgttcatgc cttctcactg agttctggct tgttcgtgac     180
   acattatgac attatgatta tgatgacttg tgagagcctc agtcttctat agcactttta     240
   gaatgcttta taaaaaccat ggggatgtca ttatattcta acctgttagc acttctgttc     300
   gtattaccca tcacatccca acatcaattc tcatatatgc aggtacctct tgtcacgcgc     360
   gtccatgtaa ggagaccaca aaacaggctt tgtttgagca acaaggtttt tatttcacct     420
   gggtgcaggt gggctgagtc tgaaaagaga gtcagtgaag ggagacaggg gtgggtccac     480
   tttataagat ttgggtaggt agtggaaaat tacaatcaaa gggggttgtt ctctggctgg     540
   ccagggtggg ggtcacaagg tgctcagtgg gagagccttt gagccaggat gagccagaag     600
   gaatttcaca aggtaatgtc atcagttaag gcagggactg gccattttca cttcttttgt     660
   ggtggaatgt catcagttaa ggcaggaacc ggccattttc acttcttttg tgattcttca    720
   cttgcttcag gccatctgga cgtataggtg caggtcacag tcacagggga taagatggca    780
   atggcatagc ttgggctcag aggcctgaca cctctgagaa actaaagatt ataaaaatga    840
   tggtcgcttc tattgcaaat ctgtgtttat tgtcaagagg cacttatttg tcaattaaga    900
   acccagtggt agaatcgaat gtccgaatgt aaaacaaaat acaaaacctc tgtgtgtgtg    960
   tgtgtgtgag tgtgtgtgta tgtgtgtgtg tgtgtattag agaggaaaag cctgtatttg   1020
   gaggtgtgat tcttagattc taggttcttt cctgcccacc ccatatgcac ccaccccaca   1080
   aaagaacaaa caacaaatcc caggacatct tagcgcaaca tttcagtttg catattttac   1140
   atatttactt ttcttacata ttaaaaaact gaaaatttta tgaacacgct aagttagatt   1200
   ttaaattaag tttgttttta cactgaaaat aatttaatat ttgtgaagaa tactaataca   1260
   ttggtatatt tcattttctt aaaattctga acccctcttc ccttatttcc ttttgacccg   1320
   attggtgtat tggtcatgtg actcatggat ttgccttaag gcaggagg                1368
<210> 34
   <211> 755
   <212> DNA
   <213> Homo sapiens
<220>
   <221> misc_feature
   <223> sequence of STAR34
<400> 34
   actgggcacc ctcctaggca ggggaatgtg agaactgccg ctgctctggg gctgggcgcc     60
   atgtcacagc aggagggagg acggtgttac accacgtggg aaggactcag ggtggtcagc    120
   cacaaagctg ctggtgatga ccaggggctt gtgtcttcac tctgcagccc taacacccag    180
   gctgggttcg ctaggctcca tcctgggggt gcagaccctg agagtgatgc cagtgggagc    240
   ctcccgcccc tccccttcct cgaaggccca ggggtcaaac agtgtagact cagaggcctg    300
   agggcacatg tttatttagc agacaaggtg gggctccatc agcggggtgg cctggggagc     360
   agctgcatgg gtggcactgt ggggagggtc tcccagctcc ctcaatggtg ttcgggctgg    420
   tgcggcagct ggcggcaccc tggacagagg tggatatgag ggtgatgggt ggggaaatgg    480
   gaggcacccg agatggggac agcagaataa agacagcagc agtgctgggg ggcaggggga    540
   tgagcaaagg caggcccaag acccccagcc cactgcaccc tggcctccca caagccccct    600
   cgcagccgcc cagccacact cactgtgcac tcagccgtcg atacactggt ctgttaggga    660
   gaaagtccgt cagaacaggc agctgtgtgt gtgtgtgcgt gtatgagtgt gtgtgtgtga    720
   tccctgactg ccaggtcctc tgcactgccc ctggg                               755
<210> 35
   <211> 1193
   <212> DNA
   <213> Homo sapiens
<220>
   <221> misc_feature
   <223> sequence of STAR35
<220>
   <221> misc_feature
   <222> (312)..(1191)
   <223> n is a, c, g, or t on various positions
<400> 35
   cgacttggtg atgcgggctc ttttttggtt ccatatgaac tttaaagtag tcttttccaa     60
   ttctgtgaag aaagtcattg gtaggttgat ggggatggca ttgaatctgt aaattacctt    120
   gggcagtatg gccattttca caatgttgat tcttcctatc catgatgatg gaatgttctt    180
   ccattagttt gtatcctctt ttatttcctt gagcagtggt ttgtagttct ccttgaagag    240
   gtccttcaca tcccttgtaa gttggattcc taggtatttt attctctttg aagcaaattg    300
   tgaatgggag tncactcacg atttggctct ctgtttgtct gctgggtgta taaanaatgt    360
   ngtgatnttn gtacattgat ttngtatccn tgagacttng ctgaatttgc ttnatcngct    420
   tnngggaacc ttttgggctg aaacnatggg attttctaaa tatacaatca tgtcgtctgc    480
   aaacagggaa caatttgact tcctcttttc ctaattgaat acactttatc tccttctcct    540
   gcctaattgc cctgggcaaa acttccaaca ctatgntngn aataggagnt ggtgagagag    600
   ggcatccctg ttcttgttgc cagnttttca aagggaatgc ttccagtttt ggcccattca    660
   gtatgatatg ggctgtgggt ngtgtcataa atagctctta tnattttgaa atgtgtccca    720
   tcaataccta atttattgaa agtttttagc atgaangcat ngttgaattt ggtcaaaggc    780
   tttttctgca tctatggaaa taatcatgtg gtttttgtct ttggctcntg tttatatgct    840
   ggatnacatt tattgatttg tgtatatnga acccagcctn ncatcccagg gatgaagccc    900
   acttgatcca agcttggcgc gcngnctagc tcgaggcagg caaaagtatg caaagcatgc    960
   atctcaatta gtcagcaccc atagtccgcc cctacctccg cccatccgcc cctaactcng   1020
   nccgttcgcc cattctcgcc catggctgac taatnttttt annatccaag cggngccgcc   1080
   ctgcttganc attcagagtn nagagnnttg gaggccnagc cttgcaaaac tccggacngn   1140
   ttctnnggat tgaccccnnt taaatatttg gttttttgtn ttttcanngg nga           1193
<210> 36
   <211> 1712
   <212> DNA
   <213> Homo sapiens
<220>
   <221> misc_feature
   <223> sequence of STAR36
<400> 36
   gatcccatcc ttagcctcat cgatacctcc tgctcacctg tcagtgcctc tggagtgtgt      60
   gtctagccca ggcccatccc ctggaactca ggggactcag gactagtggg catgtacact     120
   tggcctcagg ggactcagga ttagtgagcc ccacatgtac acttggcctc agtggactca     180
   ggactagtga gccccacatg tacacttggc ctcaggggac tcaggattag tgagccccca     240
   catgtacact tggcctcagg ggactcagga ttagtgagcc ccacatgtac acttggcctc     300
   aggggactca ggactagtga gccccacatg tacacttggc ctcaggggac tcagaactag     360
   tgagccccac atgtacactt ggcttcaggg gactcaggat tagtgagccc cacatgtaca     420
   cttggacacg tgaaccacat cgatgtgctg cagagctcag ccctctgcag atgaaatgtg     480
   gtcatggcat tccttcacag tggcacccct cgttccctcc ccacctcatc tcccattctt     540
   gtctgtcttc agcacctgcc atgtccagcc ggcagattcc accgcagcat cttctgcagc     600
   acccccgacc acacacctcc ccagcgcctg cttggccctc cagcccagct cccgcctttc     660
   ttccttgggg aagctccctg gacagacacc ccctcctccc agccatggct ttttcctgct     720
   ctgccccacg cgggaccctg ccctggatgt gctacaatag acacatcaga tacagtcctt     780
   cctcagcagc cggcagaccc agggtggact gctcggggcc tgcctgtgag gtcacacagg     840
   tgtcgttaac ttgccatctc agcaactagt gaatatgggc agatgctacc ttccttccgg     900
   ttccctggtg agaggtactg gtggatgtcc tgtgttgccg gccacctttt gtccctggat     960
   gccatttatt tttttccaca aatatttccc aggtctcttc tgtgtgcaag gtattagggc    1020
   tgcagcgggg gccaggccac agatctctgt cctgagaaga cttggattct agtgcaggag    1080
   actgaagtgt atcacaccaa tcagtgtaaa ttgttaactg ccacaaggag aaaggccagg    1140
   aaggagtggg gcatggtggt gttctagtgt tacaagaaga agccagggag ggcttcctgg    1200
   atgaagtggc atctgacctg ggatctggag gaggagaaaa atgtcccaaa agagcagaga    1260
   gcccacccta ggctctgcac caggaggcaa cttgctgggc ttatggaatt cagagggcaa    1320
   gtgataagca gaaagtcctt gggggccaca attaggattt ctgtcttcta aagggcctct    1380
   gccctctgct gtgtgacctt gggcaagtta cttcacctct agtgctttgg ttgcctcatc    1440
   tgtaaagtgg tgaggataat gctatcacac tggttgagaa ttgaagtaat tattgctgca    1500
   aagggcttat aagggtgtct aatactagta ctagtaggta cttcatgtgt cttgacaatt    1560
   ttaatcatta ttattttgtc atcaccgtca ctcttccagg ggactaatgt ccctgctgtt    1620
   ctgtccaaat taaacattgt ttatccctgt gggcatctgg cgaggtggct aggaaagcct    1680
   ggagctgttt cctgttgacg tgccagacta gt                                  1712
<210> 37
   <211> 1321
   <212> DNA
   <213> Homo sapiens
<220>
   <221> misc_feature
   <223> sequence of STAR37
<400> 37
   aggatcacat ttaaggaagt gtgtggggtc cctggatgac accagcaccc agtgcggctc     60
   tgtctggcaa ccgctcccaa ggtggcagga gtgggtgtcc cctgtgtgtc agtgggcagc    120
   tcctgctgag cctacagctc actggggagc ctgacagcgg ggccatgtgc ctgacactcc    180
   tctctgcttg tggacctggc aaggcaggga gcagaaaaca gagccacttg aaggctttct    240
   gtctgcgtct gtgtgcagtg tggatttagt tgtgcttttt tcttgctggg agagcacagc    300
   caccatttac aagcagtgtc accctcatgg gtggcgagga cagaacagga gcctctgctc    360
   tctgtaccta tctgggcccg gtgggctccc ttgtcctggc ttccatctct gtctcagcga    420
   ccattcagcc ctgcgcagga acacatgttg cttagaaaag ccaaattcag cccttgtctc    480
   tgcctcctct ggtctcatga tgtgcatctg ttaccttgaa actggaaacc agtctatcaa    540
   tgtctgtgcc aattttttat tccctcccca acctccttcc ccatacgact ttttatttat    600
   gtaggatgtg tgctgtctaa tgatgggatg accacatttt tccatgttct aaaagtgctc    660
   ctctcccgca gggtcccagg gctggtggtt gctttgggtc tacagctacg tcttacccgc    720
   ctcctgcctc aacagcctgt gtggtggcaa agccggtgtg gggctgggga acgcagcgtt    780
   ctccaggagg gggacccggc tctccttctg cagtgcaggc gaaggcctag atgccagtgt    840
   gacctcccac aaggcgtggc ttccagactc cccggctgga agtgatgctt ttttgcctcc    900
   ggccctgggt ttgaagcagc ctggctttct cttggtaagt ggctggtgtc ttagcagctg    960
   caatctgagc tcagccacct acacaccacc gtggccgaca ctttcattaa aaagtttcct   1020
   gagacgactt gcgtgcatgt tgacttcatg atcagcgccg ctgggaagaa cccctgagcc   1080
   ggtggggtgg ggctggaagc agcaggtgca gtgatggggc tgggtgccca ggaggcctca   1140
   gtgctcaatc aggccaaggt ggccaagccc aggctgcagg gaaggccggc ctgggggttg   1200
   tgggtgagca caggcaggca ccagctgggc agtgttagga tgctggagca gcatccgtaa   1260
   ccccactgag tggggtagtc tggttggggc agggaccgct gttgctttgg cagagagaga   1320
   t                                                                   1321
<210> 38
   <211> 1445
   <212> DNA
   <213> Homo sapiens
<220>
   <221> misc_feature
   <223> sequence of STAR38
<220>
   <221> misc_feature
   <222> (348)..(949)
   <223> n is a, c, g, or t on various positions
<400> 38
   gatctatggg agtagcttcc ttagtgagct ttcccttcaa atactttgca accaggtaga     60
   gaattttgga gtgaaggttt tgttcttcgt ttcttcacaa tatggatatg catcttcttt    120
   tgaaaatgtt aaagtaaatt acctctcttt tcagatactg tcttcatgcg aacttggtat    180
   cctgtttcca tcccagcctt ctataaccca gtaacatctt ttttgaaacc agtgggtgag    240
   aaagacacct ggtcaggaac gcggaccaca ggacaactca ggctcaccca cggcatcaga    300
   ctaaaggcaa acaaggactc tgtataaagt accggtggca tgtgtatnag tggagatgca    360
   gcctgtgctc tgcagacagg gagtcacaca gacacttttc tataatttct taagtgcttt    420
   gaatgttcaa gtagaaagtc taacattaaa tttgattgaa caattgtata ttcatggaat    480
   attttggaac ggaataccaa aaaatggcaa tagtggttct ttctggatgg aagacaaact    540
   tttcttgttt aaaataaatt ttattttata tatttgaggt tgaccacatg accttaagga    600
   tacatataga cagtaaactg gttactacag tgaagcaaat taacatatct accatcgtac    660
   atagttacat ttttttgtgt gacaggaaca gctaaaatct acgtatttaa caaaaatcct    720
   aaagacaata catttttatt aactatagcc ctcatgatgt acattagatc gtgtggttgt    780
   ttcttccgtc cccgccacgc cttcctcctg ggatggggat tcattcccta gcaggtgtcg    840
   gagaactggc gcccttgcag ggtaggtgcc ccggagcctg aggcgggnac tttaanatca    900
   gacgcttggg ggccggctgg gaaaaactgg cggaaaatat tataactgna ctctcaatgc    960
   cagctgttgt agaagctcct gggacaagcc gtggaagtcc cctcaggagg cttccgcgat   1020
   gtcctaggtg gctgctccgc ccgccacggt catttccatt gactcacacg cgccgcctgg   1080
   aggaggaggc tgcgctggac acgccggtgg cgcctttgcc tgggggagcg cagcctggag   1140
   ctctggcggc agcgctggga gcggggcctc ggaggctggg cctggggacc caaggttggg   1200
   cggggcgcag gaggtgggct cagggttctc cagagaatcc ccatgagctg acccgcaggg   1260
   cggccgggcc agtaggcacc gggcccccgc ggtgacctgc ggacccgaag ctggagcagc   1320
   cactgcaaat gctgcgctga ccccaaatgc tgtgtccttt aaatgtttta attaagaata   1380
   attaataggt ccgggtgtgg aggctcaagc cttaatcccc agcacctggc gaggccgagg   1440
   aggga                                                               1445
<210> 39
   <211> 2331
   <212> DNA
   <213> Homo sapiens
<220>
   <221> misc_feature
   <223> sequence of STAR39
<400> 39
   gtgaaataga tcactaaagc tgattcctct tgtctaaatg aaactttcta ccctttgatg      60
   gacagctatg ctttccccat cctctcccgt cccccagccc ttggtaacca tcatcctact     120
   ctctacttgt aggagttcaa cttgtttaga ttttgtgagt gagaacatgt ggtatttgcc     180
   tttagagtcc tctaggttta tccatattgt gttaaatgac aggattccct gcctttttaa     240
   ggctgaatag tatttcattg taatatatat acatacacac acacatatac acacacatat     300
   atatacatat atacatatat gtacatagat acatatatat gtacatatat acacacacat     360
   atacacacat atatacacat atatacatat acatatatac acatatatgt acatatatat     420
   aacttttttt catttatcca ttcacttaat acatatgatg gagggcttta tatatgccag     480
   gctctgtgat gaatgctgga aattcaatag tgagaaagac tcagtctctg cctccaaaga     540
   gcatcatggg ctaggtgctg caacgaggaa ttgccaactg ttgtcatgag agcacagaga     600
   agggactcaa ccagccttga agaatcaggg gaggcttcta agctaatggt gtgtgcctgg     660
   ggatcacatt gtttcaagca gcagtaacag gatgtgctca ggtccagatg tgagagagag     720
   agagagcata tgtcttcaag aaactaacag tagctcccta tagctgaagc aggagtacaa     780
   aatagtgagt ttaagtgatg aggcaagaga tatgaagaag cttgaccatg cagctacacc     840
   gggcagcatg ccctctgaga catctcatgg aagccggaaa tgggagtgcc ttgataccaa     900
   gccagagaaa ttataatact aagtagatag actgagcagc actcctcctg ggaagaatga     960
   gacaagccct gaatttggag gtaagttgtg gattggtgat tagaggagag gtaacaggca    1020
   ccaaagcaag aaatagtatt gatgcaaagc tgaggttaat tggatgacaa aatgaagagc    1080
   ataaggggct cagacacaga ctgagcagaa aacgagtagc atctgaacct agattgagtt    1140
   actaatggat gagaaagagt tcttaaagtt gatgaccacg ggatccatat ataagaatgt    1200
   ccaatctccc caaattgatc cacgagttca gtgcaatgcc aatcaaaatc ccactaacaa    1260
   gtttatttta aaatgtaaat gaaaatacaa aatttttaaa aagcaaagca atattgaaaa    1320
   cccaggaaaa attaggagga cttacacaac ctgatctcaa aacttaccat tatcaagaca    1380
   gagtgttatt gacacaagga gagacaaata gataaacgga atgtggtagt ctggagatgc    1440
   acccacatgt atgtggtcaa ttgatttttg gccaaggcac caagtcaatt caaaggagca    1500
   aggaaagtag tacagaaaca accaaatatt gttttggaaa ataatgacaa agggcttata    1560
   accagaatat aagcatataa atataattct ttcaaatcaa taataagaag gcaaatatct    1620
   aataaaaatg agcaaagact tgaaaagtca cttaaaaagg cttattaatt agaaatatgc    1680
   aaatgttatt agtcttcagt ggaatttaca ttaaaccaca agggatacta ttatatctta    1740
   tgcccactag aataaccaaa ggaaaaaaga cagacaaaac aaaatgctgg tgaggatgtg   1800
   aagcaactgg aactctcata cattattggt ggtaatgtaa aatttataca accattatga   1860
   ataaaggttt ggcagtttct tacaaagttg aatgcacttc tccacgatga ctaggctttt   1920
   cactcatagg cgtctggctc cctagaactg aaaacatatg ttcacaagaa gacttgcaaa   1980
   tatatattct cccacgtcag gagatatttg ctatgcattt aactgacata agattagtgc   2040
   tagagtttat aatgaggttc ttcaaatcta aaagaaaatg caaagcatat aatagtaagg   2100
   ggtgcaggcc aggcgcagtg gctcactctg taatcccagc actttgggag gccgaggtgg   2160
   gcggatcaca aggtcaggag ttcgagacca acctggccaa catagtgaaa ccctgtctct   2220
   actaaaaata caaaaactag ccaggtgcgg tgtcatgcac ctgtagtccc agctactcgg   2280
   gaggccgagg caggagaatc acttgaacct gggaggtgga ggttgcagtg a            2331
<210> 40
   <211> 1071
   <212> DNA
   <213> Homo sapiens
<220>
   <221> misc_feature
   <223> sequence of STAR40
<400> 40
   gctgtgattc aaactgtcag cgagataagg cagcagatca agaaagcact ccgggctcca      60
   gaaggagcct tccaggccag ctttgagcat aagctgctga tgagcagtga gtgtcttgag     120
   tagtgttcag ggcagcatgt taccattcat gcttgacttc tagccagtgt gacgagaggc     180
   tggagtcagg tctctagaga gttgagcagc tccagcctta gatctcccag tcttatgcgg     240
   tgtgcccatt cgctttgtgt ctgcagtccc ctggccacac ccagtaacag ttctgggatc     300
   tatgggagta gcttccttag tgagctttcc cttcaaatac tttgcaacca ggtagagaat     360
   tttggagtga aggttttgtt cttcgtttct tcacaatatg gatatgcatc ttcttttgaa     420
   aatgttaaag taaattacct ctcttttcag atactgtctt catgcgaact tggtatcctg     480
   tttccatccc agccttctat aacccagtaa catctttttt gaaaccagtg ggtgagaaag     540
   acacctggtc aggaacgcgg accacaggac aactcaggct cacccacggc atcagactaa     600
   aggcaaacaa ggactctgta taaagtaccg gtggcatgtg tattagtgga gatgcagcct     660
   gtgctctgca gacagggagt cacacagaca cttttctata atttcttaag tgctttgaat     720
   gttcaagtag aaagtctaac attaaatttg attgaacaat tgtatattca tggaatattt     780
   tggaacggaa taccaaaaaa tggcaatagt ggttctttct ggatggaaga caaacttttc     840
   ttgtttaaaa taaattttat tttatatatt tgaggttgac cacatgacct taaggataca     900
   tatagacagt aaactggtta ctacagtgaa gcaaattaac atatctacca tcgtacatag     960
   ttacattttt ttgtgtgaca ggaacagcta aaatctacgt atttaacaaa aatcctaaag    1020
   acaatacatt tttattaact atagccctca tgatgtacat tagatctcta a             1071
<210> 41
   <211> 1135
   <212> DNA
   <213> Homo sapiens
<220>
   <221> misc_feature
   <223> sequence of STAR41
<400> 41
   cgtgtgcagt ccacggagag tgtgttctcc tcatcctcgt tccggtggtt gtggcgggaa      60
   acgtggcgct gcaggacacc aacatcagtc acgtatttca ttctggaaaa aaaagtagca     120
   caagcctcgg ctggttccct ccagctctta ccaggcagcc taagcctagg ctccattccc     180
   gctcaaggcc ttcctcaggg gcctgctcac cacaggagct gttcccatgc agggactaag     240
   gacatgcagc ctgcatagaa accaagcacc caggaaaaca tgattggatg gagcgggggg     300
   gtgtggtctc tagccttgtc cacctccggt cctcatgggt ctcacacctc ctgagaatgg     360
   gcaccgcaga ggccacagcc catacagcca agatgacaga ctccgtaagt gacagggatc     420
   cacagcagag tgggtgaaat gttccctata aactttacaa aattaatgag ggcaggggga      480
   ggggagaaat gaaaatgaac ccagctcgca gcacatcagc atcagtcact aggtcggcgt     540
   gctctctgac tgcttcctcg tagctgcttg gtgtctcatt gcctcagaag catgtagacc     600
   ctgtcacaag attgtagttc ccctaactgc tccgtagatc acaacttgaa ccttaggaaa     660
   tgctgttttc cctttgagat attcctttgg gtcctgtata ctgatggagc tactgactga     720
   gctgctccga aggaccccac gaggagctga ctaaaccaag agtgcagttt gtacaccctg     780
   atgattacat cccccttgcc ccaccaatca actctcccaa ttttccagcc cctcaccctc      840
   cagtcccctt aaaagcccca gcccaggccg ggcacagtgg ctcatgcctg taatcccagc     900
   actttgggag gccaaggtgg gcagatcacc tgagggcagg aatttgagac cagcctgacc     960
   aacatgaaga aaccccgtct ctattacaaa tacaaaatta gccgggcgtg ttgctgcata    1020
   ctggtaatcc cagctacttg ggagggtgag gcaggagaat cacttgaatc tgggaggcgg    1080
   aggttgcgat gagccgagac agcgccattg cactgcagcc tgggcaacaa gagca         1135
<210> 42
   <211> 735
   <212> DNA
   <213> Homo sapiens
<220>
   <221> misc_feature
   <223> sequence of STAR42
<400> 42
   aagggtgaga tcactaggga gggaggaagg agctataaaa gaaagaggtc actcatcaca      60
   tcttacacac tttttaaaac cttggttttt taatgtccgt gttcctcatt agcagtaagc     120
   cctgtggaag caggagtctt tctcattgac caccatgaca agaccctatt tatgaaacat     180
   aatagacaca caaatgttta tcggatattt attgaaatat aggaattttt cccctcacac     240
   ctcatgacca cattctggta cattgtatga atgaatatac cataatttta cctatggctg     300
   tatatttagg tcttttcgtg caggctataa aaatatgtat gggccggtca cagtgactta     360
   cgcccgtagt cccagaactt tgggaggccg aggcgggtgg atcacctgag gtcgggagtt     420
   caaaaccagc ctgaccaaca tggagaaacc ccgtctctgc taaaaataca aaaattaact     480
   ggacacggtg gcgtatgcct gtaatcccag ctactcggga agctgaggca ggagaactgc     540
   ttgaacccag gaggcggagg ttgtggtgag tcgagattgc gccattgcac tccagcctgg     600
   gcaacaagag cgaaattcca tctcaaaaaa aagaaaaaag tatgactgta tttagagtag     660
   tatgtggatt tgaaaaatta ataagtgttg ccaacttacc ttagggttta taccatttat     720
   gagggtgtcg gtttc                                                      735
<210> 43
   <211> 1227
   <212> DNA
   <213> Homo sapiens
<220>
   <221> misc_feature
   <223> sequence of STAR43
<400> 43
   caaatagatc tacacaaaac aagataatgt ctgcccattt ttccaaagat aatgtggtga      60
   agtgggtaga gagaaatgca tccattctcc ccacccaacc tctgctaaat tgtccatgtc     120
   acagtactga gaccaggggg cttattccca gcgggcagaa tgtgcaccaa gcacctcttg     180
   tctcaatttg cagtctaggc cctgctattt gatggtgtga aggcttgcac ctggcatgga     240
   aggtccgttt tgtacttctt gctttagcag ttcaaagagc agggagagct gcgagggcct     300
   ctgcagcttc agatggatgt ggtcagcttg ttggaggcgc cttctgtggt ccattatctc     360
   cagcccccct gcggtgttgc tgtttgcttg gcttgtctgg ctctccatgc cttgttggct     420
   ccaaaatgtc atcatgctgc accccaggaa gaatgtgcag gcccatctct tttatgtgct     480
   ttgggctatt ttgattcccc gttgggtata ttccctaggt aagacccaga agacacagga     540
   ggtagttgct ttgggagagt ttggacctat gggtatgagg taatagacac agtatcttct     600
   ctttcatttg gtgagactgt tagctctggc cgcggactga attccacaca gctcacttgg     660
   gaaaacttta ttccaaaaca tagtcacatt gaacattgtg gagaatgagg gacagagaag     720
   aggccctaga tttgtacatc tgggtgttat gtctataaat agaatgcttt ggtggtcaac     780
   tagacttgtt catgttgaca tttagtcttg ccttttcggt ggtgatttaa aaattatgta     840
   tatcttgttt ggaatatagt ggagctatgg tgtggcattt tcatctggct ttttgtttag     900
   ctcagcccgt cctgttatgg gcagccttga agctcagtag ctaatgaaga ggtatcctca     960
   ctccctccag agagcggtcc cctcacggct cattgagagt ttgtcagcac cttgaaatga    1020
   gtttaaactt gtttattttt aaaacattct tggttatgaa tgtgcctata ttgaattact    1080
   gaacaacctt atggttgtga agaattgatt tggtgctaag gtgtataaat ttcaggacca    1140
   gtgtctctga agagttcatt tagcatgaag tcagcctgtg gcaggttggg tggagccagg    1200
   gaacaatgga gaagctttca tgggtgg                                        1227
<210> 44
   <211> 1586
   <212> DNA
   <213> Homo sapiens
<220>
   <221> misc_feature
   <223> sequence of STAR44
<400> 44
   cacctgcctc agcctcccaa agtgctgaga ttcaaagaaa ttttcatgga gaggggacag     60
   atggagtcaa ttcttgtggg gtgaacatga gtaccacagt tagactgagg ttgggaaaga    120
   ttttccagac aattggaaga gcatgtgaaa gacacagatt ttgagaaatg ttaagtctag    180
   ggaactgcaa ggcttttggc acaagaaagc cactgtagac tatagaggca ggatgcctag    240
   attcaaatcc caactgctac acttctaagc tttgtaattt tggcaagttt ttaccctcta    300
   ttttcttatc tataaaatat agattttata tatatagata tagatatata gatagataat    360
   aattgtgcat gcctaataaa gttgtcaaag attaaatgtt atatgtgaag tattttgtac    420
   ggtgatagga acccaggaag ggctctatga atattatgta ttattattat tctaaagtag    480
   ctggaataca atgttcaaag gagatagtgg caggagataa gtttgaattg aaagattgag    540
   gccagaacat aaagtgcctc ctatattata ttttacataa ttggaacatc attgaaaaat    600
   ttaagtatta tttatgtgtg tatgtgtgtt ttatataatt aattctagtt catcatttta    660
   aaatatcttt ctgatgtcac tgtgaacaac agatgagaag aagtgaatcc tgagttaagg    720
   agaccagctc tctgattact gccataatcc agggagggta ccataaggat ttcaactgga    780
   agtgaatcca tcatgatgga gaggaaggac agggctgaaa aatacttagg aagtagtatc    840
   agtaggactg gttaagagag agcagaggca ggctacaggg gttggaggtg tcaatcacag    900
   agatagggaa aatgggagga gaagcaggct ttgaaaaagt ggcttgtctt gtaaaattat    960
   gtgctgttaa aacagtacaa gaaattaata tattcaatcc caaaatacag ggacaattct   1020
   ttttgaaaga gttacccaga tagtcttcct tgaagttttc agttaaagaa atttcttgtt   1080
   aacaaataat gtagtcatag aagaaaacac ttaaaacttt attgaataaa gctaataaat   1140
   catttaatat aatttatagg aaattgttac ataacacaca cattcaatac tttttgctaa   1200
   agtataaatt aatggaagga gagcacgcac acagaggttg aattatgttt atgactttat   1260
   tagtcaagaa tacaaaattg agtagctaca tcaagcagaa gcacatgctt tacaatccag   1320
   cacagaatcc cttgacatcc aaactcccga aacagacatg taaatacaga tgacattgtc   1380
   agaacaaaat agggtctcac ccgacctata atgttctttt cttgatataa atatgcacat   1440
   gaattgcata cggtcatatg gttccaatta ccattatttc ctctgggctt agctatccat   1500
   ctaaggggaa tttacaccaa cactgtactt ctacttgcaa gaatatatga aagcatagtt   1560
   aacttctggc ttaggacccc aactca                                        1586
<210> 45
   <211> 1981
   <212> DNA
   <213> Homo sapiens
<220>
   <221> misc_feature
   <223> sequence of STAR45
<400> 45
   atggatcata gggtaaataa atttataatt tcttgagaaa gcttcgtact gttttccaag      60
   atggctgtac taatttccat tcctaccaac agtgtacagg gtttcttttt ctccacatcc     120
   tcaccaacac ttatcttcca tcttttttta taatagccct agtaaaatgt gtgaggtgat     180
   atctcattgt ggcattgatt tgcacttctc tgataattag gaatgtttat gattttttca     240
   tgtacctggt tggccttttg tatgatgtag gaaatgtcta ttctgattct ttgcttattt     300
   tttaataagc atagtttttt tcttattttt gagtaggttg agttgcttat atattattat     360
   atgagcccct tacctgatgt atggtttaaa aatattatcc catttgtggg ttctcttaat     420
   tctatcattg cttcttttcc tgtggaaaag ttttaagttt tatgcagtct catttgtgtg     480
   ttttgctttt gttgcctttt ggaataatct acagaaaatc atagctcagg ccaatgtcat     540
   acagtctcct tctatatttc cttgtagtag ttttacattt aaactttaat tttgatttga     600
   tgcttgtata aagagcaaaa taaaagtcaa attttattct tctgtatgtg gatagtcagt     660
   tttgtctaca ccatttattg aaaataattt tctttcttca ctgtgtattt ttagttattt     720
   tatcaaaaaa tcaattgacc acagacacac ggatttattt acaggttcta tatccctttg     780
   tactgtttta catgtctgtt tttatgccat tgctatgctg ttttaattcc tatagctttg     840
   taatagagtt tggagtcagg tagtctgatg cctccagctt tgttcttttt gttcaagatt     900
   gctttggttg gtccaggtct tttgtggttc catacaaatt ttagcagtaa tttttctatt     960
   tctgtgaaga atgacattgg aatttgatag tggttgcatt taatctgtag attgctttgg    1020
   gtagcattga cacttttaca atactaattt ttgaatccat caatgaagga tgtttctcca   1080
   tttatttatg ccattttaat ttttttcatc aatgtgctat agttttcagt atgtaaatct   1140
   tttatggttt tgattaaatt tactcctgtc ttttatatat ttatatatct gttttgattc   1200
   tattataaat tgaattgcct ttatttttca ggtaatagtt tgtcattagt taatagaaac   1260
   aataatgata tttgtatgtt gattttgtaa ctattaactt tattgaattt cttcatcagc   1320
   tataaccatt tattttggtg gaatctttaa gattttctct atcttaagat tatattttca   1380
   aaaaacagaa acaatcttac ctcttccttc cctatgtgga tttcttttac gtctttgtct   1440
   tgtgtaactg ttctggctag gcaattacac ataatgtttt catcatttat aattttacat   1500
   cacatccatc tattgtggca cattgattgc tacttttcaa gttgtaaacc tggacattta   1560
   tcactactct tcctccaata caggagtcca tggcgtggtg tgggccctac tgtgccacag   1620
   tccagggcac ggctgggctg aggttctctt gtgcaagagt ccgtggctct gcggagcaag   1680
   agttctccag tgccttagtc cagggttagg caggggtggg gctccttcag tagcttagtc   1740
   cagtgcgccg ccctgcgagg gtcctcctga gcaggagtac acgatgaggc agggtcctac   1800
   tgtgccttag cccaggaagc ggggggctgg gtcctctggt gccatagtcc aggctgccgg   1860
   gagctgggtc ctctggtgcc atagctcagg ccggcgggag ctgggtcctc tggtgccgta   1920
   gtccagggtg cagcagaaca ggagtcctgc ggagcagtag tccagggcac gctggggcgt   1980
   g                                                                   1981
<210> 46
   <211> 1859
   <212> DNA
   <213> Homo sapiens
<220>
   <221> misc_feature
   <223> sequence of STAR46
<400> 46
   attgtttttc tcgcccttct gcattttctg caaattctgt tgaatcattg cagttactta     60
   ggtttgcttc gtctccccca ttacaaacta cttactgggt ttttcaaccc tagttccctc    120
   atttttatga tttatgctca tttctttgta cacttcgtct tgctccatct cccaactcat    180
   ggcccctggc tttggattat tgttttggtc ttttattttt tgtcttcttc tacctcaaca    240
   cttatcttcc tctcccagtc tccggtaccc tatcaccaag gttgtcatta acctttcata    300
   ttattcctca ttatccatgt attcatttgc aaataagcgt atattaacaa aatcacaggt    360
   ttatggagat ataattcaca taccttaaaa ttcaggcttt taaagtgtac ctttcatgtg    420
   gtttttggta tattcacaaa gttatgcatt gatcaccacc atctgattcc ataacatgtt    480
   caatacctca aaaagaagtc tgtactcatt agtagtcatt tcacattcac cactccctct    540
   ggctctgggc agtcactgat ctttgtgtct ctatggattt gcctagtcta ggtattttta    600
   tgtaaatggc atcatacaac atgtgacctt ttgtttggct tttttcattt agcaaaatgt    660
   tatcaaggtc tgtccctgtt gtagcatgta ttagcacttc atttcttata tgctgaatga    720
   tatactttat ttgtccatca gttgttcatg ctttatttgt ccatcagttg atgaacattt    780
   gcgtttttgc cactttgggc tattaagaat aatgctactg tgaacaagtg tgtacaagtt    840
   cctctacaaa tttttgtgtg gacatatcct ttcagttctc tcaggtgtat atctgggaat    900
   tgaattgctg ggtcgtgtag tagctatgtt aaacactttg agaaactgct ataatgttct    960
   ccagagctgt accattttaa attctgtgta tgaggattcc acgttctcca cttcctcacc   1020
   agtgtatgga tttgggggta tactttttaa aaagtgggat taggctgggc acagtggctc   1080
   acacctgtaa tcccaacact tcaggaagct gaggtgggag gatcacttga gcctagtagt   1140
   ttgagaccag cctgggcaac atagggagac cctgtctcta caaaaaataa tttaaaataa   1200
   attagctggg cgttgtggca cacacctgta gtcccagcta catgggaggc tgaggtggaa   1260
   ggattccctg agcccagaag tttgaggttg cagtgagcca tgatggcagc actatactgt   1320
   agcctgggtg tcagagcaag actccgtttc agggaagaaa aaaaaaagtg ggatgatatt   1380
   tttgacactt ttcttcttgt tttcttaatt tcatacttct ggaaattcca ttaaattagc   1440
   tggtaccact ctaactcatt gtgtttcatg gctgcatagt aatattgcat aatataaata   1500
   taccattcat tcatcaaagt tagcagatat tgactgttag gtgccaggca ctgctctaag   1560
   cgttaaagaa aaacacacaa aaacttttgc attcttagag tttattttcc aatggagggg   1620
   gtggagggag gtaagaattt aggaaataaa ttaattacat atatagcata gggtttcacc   1680
   agtgagtgca gcttgaatcg ttggcagctt tcttagtagt ataaatacag tactaaagat   1740
   gaaattactc taaatggtgt tacttaaatt actggaatag gtattactat tagtcacttt   1800
   gcaggtgaaa gtggaaacac catcgtaaaa tgtaaaatag gaaacagctg gttaatgtt    1859
<210> 47
   <211> 1082
   <212> DNA
   <213> Homo sapiens
<220>
   <221> misc_feature
   <223> sequence of STAR47
<400> 47
   atcattagtc attagggaaa tgcaaatgaa aaacacaagc agccaccaat atacacctac      60
   taggatgatt taaaggaaaa taagtgtgaa gaaggacgta aagaaattgt aaccctgata     120
   cattgatggt agaaatggat aaagttgcag ccactgtgaa aaacagtctg cagtggctca     180
   gaaggttaaa tatagaaccc ctgttggacc caggaactct actcttaggc accccaaaga     240
   atagagaaca gaaatcaaac agatgtttgt atactaatgt ttgtagcatc acttttcaca     300
   ggagccaaaa ggtggaaata atccaaccat cagtgaacaa atgaatgtaa taaaagcaag     360
   gtggtctgca tgcaatgcta catcatccat ctgtaaaaaa cgaacatcat tttgatagat     420
   gatacaacat gggtggacat tgagaacatt atgcttagtg aaataagcca gacacaaaag     480
   gaatatattg tataattgta attacatgaa gtgcctagaa tagtcaaatt catacaagag     540
   aaagtgggat aggaatcacc atgggctgga aataggggga aggtgctata ctgcttattg     600
   tggacaaggt ttcgtaagaa atcatcaaaa ttgtgggtgt agatagtggt gttggttatg     660
   caaccctgtg aatatattga atgccatgga gtgcacactt tggttaaaag gttcaaatga     720
   taaatattgt gttatatata tttccccacg atagaaaaca cgcacagcca agcccacatg     780
   ccagtcttgt tagctgcctt cctttacctt caagagtggg ctgaagcttg tccaatcttt     840
   caaggttgct gaagactgta tgatggaagt catctgcatt gggaaagaaa ttaatggaga     900
   gaggagaaaa cttgagaatc cacactactc accctgcagg gccaagaact ctgtctccca     960
   tgctttgctg tcctgtctca gtatttcctg tgaccacctc ctttttcaac tgaagacttt    1020
   gtacctgaag gggttcccag gtttttcacc tcggcccttg tcaggactga tcctctcaac    1080
   ta                                                                   1082
<210> 48
   <211> 1242
   <212> DNA
   <213> Homo sapiens
<220>
   <221> misc_feature
   <223> sequence of STAR48
<400> 48
   atcatgtatt tgttttctga attaattctt agatacatta atgttttatg ttaccatgaa     60
   tgtgatatta taatataata tttttaattg gttgctactg tttataagaa tttcattttc    120
   tgtttacttt gccttcatat ctgaaaacct tgctgatttg attagtgcat ccacaaattt    180
   tcttggattt tctatgggta attacaaatc tccacacaat gaggttgcag tgagccaaga    240
   tcacaccact gtactccagc ctgggcgaca gagtgagaca ccatctcaca aaaacacata    300
   aacaaacaaa cagaaactcc acacaatgac aacgtatgtg ctttcttttt ttcttcctct    360
   ttctataata tttctttgtc ctatcttaac tgaactggcc agaaacccca ggacaatgat    420
   aaatacgagc agtgtcaaca gacatctcat tccctttcct agcttttata aaaataacga    480
   ttatgcttca acattacata tggtggtgtc gatggttttg ttatagataa gcttatcagg    540
   ttaagaaatt tgtctgcgtt tcctagtttg gtataaagat tttaatataa atgaatgttg    600
   tattttatca tcttattttt ttcctacatc tgctaaggta atcctgtgtt ttcccctttt    660
   caatctccta atgtggtgaa tgacattaaa ataccttcta ttgttaaaat attcttgcaa    720
   cgctgtatag aaccaatgcc tttattctgt attgctgatg gatttttgaa aaatatgtag    780
   gtggacttag ttttctaagg ggaatagaat ttctaatata tttaaaatat tttgcatgta    840
   tgttctgaag gacattggtg tgtcatttct ataccatctg gctactagag gagccgactg    900
   aaagtcacac tgccggagga ggggagaggt gctcttccgt ttctggtgtc tgtagccatc    960
   tccagtggta gctgcagtga taataatgct gcagtgccga cagttctgga aggagcaaca   1020
   acagtgattt cagcagcagc agtattgcgg gatccccacg atggagcaag ggaaataatt   1080
   ctggaagcaa tgacaatatc agctgtggct atagcagctg agatgtgagt tctcacggtg   1140
   gcagcttcaa ggacagtagt gatggtccaa tggcgcccag acctagaaat gcacatttcc   1200
   tcagcaccgg ctccagatgc tgagcttgga cagctgacgc ct                      1242
<210> 49
   <211> 1015
   <212> DNA
   <213> Homo sapiens
<220>
   <221> misc_feature
   <223> sequence of STAR49
<400> 49
   aaaccagaaa cccaaaacaa tgggagtgac atgctaaaac cagaaaccca aaacaatggg 60
   agggtcctgc taaaccagaa acccaaaaca atgggagtga agtgctaaaa ccagaaaccc 120
   aaaacaatgg gagtgtcctg ctacaccaga aacccaaaac gatgggagtg acgtgataaa 180
   accagacacc caaaacaatg ggagtgacgt gctaaaccag aaacccaaaa caatgggagt 240
   gacgtgctaa aacctggaaa cctaaaacaa tgcgagtgag gtgctaacac cagaatccat 300
   aacaatgtga gtgacgtgct aaaccagaac ccaaaacaat gggagtgacg tgctaaaaca 360
   ggaacccaaa acaatgagag tgacgtgcta aaccagaaac ccaaaacaat gggaatgacg 420
   tgctaaaacc ggaacccaaa acaatgggag tgatgtgcta aaccagaaac ccaaaacaat 480
   gggaatgaca tgctaaaact ggaacccaaa acaatggtaa ctaagagtga tgctaaggcc 540
   ctacattttg gtcacactct caactaagtg agaacttgac tgaaaaggag gatttttttt 600
   tctaagacag agttttggtc tgtcccccag agtggagtgc agtggcatga tctcggctca 660
   ctgcaagctc tgcctcccgg gttcaggcca ttctcctgcc tcagcctcct gagtagctgg 720
   gaatacaggc acccgccacc acacttggct aattttttgt atttttagta gagatggggt 780
   ttcaccatat tagcaaggat ggtctcaatc tcctgacctc gtgatctgcc cacctcaggc 840
   tcccaaagtg ctgggattac aggtgtgagc caccacaccc agcaaaaagg aggaattttt 900
   aaagcaaaat tatgggaggc cattgttttg aactaagctc atgcaatagg tcccaacaga 960
   ccaaaccaaa ccaaaccaaa atggagtcac tcatgctaaa tgtagcataa tcaaa 1015
<210> 50
   <211> 2355
   <212> DNA
   <213> Homo sapiens
<220>
   <221> misc_feature
   <223> sequence of STAR50
<400> 50
   caaccatcgt tccgcaagag cggcttgttt attaaacatg aaatgaggga aaagcctagt    60
   agctccattg gattgggaag aatggcaaag agagacaggc gtcattttct agaaagcaat   120
   cttcacacct gttggtcctc acccattgaa tgtcctcacc caatctccaa cacagaaatg   180
   agtgactgtg tgtgcacatg cgtgtgcatg tgtgaaagta tgagtgtgaa tgtgtctata   240
   tgggaacata tatgtgattg tatgtgtgta actatgtgtg actggcagcg tggggagtgc   300
   tggttggagt gtggtgtgat gtgagtatgc atgagtggct gtgtgtatga ctgtggcggg   360
   aggcggaagg ggagaagcag caggctcagg tgtcgccaga gaggctggga ggaaactata   420
   aacctgggca atttcctcct catcagcgag cctttcttgg gcaatagggg cagagctcaa   480
   agttcacaga gatagtgcct gggaggcatg aggcaaggcg gaagtactgc gaggaggggc   540
   agagggtctg acacttgagg ggttctaatg ggaaaggaaa gacccacact gaattccact   600
   tagccccaga ccctgggccc agcggtgccg gcttccaacc ataccaacca tttccaagtg   660
   ttgccggcag aagttaacct ctcttagcct cagtttcccc acctgtaaaa tggcagaagt   720
   aaccaagctt accttcccgg cagtgtgtga ggatgaaaag agctatgtac gtgatgcact   780
   tagaagaagg tctagggtgt gagtggtact cgtctggtgg gtgtggagaa gacattctag   840
   gcaatgagga ctggggagag cctggcccat ggcttccact cagcaaggtc agtctcttgt   900
   cctctgcact cccagccttc cagagaggac cttcccaacc agcactcccc acgctgccag   960
   tcacacatag ttacacacat acaatcacat atatgttccc atatagacac attcacactc  1020
   ataccttcac acatgcacac gcatgtgcac acacagtcac tcatttctgt gttggagatt  1080
   gggtgaggac attcaatggg tgaggaccaa caggtgtgaa gattgctttc tagaaaatga  1140
   ctcctgtctc tctttgccat tcttcccaat ccgatggagc tactaggctt ttccctcatt  1200
   tcatgtttaa taaaccttcc caatggcgaa atgggctttc tcaagaagtg gtgagtgtcc  1260
   catccctgcg gtggggacag gggtggcagc ggacaagcct gcctggaggg aactgtcagg  1320
   ctgattccca gtccaactcc agcttccaac acctcatcct ccaggcagtc ttcattcttg  1380
   gctctaattt cgctcttgtt ttctttttta tttttatcga gaactgggtg gagagctttt  1440
   ggtgtcattg gggattgctt tgaaaccctt ctctgcctca cactgggagc tggcttgagt  1500
   caactggtct ccatggaatt tcttttttta gtgtgtaaac agctaagttt taggcagctg   1560
   ttgtgccgtc cagggtggaa agcagcctgt tgatgtggaa ctgcttggct cagatttctt   1620
   gggcaaacag atgccgtgtc tctcaactca ccaattaaga agcccagaaa atgtggcttg   1680
   gagaccacat gtctggttat gtctagtaat tcagatggct tcacctggga agccctttct   1740
   gaatgtcaaa gccatgagat aaaggacata tatatagtag ctagggtggt ccacttctta   1800
   ggggccatct ccggaggtgg tgagcactaa gtgccaggaa gagaggaaac tctgttttgg   1860
   agccaaagca taaaaaaacc ttagccacaa accactgaac atttgttttg tgcaggttct   1920
   gagtccaggg agggcttctg aggagagggg cagctggagc tggtaggagt tatgtgagat   1980
   ggagcaaggg ccctttaaga ggtgggagca gcatgagcaa aggcagagag gtggtaatgt   2040
   ataaggtatg tcatgggaaa gagtttggct ggaacagagt ttacagaata gaaaaattca   2100
   acactattaa ttgagcctct actacgtgct cgacattgtt ctagtcactg agataggttt   2160
   ggtatacaaa acaaaatcca tcctctatgg acattttagt gactaacaac aatataaata   2220
   ataaaagtga acaaaagctc aaaacatgcc aggcactatt atttatttat ttatttattt   2280
   atttatttat tttttgaaac agagtctcgc tctgttgccc aggctggagt gtagtggtgc   2340
   gatctcggct cactg                                                    2355
<210> 51
   <211> 2289
   <212> DNA
   <213> Homo sapiens
<220>
   <221> misc_feature
   <223> sequence of STAR51
<400> 51
   tcacaggtga caccaatccc ctgaccacgc tttgagaagc actgtactag attgactttc     60
   taatgtcagt cttcattttc tagctctgtt acagccatgg tctccatatt atctagtaca    120
   acacacatac aaatatgtgt gatacagtat gaatataata taaaaatatg tgttataata    180
   taaatataat attaaaatat gtctttatac tagataataa tacttaataa cgttgagtgt    240
   ttaactgctc taagcacttt acctgcagga aacagttttt tttttatttt ggtgaaatac    300
   aactaacata aatttattta caattttaag catttttaag tgtatagttt agtggagtta    360
   atatattcaa aatgttgtgc agccgtcacc atcatcagtc ttcataactc ttttcatatt    420
   gtaaaattaa aagtttatgc tcatttaaaa atgactccca atttcccccc tcctcaacct    480
   ctggaaacta ccattctatt ttctgcctcc gtagttttgc ccactctaag tacctcacat    540
   aagtggaatt tgtcttattt gcctgtttgt gaccggctga tttcatttag tataatgtcc    600
   tcaagtttta ttcacgttat atagcatatg tcataatttt cttcactttt aagcttgagt    660
   aatatttcat cgtatgtatc tcacattttg cttatccatt catctctcag tggacacttg 720
   agttgcttct acattttagc tgttgtgaat actgctgcta tgaacatggg tgtataaata 780
   tctcaagacc tttttatcag ttttttaaaa tatatactca gtagtagttt agctggatta 840
   tatggtaatt ttatttttaa tttttgagga actgtcctac ccttttattc aatagtagct 900
   ataccaattg acaattggca ttcctaccaa cagggcataa gggttctcaa ttctccacat 960
   attccctgat acttgttatt ttcaggtgtt tttttttttt tttttttttt atgggagcca 1020
   tgttaatggg tgtaaggtga tatttcatta tagttttgat ttgcatttcc ctaatgatta 1080
   gtgatgttaa gcatctcttc atgtgcctat tggccatttg tatatcttct ttaaaaatat 1140
   atatatactc attcctttgc ccatttttga attatgttta ttttttgtta ttgagtttca 1200
   atacttttct atataaccta ggtattaatc ctttatcaga cttaagattt gcaaatattc 1260
   tctttcattc cacaggttgc taattctctc tgttggtaat atcttttgat gctgttgtgt 1320
   ccagaattga ttcattcctg tgggttcttg gtctcactga cttcaagaat aaagctgcgg 1380
   accctagtgg tgagtgttac acttcttata gatggtgttt ccggagtttg ttccttcaga 1440
   tgtgtccaga gtttcttcct tccaatgggt tcatggtctt gctgacttca ggaatgaagc 1500
   cgcagacctt cgcagtgagg tttacagctc ttaaaggtgg cgtgtccaga gttgtttgtt 1560
   ccccctggtg ggttcgtggt cttgctgact tcaggaatga agccgcagac cctcgcagtg 1620
   agtgttacag ctcataaagg tagtgcggac acagagtgag ctgcagcaag atttactgtg 1680
   aagagcaaaa gaacaaagct tccacagcat agaaggacac cccagcgggt tcctgctgct 1740
   ggctcaggtg gccagttatt attcccttat ttgccctgcc cacatcctgc tgattggtcc 1800
   attttacaga gtactgattg gtccatttta cagagtgctg attggtgcat ttacaatcct 1860
   ttagctagac acagagtgct gattgctgca ttcttacaga gtgctgattg gtgcatttac 1920
   agtcctttag ctagatacag aacgctgatt gctgcgtttt ttacagagtg ctgattggtg 1980
   catttacaat cctttagcta gacacagtgc tgattggtgg gtttttacag agtgctgatt 2040
   ggtgcgtctt tacagagtgc tgattggtgc atttacaatc ctttagctag acacagagtg 2100
   ctgattggtg cgtttataat cctctagcta gacagaaaag ttttccaagt ccccacctga 2160
   ccgagaagcc ccactggctt cacctctcac tgttatactt tggacatttg tccccccaaa 2220
   atctcatgtt gaaatgtaac ccctaatgtt ggaactgagg ccagactgga tgtggctggg 2280
   ccatgggga 2289
<210> 52
   <211> 1184
   <212> DNA
   <213> Homo sapiens
<220>
   <221> misc_feature
   <223> sequence of STAR52
<400> 52
   ctcttctttg tttttttatt ttggggtgtg tgggtacgtg taagatgaga aatgtacaaa      60
   cacaagtatt tcagaaactc caagtaatat tctgtctgtg agttcacggt aaataaataa     120
   aaagggcaaa gtgacagaaa tacaggatta ttaaaagcaa aataatgttc tttgaaatcc     180
   cccccttggt gtatttttta tcttaggatg cagcactttc agcatgccca agtattgaaa     240
   gcagtgtttt tacgctacca cggtaatttt atttagaaac cccatgttca cttttagttt     300
   taaaatggtc tttatgacat aaaattatca gcattcatat ttttgtgttt taatattcct     360
   ttggctactt attgaaacag taaacattac gaaaattagt aaacaaatct ttgatagttg     420
   cttatttttg tttaattgaa tgtttatttt attaggtaaa tatacaatca aatttattta     480
   aaaataatga ggaaaagaat acttttcttt cgctttgcga aagcaaagtg atttttcatt     540
   cttctccgtc cgattccttc tcttccagct gccacagccg actgacaggc tcccggcggc     600
   ctgaggagta gtatgcaaat tttggatgat tgacacctac agtagaagcc aatcacgtca     660
   aagtaggatg ctgattggtt gacaacaata ggcgtaaacc ttgacgtttt aaaaacctga     720
   cacccaatcc aggcgattca tgcaaataaa ggaagggagt cacattacca ggggccagag     780
   agacttgagt acgacctcac gtgttcagtg gtggatattg cacagacgtc tgcaaggtct     840
   atataaacgc tacataatgt tcaactcaat tgcttgcctt ggcctttccc aaacttgtca     900
   ctggaatata aattatccct tttttaaaaa taaaaaaata agaattatgt agtgcacata     960
   tatgatggtt catgtagaaa tctaaatgga cttccaacgc atggaatttt cctatttccc    1020
   cctttcttta aattaatcct cagtgaagga ggctgttttc ccctagattt caaaaggacg    1080
   agatttacag agcctttcct tggagaaacc cgctctaggc acagatggtc agtaaattta    1140
   gcttcttcag cgaagttcca catggcaccg ccagatggca taag                      1184
<210> 53
   <211> 1431
   <212> DNA
   <213> Homo sapiens
<220>
   <221> misc_feature
   <223> sequence of STAR53
<400> 53
   ccctgaggaa gatgacgagt aactccgtaa gagaaccttc cactcatccc ccacatccct      60
   gcagacgtgc tattctgtta tgatactggt atcccatctg tcacttgctc cccaaatcat     120
   tcccttctta caattttcta ctgtacagca ttgaggctga acgatgagag atttcccatg     180
   ctctttctac tccctgccct gtatatatcc ggggatcctc cctacccagg atgctgtggg     240
   gtcccaaacc ccaagtaagc cctgatatgc gggccacacc tttctctagc ctaggaattg    300
   ataacccagg cgaggaagtc actgtggcat gaacagatgg ttcacttcga ggaaccgtgg    360
   aaggcgtgtg caggtcctga gatagggcag aatcggagtg tgcagggtct gcaggtcagg    420
   aggagttgag attgcgttgc cacgtggtgg gaactcactg ccacttattt ccttctctct    480
   tcttgcctca gcctcaggga tacgacacat gcccatgatg agaagcagaa cgtggtgacc    540
   tttcacgaac atgggcatgg ctgcggaccc ctcgtcatca ggtgcatagc aagtgaaagc    600
   aagtgttcac aacagtgaaa agttgagcgt catttttctt agtgtgccaa gagttcgatg    660
   ttagcgttta cgttgtattt tcttacactg tgtcattctg ttagatacta acattttcat    720
   tgatgagcaa gacatactta atgcatattt tggtttgtgt atccatgcac ctaccttaga    780
   aaacaagtat tgtcggttac ctctgcatgg aacagcatta ccctcctctc tccccagatg    840
   tgactactga gggcagttct gagtgtttaa tttcagattt tttcctctgc atttacacac    900
   acacgcacac aaaccacacc acacacacac acacacacac acacacacac acacacacac    960
   acacaccaag taccagtata agcatctgcc atctgctttt cccattgcca tgcgtcctgg   1020
   tcaagctccc ctcactctgt ttcctggtca gcatgtactc ccctcatccg attcccctgt   1080
   agcagtcact gacagttaat aaacctttgc aaacgttccc cagttgtttg ctcgtgccat   1140
   tattgtgcac acagctctgt gcacgtgtgt gcatatttct ttaggaaaga ttcttagaag   1200
   tggaattgct gtgtcaaagg agtcatttat tcaacaaaac actaatgagt gcgtcctcgt   1260
   gctgagcgct gttctaggtg ctggagcgac gtcagggaac aaggcagaca ggagttcctg   1320
   acccccgttc tagaggagga tgtttccagt tgttgggttt tgtttgtttg tttcttctag   1380
   agatggtggt cttgctctgt ccaggctaga gtgcagtggc atgatcatag c            1431
<210> 54
   <211> 975
   <212> DNA
   <213> Homo sapiens
<220>
   <221> misc_feature
   <223> sequence of STAR54
<400> 54
   ccataaaagt gtttctaaac tgcagaaaaa tccccctaca gtcttacagt tcaagaattt      60
   tcagcatgaa atgcctggta gattacctga ctttttttgc caaaaataag gcacagcagc    120
   tctctcctga ctctgacttt ctatagtcct tactgaatta tagtccttac tgaattcatt    180
   cttcagtgtt gcagtctgaa ggacacccac attttctctt tgtctttgtc aattctttgt    240
   gttgtaaggg caggatgttt aaaagttgaa gtcattgact tgcaaaatga gaaatttcag    300
   agggcatttt gttctctaga ccatgtagct tagagcagtg ttcacactga ggttgctgct    360
   aatgtttctg cagttcttac caatagtatc atttacccag caacaggata tgatagagga    420
   cttcgaaaac cccagaaaat gttttgccat atatccaaag ccctttggga aatggaaagg    480
   aattgcgggc tcccattttt atatatggat agatagagac caagaaagac caaggcaact    540
   ccatgtgctt tacattaata aagtacaaaa tgttaacatg taggaagtct aggcgaagtt    600
   tatgtgagaa ttctttacac taattttgca acattttaat gcaagtctga aattatgtca    660
   aaataagtaa aaatttttac aagttaagca gagaataaca atgattagtc agagaaataa    720
   gtagcaaaat cttcttctca gtattgactt ggttgctttt caatctctga ggacacagca    780
   gtcttcgctt ccaaatccac aagtcacatc agtgaggaga ctcagctgag actttggcta    840
   atgttggggg gtccctcctg tgtctcccca ggcgcagtga gcctgcaggc cgacctcact    900
   cgtggcacac aactaaatct ggggagaagc aacccgatgc cagcatgatg cagatatctc    960
   agggtatgat cggcc                                                      975
<210> 55
   <211> 501
   <212> DNA
   <213> Homo sapiens
<220>
   <221> misc_feature
   <223> sequence of STAR55
<400> 55
   cctgaactca tgatccgccc acctcagcct cctgaagtgc tgggattaca ggtgtgagcc      60
   accacaccca gccgcaacac actcttgagc aaccaatgtg tcataaaaga aataaaatgg     120
   aaatcagaaa gtatcttgag acagacaaaa atggaaacac aacataccaa aatttatggg     180
   acacagcaaa agcagtttta ggagggaagt ttatagtgat gaatacctac ctcaaaatca     240
   ttagcctgat tggatgacac tacagtgtat aaatgaattg aaaaccacat tgtgccccat     300
   acatatatac aatttttatt tgttaattaa aaataaaata aaactttaaa aaagaagaaa     360
   gagctcaaat aaacaaccta actttatacc tcaaggaaat agaagagcca gctaagccca     420
   aagttgacag aaggaaaaaa atattggcag aaagaaatga aacagagact agaaagacaa     480
   ttgaagagat cagcaaaact a                                               501
<210> 56
   <211> 741
   <212> DNA
   <213> Homo sapiens
<220>
   <221> misc_feature
   <223> sequence of STAR56
<400> 56
   acacaggaaa agatcgcaat tgttcagcag agctttgaac cggggatgac ggtctccctc      60
   gttgcccggc aacatggtgt agcagccagc cagttatttc tctggcgtaa gcaataccag     120
   gaaggaagtc ttactgctgt cgccgccgga gaacaggttg ttcctgcctc tgaacttgct     180
   gccgccatga agcagattaa agaactccag cgcctgctcg gcaagaaaac gatggaaaat     240
   gaactcctca aagaagccgt tgaatatgga cgggcaaaaa agtggatagc gcacgcgccc     300
   ttattgcccg gggatgggga gtaagcttag tcagccgttg tctccgggtg tcgcgtgcgc     360
   agttgcacgt cattctcaga cgaaccgatg actggatgga tggccgccgc agtcgtcaca     420
   ctgatgatac ggatgtgctt ctccgtatac accatgttat cggagagctg ccaacgtatg     480
   gttatcgtcg ggtatgggcg ctgcttcgca gacaggcaga acttgatggt atgcctgcga     540
   tcaatgccaa acgtgtttac cggatcatgc gccagaatgc gctgttgctt gagcgaaaac     600
   ctgctgtacc gccatcgaaa cgggcacata caggcagagt ggccgtgaaa gaaagcaatc     660
   agcgatggtg ctctgacggg ttcgagttct gctgtgataa cggagagaga ctgcgtgtca     720
   cgttcgcgct ggactgctgt g                                               741
<210> 57
   <211> 1365
   <212> DNA
   <213> Homo sapiens
<220>
   <221> misc_feature
   <223> sequence of STAR57
<400> 57
   tccttctgta aataggcaaa atgtatttta gtttccacca cacatgttct tttctgtagg      60
   gcttgtatgt tggaaatttt atccaattat tcaattaaca ctataccaac aatctgctaa     120
   ttctggagat gtggcagtga ataaaaaagt tatagtttct gattttgtgg agcttggact     180
   ttaatgatgg acaaaacaac acattcttaa atatatattt catcaaaatt atagtgggtg     240
   aattatttat atgtgcattt acatgtgtat gtatacataa atgggcggtt actggctgca     300
   ctgagaatgt acacgtggcg cgaacgaggc tgggcggtca gagaaggcct cccaaggagg     360
   tggctttgaa gctgagtggt gcttccacgt gaaaaggctg gaaagggcat tccaagaaaa     420
   ggctgaggcc agcgggaaag aggttccagt gcgctctggg aacggaaagc gcacctgcct     480
   gaaacgaaaa tgagtgtgct gaaataggac gctagaaagg gaggcagagg ctggcaaaag     540
   cgaccgagga ggagctcaaa ggagcgagcg gggaaggccg ctgtggagcc tggaggaagc     600
   acttcggaag cgcttctgag cgggtaaggc cgctgggagc atgaactgct gagcaggtgt     660
   gtccagaatt cgtgggttct tggtctcact gacttcaaga atgaagaggg accgcggacc     720
   ctcgcggtga gtgttacagc tcttaaggtg gcgcgtctgg agtttgttcc ttctgatgtt     780
   cggatgtgtt cagagtttct tccttctggt gggttcgtgg tctcgctggc tcaggagtga     840
   agctgcagac cttcgcggtg agtgttacag ctcataaaag cagggtggac tcaaagagtg     900
   agcagcagca agatttattg caaagaatga aagaacaaag cttccacact gtggaagggg     960
   accccagcgg gttgccactg ctggctccgc agcctgcttt tattctctta tctggcccca    1020
   cccacatcct gctgattggt agagccgaat ggtctgtttt gacggcgctg attggtgcgt    1080
   ttacaatccc tgcgctagat acaaaggttc tccacgtccc caccagatta gctagataga    1140
   gtctccacac aaaggttctc caaggcccca ccagagtagc tagatacaga gtgttgattg    1200
   gtgcattcac aaaccctgag ctagacacag ggtgatgact ggtgtgttta caaaccttgc    1260
   ggtagataca gagtatcaat tggcgtattt acaatcactg agctaggcat aaaggttctc    1320
   caggtcccca ccagactcag gagcccagct ggcttcaccc agtgg                    1365
<210> 58
   <211> 1401
   <212> DNA
   <213> Homo sapiens
<220>
   <221> misc_feature
   <223> sequence of STAR58
<400> 58
   aagtttacct tagccctaaa ttatttcatt gtgattggca ttttaggaaa tatgtattaa      60
   ggaatgtctc ttaggagata aggataacat atgtctaaga aaattatatt gaaatattat     120
   tacatgaact aaaatgttag aactgaaaaa aaattattgt aactccttcc agcgtaggca     180
   ggagtatcta gataccaact ttaacaactc aactttaaca acttcgaacc aaccagatgg     240
   ctaggagatt cacctattta gcatgatatc ttttattgat aaaaaaatat aaaacttcca     300
   ttaaattttt aagctactac aatcctatta aattttaact taccagtgtt ctcaatgcta     360
   cataatttaa aatcattgaa atcttctgat tttaactcct cagtcttgaa atctacttat     420
   ttttagttac atatatatcc aatctactgc cgctagtaga agaagcttgg aatttgagaa     480
   aaaaatcaga cgttttgtat attctcatat tcactaattt attttttaaa tgagtttctg      540
   caatgcatca agcagtggca aaacaggaga aaaattaaaa ttggttgaaa agatatgtgt     600
   gccaaacaat cccttgaaat ttgatgaagt gactaatcct gagttattgt ttcaaatgtg     660
   tacctgttta tacaagggta tcacctttga aatctcaaca ttaaatgaaa ttttataagc     720
   aatttgttgt aacatgatta ttataaaatt ctgatataac attttttatt acctgtttag   780
   agtttaaaga gagaaaagga gttaagaata attacatttt cattagcatt gtccgggtgc     840
   aaaaacttct aacactatct tcaaatcttt ttctccattg ccttctgaac atacccactt     900
   gggtatctca ttagcactgc aaattcaaca ttttcgattg ctaatttttc tccctaaata     960
   tttatttgtt ttctcagctt tagccaatgt ttcactattg accatttgct caagtatagt    1020
   gacgcttcaa tgaccttcag agagctgttt cagtccttcc tggactactt gcatgcttcc    1080
   aacaaaatga agcactcttg atgtcagtca ctcaaataaa tggaaatggg cccatttact    1140
   aggaatgtta acagaataaa aagatagacg tgacaccagt tgcttcagtc catctccatt    1200
   tacttgctta aggcctggcc atatttctca cagttgatat ggcgcagggc acatgtttaa    1260
   atggctgttc ttgtaggatg gtttgactgt tggattcctc atcttccctc tccttaggaa    1320
   ggaaggttac agtagtactg ttggctcctg gaatatagat tcataaagaa ctaatggagt    1380
   atcatctccc actgctcttg t                                              1401
<210> 59
   <211> 866
   <212> DNA
   <213> Homo sapiens
<220>
   <221> misc_feature
   <223> sequence of STAR59
<400> 59
   gagatcacgc cactgcactc cagcctgggg gacagagcaa gactccatct cagaaacaaa     60
   caaacacaca aagccagtca aggtgtttaa ttcgacggtg tcaggctcag gtctcttgac    120
   aggatacatc cagcacccgg gggaaacgtc gatgggtggg gtggaatcta ttttgtggcc    180
   tcaagggagg gtttgagagg tagtcccgca agcggtgatg gcctaaggaa gcccctccgc    240
   ccaagaagcg atattcattt ctagcctgta gccacccaag agggagaatc gggctcgcca    300
   cagaccccac aacccccaac ccaccccacc cccacccctc ccacctcgtg aaatgggctc    360
   tcgctccgtc aggctctagt cacaccgtgt ggttttggaa cctccagcgt gtgtgcgtgg    420
   gttgcgtggt ggggtggggc cggctgtgga cagaggaggg gataaagcgg cggtgtcccg    480
   cgggtgcccg ggacgtgggg cgtggggcgt gggtggggtg gccagagcct tgggaactcg    540
   tcgcctgtcg ggacgtctcc cctcctggtc ccctctctga cctacgctcc acatcttcgc    600
   cgttcagtgg ggaccttgtg ggtggaagtc accatccctt tggactttag ccgacgaagg    660
   ccgggctccc aagagtctcc ccggaggcgg ggccttgggc aggctcacaa ggatgctgac    720
   ggtgacggtt ggtgacggtg atgtacttcg gaggcctcgg gccaatgcag aggtatccat    780
   ttgacctcgg tgggacaggt cagctttgcg gagtcccgtg cgtccttcca gagactcatc    840
   cagcgctagc aagcatggtc ccgagg                                         866
<210> 60
   <211> 2067
   <212> DNA
   <213> Homo sapiens
<220>
   <221> misc_feature
   <223> sequence of STAR60
<220>
   <221> misc_feature
   <222> (92)..(1777)
   <223> n is a, c, g, or t on various positions
<400> 60
   agcagtgcag aactggggaa gaagaagagt ccctacacca cttaatactc aaaagtactc      60
   gcaaaaaata acacccctca ccaggtggca tnattactct ccttcattga gaaaattagg     120
   aaactggact tcgtagaagc taattgcttt atccagagcc acctgcatac aaacctgcag     180
   cgccacctgc atacaaacct gtcagccgac cccaaagccc tcagtcgcac caagcctctg     240
   ctgcacaccc tcgtgccttc acactggccg ttccccaagc ctggggcata ctncccagct     300
   ctgagaaatg tattcatcct tcaaagccct gctcatgtgt cctnntcaac aggaaaatct     360
   cccatgagat gctctgctat ccccatctct cctgccccat agcttaggca nacttctgtg     420
   gtggtgagtc ctgggctgtg ctgtgatgtg ttcgcctgcn atgtntgttc ttccccacaa     480
   tgatgggccc ctgaattctc tatctctagc acctgtgctc agtaaaggct tgggaaacca     540
   ggctcaaagc ctggcccaga tgccaccttt tccagggtgc ttccgggggc caccaaccag     600
   agtgcagcct tctcctccac caggaactct tgcagcccca cccctgagca cctgcacccc     660
   attacccatc tttgtttctc cgtgtgatcg tattattaca gaattatata ctgtattctt     720
   aatacagtat ataattgtat aattattctt aatacagtat ataattatac aaatacaaaa     780
   tatgtgttaa tggaccgttt atgttactgg taaagcttta agtcaacagt gggacattag     840
   ttaggttttt ggcgaagtca aaagttatat gtgcattttc aacttcttga ggggtcggta     900
   cntctnaccc ccatgttgtt caanggtcaa ctgtctacac atatcatagc taattcacta     960
   cagaaatgtt agcttgtgtc actagtatct ccccttctca taagcttaat acacatacct    1020
   tgagagagct cttggccatc tctactaatg actgaagttt ttatttatta tagatgtcat    1080
   aataggcata aaactacatt acatcattcg agtgccaatt ttgccacctt gaccctcttt    1140
   tgcaaaacac caacgtcagt acacatatga agaggaaact gcccgagaac tgaagttcct    1200
   gagaccagga gctgcaggcg ttagatagaa tatggtgacg agagttacga ggatgacgag    1260
   agtaaatact tcatactcag tacgtgccaa gcactgctat aagcgctctg tatgtgtgaa    1320
   gtcatttaat cctcacagca tcccacggtg taattatttt cattatcccc atgagggaac    1380
   agaaactcag aacggttcaa cacatatgcg agaagtcgca gccggtcagt gagagagcag    1440
   gttcccgtcc aagcagtcag accccgagtg cacactctcg acccctgtcc agcagactca    1500
   ctcgtcataa ggcggggagt gntctgtttc agccagatgc tttatgcatc tcagagtacc    1560
   caaaccatga aagaatgagg cagtattcan gagcagatgg ngctgggcag taaggctggg    1620
   cttcagaata gctggaaagc tcaagtnatg ggacctgcaa gaaaaatcca ttgtttngat   1680
   aaatagccaa agtccctagg ctgtaagggg aaggtgtgcc aggtgcaagt ggagctctaa   1740
   tgtaaaatcg cacctgagtc tcctggtctt atgagtnctg ggtgtacccc agtgaaaggt   1800
   cctgctgcca ccaagtgggc catggttcag ctgtgtaagt gctgagcggc agccggaccg   1860
   cttcctctaa cttcacctcc aaaggcacag tgcacctggt tcctccagca ctcagctgcg   1920
   aggcccctag ccagggtccc ggcccccggc ccccggcagc tgctccagct tccttcccca    1980
   cagcattcag gatggtctgc gttcatgtag acctttgttt tcagtctgtg ctccgaggtc   2040
   actggcagca ctagccccgg ctcctgt                                        2067
<210> 61
   <211> 1470
   <212> DNA
   <213> Homo sapiens
<220>
   <221> misc_feature
   <223> sequence of STAR61
<220>
   <221> misc_feature
   <222> (130)..(976)
   <223> n is a, c, g, or t on various positions
<400> 61
   cagcccccac atgcccagcc ctgtgctcag ctctgcagcg gggcatggtg ggcagagaca     60
   cagaggccaa ggccctgctt cggggacggt gggcctggga tgagcatggc cttggccttc    120
   gccgagagtn ctcttgtgaa ggaggggtca ggaggggctg ctgcagctgg ggaggagggc    180
   gatggcactg tggcangaag tgaantagtg tgggtgcctn gcaccccagg cacggccagc    240
   ctggggtatg gacccggggc cntctgttct agagcaggaa ggtatggtga ggacctcaaa    300
   aggacagcca ctggagagct ccaggcagag gnacttgaga ggccctgggg ccatcctgtc    360
   tcttttctgg gtctgtgtgc tctgggcctg ggcccttcct ctgctccccc gggcttggag    420
   agggctggcc ttgcctcgtg caaaggacca ctctagactg gtaccaagtc tggcccatgg    480
   cctcctgtgg gtgcaggcct gtgcgggtga cctgagagcc agggctggca ggtcagagtc    540
   aggagaggga tggcagtgga tgccctgtgc aggatctgcc taatcatggt gaggctggag    600
   gaatccaaag tgggcatgca ctctgcactc atttctttat tcatgtgtgc ccatcccaac    660
   aagcagggag cctggccagg agggcccctg ggagaaggca ctgatgggct gtgttccatt    720
   taggaaggat ggacggttgt gagacgggta agtcagaacg ggctgcccac ctcggccgag    780
   agggccccgt ggtgggttgg caccatctgg gcctggagag ctgctcagga ggctctctag    840
   ggctgggtga ccaggnctgg ggtacagtag ccatgggagc aggtgcttac ctggggctgt    900
   ccctgagcag gggctgcatt gggtgctctg tgagcacaca cttctctatt cacctgagtc     960
   ccnctgagtg atgagnacac ccttgttttg cagatgaatc tgagcatgga gatgttaagt   1020
   ggcttgcctg agccacacag cagatggatg gtgtagctgg gacctgaggg caggcagtcc   1080
   cagcccgagg acttcccaag gttgtggcaa actctgacag catgacccca gggaacaccc   1140
   atctcagctc tggtcagaca ctgcggagtt gtgttgtaac ccacacagct ggagacagcc    1200
   accctagccc cacccttatc ctctcccaaa ggaacctgcc ctttcccttc attttcctct     1260
   tactgcattg agggaccaca cagtgtggca gaaggaacat gggttcagga cccagatgga   1320
   cttgcttcac agtgcagccc tcctgtcctc ttgcagagtg cgtcttccac tgtgaagttg   1380
   ggacagtcac accaactcaa tactgctggg cccgtcacac ggtgggcagg caacggatgg   1440
   cagtcactgg ctgtgggtct gcagaggtgg                                    1470
<210> 62
   <211> 1011
   <212> DNA
   <213> Homo sapiens
<220>
   <221> misc_feature
   <223> sequence of STAR62
<400> 62
   agtgtcaaat agatctacac aaaacaagat aatgtctgcc catttttcca aagataatgt     60
   ggtgaagtgg gtagagagaa atgcatccat tctccccacc caacctctgc taaattgtcc    120
   atgtcacagt actgagacca gggggcttat tcccagcggg cagaatgtgc accaagcacc    180
   tcttgtctca atttgcagtc taggccctgc tatttgatgg tgtgaaggct tgcacctggc    240
   atggaaggtc cgttttgtac ttcttgcttt agcagttcaa agagcaggga gagctgcgag    300
   ggcctctgca gcttcagatg gatgtggtca gcttgttgga ggcgccttct gtggtccatt    360
   atctccagcc cccctgcggt gttgctgttt gcttggcttg tctggctctc catgccttgt    420
   tggctccaaa atgtcatcat gctgcacccc aggaagaatg tgcaggccca tctcttttat    480
   gtgctttggg ctattttgat tccccgttgg gtatattccc taggtaagac ccagaagaca    540
   caggaggtag ttgctttggg agagtttgga cctatgggta tgaggtaata gacacagtat    600
   cttctctttc atttggtgag actgttagct ctggccgcgg actgaattcc acacagctca    660
   cttgggaaaa ctttattcca aaacatagtc acattgaaca ttgtggagaa tgagggacag    720
   agaagaggcc ctagatttgt acatctgggt gttatgtcta taaatagaat gctttggtgg    780
   tcaactagac ttgttcatgt tgacatttag tcttgccttt tcggtggtga tttaaaaatt    840
   atgtatatct tgtttggaat atagtggagc tatggtgtgg cattttcatc tggctttttg    900
   tttagctcag cccgtcctgt tatgggcagc cttgaagctc agtagctaat gaagaggtat    960
   cctcactccc tccagagagc ggtcccctca cggctcattg agagtttgtc a            1011
<210> 63
   <211> 1410
   <212> DNA
   <213> Homo sapiens
<220>
   <221> misc_feature
   <223> sequence of STAR63
<400> 63
   ccacagcctg atcgtgctgt cgatgagagg aatctgctct aagggtctga gcggagggag      60
   atgccgaagc tttgagcttt ttgtttctgg cttaaccttg gtggattttc accctctggg     120
   cattacctct tgtccagggg aggggctggg ggagtgcctg gagctgtagg gacagagggc     180
   tgagtggggg ggactgcttg ggctgaccac ataatattct gctgcgtatt aatttttttt     240
   tgagacagtc tttctctgtt gcccaggctg gagtgtaatg gcttgatagc tcactgccac     300
   ctccgcctcc tgggttcaag tgattctcct gcttcagctt ccggagtagc tgggactgca     360
   ggtgcccgcc accatggctg gctaattttt gtatttttat tagcaatggg gttttgctat     420
   gttgcccagg ccggtcccga actcctgccc tcaagtgata cacctgcctc ggcctcccaa     480
   agtgctggga ttagaggctt gagccactgc gcctggccag ctgcatattg ttaattagac     540
   ataaaatgca aaataagatg atataaacac aaaggtgtga aataagatgg acacctgctg     600
   agcgcgcctg tcctgaagca tcgcccctct gcaaaagcag gggtcagcat gtgttctccg     660
   gtccttgctc ttacagagga gtgagctgcc tatgcgtctt ccagccactt cctgggctgc     720
   tcagaggcct ctcacgggtg ttctgggttg ctgccacttg caggggtgct gaggcggggc     780
   tcctcccgtg cggggcatgt ccaggccgcc ctctctgaag gcttggcagg tacaggtggg     840
   agtgggggtc tctgggctgc tgtggggact gggcaggctc ctggaagacc tccctgtgtt     900
   tgggctgaaa gcgcagcccg aggggaggtc cccagggagg ccgctgtcgg gggtgggggc     960
   ttggaggagg gaggggccga ggagccggcg acactccgtg acggcccagg aacgtcccta    1020
   aacaaggcgc cgcgttctcg atggggtggg gtccgctttc ttttctcaaa agctgcagtt    1080
   actccatgct cggaggactg gcgtccgcgc cctgttccaa tgctgccccg gggccctggc    1140
   cttggggaat cggggccttg gactggaccc tgggggcttc gcggagccgg gcctggcggg    1200
   gcgagcggag cagaggctgg gcagccccgg ggaagcgctc gccaaagccg ggcgctgctc    1260
   ccagagcgcg aggtgcagaa ccagaggctg gtcccgcggc gctaacgaga gaagaggaag    1320
   cgcgctgtgt agagggcgcc caccccgtgg ggcgaacccc cttcctcaac tccatggacg    1380
   gggctcatgg gttcccagcg gctcagacgc                                     1410
<210> 64
   <211> 1414
   <212> DNA
   <213> Homo sapiens
<220>
   <221> misc_feature
   <223> sequence of STAR64
<400> 64
   tggatcagat ttgttttata ccctcccttc tactgctctg agagttgtac atcacagtct      60
   actgtatctg tttcccatta ttataatttt tttgcactgt gcttgcctga agggagcctc     120
   aagttcatga gtctccctac cctcctccca aatgagacat ggacctttga atgctttcct     180
   gggaccacca ccccaccttt catgctgctg ttatccagga ttttagttca acagtgtttt     240
   aaccccccaa atgagtcatt tttattgttt cgtatagtga atgtgtattt gggtttgctt      300
   atatggtgac ctgtttattt gctcctcatt gtacctcatg ctctgctctt tccttctaga     360
   ttcagtctct ttcctaatga ggtgtctcgc agcaattctt tacaagacag ccaagatagg     420
   ccagctctca gagcacttgt tgtctgaaaa agtcttgtct tatttaattt ctttttctta     480
   gagatggggt ctcattatgt tacccacact ggtctcaaac ttctggctta aagcggtcct     540
   cccaccttgg cctcccaaag tgctaggatt acaggcgtga gcgacctcgt ccagcctgtc     600
   tgagaaagcg tttgttttgc ccttgctctc agatgacagt ttggggatag aattctaggt     660
   ggacggtttt tttccttcag ccctttgaag agtctgtatt ttcattatct ccctgcatta     720
   gatgttcttt tgcaagtaac gtgtcttttc tctctgggta ttcttaaggt tttctctttg     780
   cctttggtga gctgcagtgg atttgctttt ttcaagaggt caagagaaag gaaagtgtga     840
   ggtttctgtt ttttactgac aatttgtttg ttgatttgtt ttcccaccca gaggttcctt     900
   gccactttgc caggctggaa ggcagacttc ttctggtgtc ctgttcacag acggggcagc     960
   ctgcggaagg ccctgccaca tgcagggcct cggtcctcat tcccttgcat gtggacccgg    1020
   gcgtgactcc tgttcaggct ggcacttccc agagctgagc cccagcctga ccttcctccc    1080
   atactgtctt cacaccccct cctttcttct gatacctgga ggttttcctt tctttcctgt    1140
   cacctccact tggattttaa atcctctgtc tgtggaattg tattcggcac aggaagatgc    1200
   ttgcaagggc caggctcatc agccctgtcc ctgctgctgg aagcagcaca gcagagcctc    1260
   atgctcaggc tgagatggag cagaggcctg cagacgagca cccagctcag ctggggttgg    1320
   cgccgatggt ggagggtcct cgaaagctct ggggacgatg gcagagctat tggcagggga    1380
   gccgcagggt cttttgagcc cttaaaagat ctct                                1414
<210> 65
   <211> 1310
   <212> DNA
   <213> Homo sapiens
<220>
   <221> misc_feature
   <223> sequence of STAR65
<400> 65
   gtgaatgttg atggatcaaa tatctttctg tgttgtttat caaagttaaa ataaatgtgg     60
   tcatttaaag gacaaaagat gaggggttgg agtctgttca agcaaagggt atattaggag    120
   aaaagcagaa ttctctccct gtgaagggac agtgactcct attttccacc tcatttttac    180
   taactctcct aactatctgc ttaggtagag atatatccat gtacatttat aaaccacagt    240
   gaatcatttg attttggaat aaagatagta taaaatgtgt cccagtgttg atatacatca    300
   tacattaaat atgtctggca gtgttctaat tttacagttg tccaaagata atgttagggc    360
   atactggcta tggatgaagc tccaatgttc agattgcaaa gaaacttaga attttactaa    420
   tgaaaccaaa tacatcccaa gaaatttttc agaagaaaaa aagagaaact agtagcaaag     480
   taaagaatca ccacaatatc atcagatttt ttttatatgt agaatattta ttcagttctt    540
   ttttcaagta caccttgtct tcattcattg tactttattt tttgtgaagg tttaaattta    600
   tttcttctat gtgtttagtg atatttaaaa tttttattta atcaagttta tcagaaagtt    660
   ctgttagaaa atatgacgag gctttaattc cgccatctat attttccgct attatataaa    720
   gataattgtt ttctcttttt aaaacaactt gaattgggat tttatatcat aattttttaa   780
   tgtctttttt tattatactt taagttctgg gatacatgtg cagaacgtgc aggtgtgtta    840
   catagatata cacgtgccat ggtggtttgc tgcacccact aacctgttat cgacattagg    900
   tatttctcct aatgctatca ccccctattt ccccaccccc cgagaggccc cagtgtgtga    960
   tgttctcctc cctgtgtcca tgtgttctca ttgttcatct cccacttatg gtatctacca   1020
   taaccttgaa attgtcttat gcattcactt gtttggttgt tatatagcct ccatcaggac   1080
   agggatattt gctgctgctt cttttttttt tctttttgag acagtcttgc tccgtcatcc   1140
   aggctggagt gcttctcggc tcaatgcaac ctccacctcc caggtttaag cgattctcca   1200
   acttcagcct cccaaatggc tgggactgca ggcatgcacc actacacctg gctaattttt   1260
   gtatttgtaa tagagacaat gtttcaccat gttggccagg ctggtctcga               1310
<210> 66
   <211> 1917
   <212> DNA
   <213> Homo sapiens
<220>
   <221> misc_feature
   <223> sequence of STAR67
<400> 66
   aggatcctaa aattttgtga ccctagagca agtactaact atgaaagtga aatagagaat     60
   gaaggaatta tttaattaag tccagcaaaa cccaaccaaa tcatctgtaa aatatatttg    120
   ttttcaacat ccaggtattt tctgtgtaaa aggttgagtt gtatgctgac ttattgggaa    180
   aaataattga gttttcccct tcactttgcc agtgagagga aatcagtact gtaattgtta    240
   aaggttaccc atacctacct ctactaccgt ctagcatagg taaagtaatg tacactgtga    300
   agtttcctgc ttgactgtaa tgttttcagt ttcatcccat tgattcaaca gctatttatt    360
   cagcacttac tacaaccatg ctggaaaccc aagagtaaat aggctgtgtt actcaacagg    420
   actgaggtac agccgaactg tcaggcaagg ttgctgtcct ttggacttgc ctgctttctc    480
   tctatgtagg aagaagaaat ggacataccg tccaggaaat agatatatgt tacatttcct    540
   tattccataa ttaatattaa taaccctgga cagaaactac caagtttcta gacccttata    600
   gtaccacctt accctttctg gatgaatcct tcacatgttg atacatttta tccaaatgaa    660
   aattttggta ctgtaggtat aacagacaaa gagagaacag aaaactagag atgaagtttg    720
   ggaaaaggtc aagaaagtaa ataatgcttc tagaagacac aaaaagaaaa atgaaatggt    780
   aatgttggga aagttttaat acattttgcc ctaaggaaaa aaactacttg ttgaaattct    840
   acttaagact ggaccttttc tctaaaaatt gtgcttgatg tgaattaaag caacacaggg    900
   aaatttatgg gctccttcta agttctaccc aactcaccgc aaaactgttc ctagtaggtg    960
   tggtatactc tttcagattc tttgtgtgta tgtatatgtg tgtgtgtgtg tgtgtttgta   1020
   tgtgtacagt ctatatacat atgtgtacct acatgtgtgt atatataaat atatatttac   1080
   ctggatgaaa tagcatatta tagaatattc ttttttcttt aaatatatat gtgcatacat   1140
   atgtatatgc acatatatac ataaatgtag atatagctag gtaggcattc atgtgaaaca   1200
   aagaagccta ttacttttta atggttgcat gatattccat cataggagta tagtacaact   1260
   tatgtaacac acatttggct tgttgtaaaa ttttggtatt aataaaatag cacatatcat   1320
   gcaaagacac ccttgcatag gtctattcat tctttgattt ttaccttagg acaaaattta   1380
   aaagtagaat ttctgggtca agcagtatgc tcatttaaaa tgtcattgca tatttccaaa   1440
   ttgtcctcca gaaaagtagt aacagtaaca attgatggac tgcgtgtttt ctaaaacttg   1500
   catttttttc cttattggtg aggtttggca ttttccatat gtttattggc attttaattt   1560
   tttttggttc atgtctttta ttcccttcct gcaaatttgt ggtgtgtctc aactttattt   1620
   atactctcat tttcataatt ttctaaagga atttgacttt aaaaaaataa gacagccaat   1680
   gctttggttt aatttcattg ctgctttttg aagtgactgc tgtgttttta tatactttta   1740
   tattttgttg ttttagcaaa ttcttctata ttataattgt gtatgctgga acaaaaagtt   1800
   atatttctta atctagataa aatatttcaa gatgttgtaa ttacagtccc ctctaaaatc   1860
   atataaatag acgcatagct gtgtgatttg taattagtta tgtccattga tagatcc      1917
<210> 67
   <211> 26
   <212> DNA
   <213> Artificial
<220>
   <223> oligo for making linker containing MCSII of pd2EGFP-link
<400> 67
   gtacggatat cagatcttta attaag 26
<210> 68
   <211> 24
   <212> DNA
   <213> Artificial
<220>
   <223> oligo for making linker containing MCSII of pd2EGFP-link
<400> 68
   gtaccttaat taaagatctg atat 24
<210> 69
   <211> 52
   <212> DNA
   <213> Artificial
<220>
   <223> primer for amplification of 0.37 kb from pd2EGFP
<400> 69
   gatcagatct ggcgcgccat ttaaatcgtc tcgcgcgttt cggtgatgac gg 52
<210> 70
   <211> 41
   <212> DNA
   <213> Artificial
<220>
   <223> primer for amplification of 0.37 kb from pd2EGFP
<400> 70
   aggcggatcc gaatgtattt agaaaaataa acaaataggg g 41
<210> 71
   <211> 36
   <212> DNA
   <213> Artificial
<220>
   <223> primer for amplifying zeocin resistance gene ORF
<400> 71
   gatcggatcc ttcgaaatgg ccaagttgac cagtgc 36
<210> 72
   <211> 32
   <212> DNA
   <213> Artificial
<220>
   <223> primer for amplifying zeocin resistance gene ORF
<400> 72
   aggcgcggcc gcaattctca gtcctgctcc tc 32
<210> 73
   <211> 40
   <212> DNA
   <213> Artificial
<220>
   <223> primer for amplifying d2EGFP ORF
<400> 73
   gatcgaattc tcgcgaatgg tgagcaagca gatcctgaag 40
<210> 74
   <211> 52
   <212> DNA
   <213> Artificial
<220>
   <223> primer for amplifying d2EGFP ORF
<400> 74
   aggcgaattc accggtgttt aaacttacac ccactcgtgc aggctgccca gg 52
<210> 75
   <211> 41
   <212> DNA
   <213> Artificial
<220>
   <223> primer for amplifying d2EGFP gene
<400> 75
   ttggttggtc atgaatggtg agcaagggcg aggagctgtt c 41
<210> 76
   <211> 36
   <212> DNA
   <213> Artificial
<220>
   <223> primer for amplifying d2EGFP gene
<400> 76
   attctctaga ctacacattg atcctagcag aagcac 36
<210> 77
   <211> 42
   <212> DNA
   <213> Artificial
<220>
   <223> oligo for preparing linker for creating MCS in pGL3-promoter-GFP
<400> 77
   cgatatcttg gagatctact agtggcgcgc cttgggctag ct 42
<210> 78
   <211> 50
   <212> DNA
   <213> Artificial
<220>
   <223> oligo for preparing linker for creating MCS in pGL3-promoter-GFP
<400> 78
   gatcagctag cccaaggcgc gccactagta gatctccaag atatcgagct 50
<210> 79
   <211> 40
   <212> DNA
   <213> Artificial
<220>
   <223> primer for amplification of EF-lalfa promoter
<400> 79
   gatcggcgcg ccatttaaat ccgaaaagtg ccacctgacg 40
<210> 80
   <211> 34
   <212> DNA
   <213> Artificial
<220>
   <223> primer for amplification of EF-lalfa promoter
<400> 80
   aggcgggacc ccctcacgac acctgaaatg gaag 34
<210> 81
   <211> 51
   <212> DNA
   <213> Artificial
<220>
   <223> primer for amplification of SV40 promoter
<400> 81
   ttggttgggg cgcgccgcag caccatggcc tgaaataacc tctgaaagag g 51
<210> 82
   <211> 55
   <212> DNA
   <213> Artificial
<220>
   <223> primer for amplification of SV40 promoter
<400> 82
   ttggttggga gctcaagctt tttgcaaaag cctaggcctc caaaaaagcc tcctc 55